# EUROPEAN PATENT SPECIFICATION

(11) **EP 0 674 623 B1**
(45) Date of publication and mention of the grant of the patent: **09.04.2003**
(21) Application number: 94904878.9
(22) Date of filing: 21.12.1993
(51) Int. Cl.: C07D 223/16, A61K 31/55, C07D 243/14, C07D 401/12, C07D 401/14

(54) **BICYCLIC FIBRINOGEN ANTAGONISTS**
BICYKLISCHE FIBRINOGEN ANTAGONISTE
ANTAGONISTES BICYCLIQUES DU FIBRINOGENE

(30) Priority: 21.12.1992 US 992885; 26.03.1993 US 37948
(43) Date of publication of application: 04.10.1995
(73) Proprietor: SMITHKLINE BEECHAM CORPORATION, Philadelphia Pennsylvania 19103 (US)
(72) Inventor: BONDINELL, William, Edward, Wayne, PA 19087 (US); CALLAHAN, James, Francis, Philadelphia, PA 19111 (US); HUFFMAN, William, Francis, Malvern, PA 19355 (US); KEENAN, Richard, McCulloch, Malvern, PA 19355 (US); KU, Thomas, Wen-Fu, Dresher, PA 19025 (US); NEWLANDER, Kenneth, Allen, West Chester, PA 19382 (US); SAMANEN, James, Martin, Phoenixville, PA 19460 (US); UZINSKAS, Irene, Nijole, Villanova, PA 19085 (US)
(74) Representative: Valentine, Jill Barbara
(86) International application number: US9312436
(87) International publication number: WO94014776

(56) References cited:
- EP-A- 0 048 045
- EP-A- 0 447 857
- WO-A-92/07568
- WO-A-93/00095
- DE-A- 3 702 755
- TETRAHEDRON, vol.36, no.10, 1980 pages 1385 - 1397 TIGIHINEANU 'Double cyclisation of phenylglycine-ortho-carboxylic acids-I.'

## Description

### Field of the Invention

This invention relates to novel bicyclic compounds which inhibit platelet aggregation, pharmaceutical compositions containing the compounds and methods of using the compounds.

### Background of the Invention

Platelet aggregation is believed to be mediated primarily through the fibrinogen receptor, or GPIIb-IIIa platelet receptor complex, which is a member of a family of adhesion receptors referred to as integrins. It has been found that frequently the natural ligands of integrin receptors are proteins which contain an Arg-Gly-Asp sequence. Von Willebrand factor and fibrinogen, which are considered to be natural ligands for the GPIIb-IIIa receptor, possess an Arg-Gly-Asp (RGD in single letter amino acid code) sequence in their primary structure. Functionally, these proteins are able to bind and crosslink GPIIb-IIIa receptors on adjacent platelets and thereby effect aggregation of platelets.

Fibronectin, vitronectin and thrombospondin are RGD-containing proteins which have also been demonstrated to bind to GPIIb-IIIa. Fibronectin is found in plasma and as a structural protein in the intracellular matrix. Binding between the structural proteins and GPIIb-IIIa may function to cause platelets to adhere to damaged vessel walls.

Linear and cyclic peptides which bind to vitronectin and contain an RGD sequence are disclosed in WO 89/05150 (PCT US88/04403). EP 0 275 748 discloses linear tetra- to hexapeptides and cyclic hexa- to octapeptides which bind to the GPIIb-IIIa receptor and inhibit platelet aggregation. Other linear and cyclic peptides are reported in EP-A 0 341 915. However, the peptide like structures of such inhibitors often pose problems, such as in drug delivery, metabolic stability and selectivity. Inhibitors of the fibrinogen receptor which are not constructed of natural amino acid sequences are disclosed in EP-A 0 372,486, EP-A 0 381 033 and EP-A 0 478 363. WO 92/07568 (PCT/US91/08166) discloses fibrinogen receptor antagonists which mimic a conformational γ-turn in the RGD sequence by forming a monocyclic seven-membered ring structure. There remains a need, however, for novel fibrinogen receptor antagonists (e.g. inhibitors of the GPIIb-IIIa protein) which have potent *in vivo* and *in vitro* effects and lack the peptide backbone structure of amino acid sequences.

The present invention discloses novel bicyclic compounds including benzazepines and benzodiazepines, which are inhibitors of the GPIIb-IIIa receptor and inhibit platelet aggregation. Certain 5-phenyl-1,4-benzodiazepines are known as a class of drugs which affect the central nervous system, and have been used as anxiolytics. See Stembach, L.H., *J. Med. Chem.,* 22, 2 (1979). It has also been disclosed that certain 5-phenyl-1,4-benzodiazepines antagonize the effects of cholecystokinin. See Friedinger, *Med. Res. Rev.,* 9, 271 (1989). Certain bicyclic compounds which have fibrinogen antagonist activity are disclosed in WO 93/08174 (PCT/US92/08788) and WO 93/00095 (PCT/US/92/05463, published 7 January 1993).

Certain oxazolo[3,2-a]quinolinium compounds, and an unusual rearrangement to a 1,4 benzoxazepine, are reported in Tighneanu *et al*., *Tetrahedron*, 36, 1385 (1980). No biological activity is reported for such compounds. EP 447 857 (Hokuriku Pharmaceutical) discloses certain tricyclic and tetracyclic carboxylic acids which have anti-allergic properties. EP 048 045 (Duphar) discloses phenyl piperazine derivatives which have antiagressive activity. DE 3702755 (Hoechst AG) discloses certain 3,5-di-t-butyl-4-hydroxybenzamide derivatives for the treatment of pain and inflammation. WO 92/07568 (SmithKline Beecham) discloses certain substituted azepine and diazepine acetic acids which are useful for inhibiting platelet aggregation.

### Summary of the Invention

In one aspect this invention is a bicyclic compound comprising a substituted six-membered ring fused to a substituted seven-membered ring as described hereinafter in formula (I).

This invention is also a pharmaceutical composition for inhibiting platelet aggregation or clot formation, which comprises a compound of formula (I) and a pharmaceutically acceptable carrier.

This invention further comprises the use of a compound of formula (I) in the manufacture of a medicament for inhibiting platelet aggregation

A method for inhibiting reocclusion of an artery or vein in a mammal following fibrinolytic therapy comprises internally administering an effective amount of a fibrinolytic agent and a compound of formula (I). This invention is also a method for treating stroke, transient ischemia attacks, or myocardial infarction.

### Detailed Description of the Invention

This invention discloses novel bicyclic compounds which inhibit platelet aggregation. The novel bicyclic compounds comprise a seven-membered ring fused to an aromatic six membered ring and having a nitrogen-containing substituent on the six membered ring and an aliphatic substituent, preferably containing an acidic moiety, on the seven membered ring. The fused 6-7 ring system is believed to interact favorably with the GPIIb-IIIa receptor and to orient the substituent sidechains on the six and seven membered rings so that they may also interact favorably with the receptor.

Although not intending to be bound to any specific mechanism of action, these compounds are believed to inhibit the binding of fibrinogen to the platelet-bound fibrinogen receptor GPIIb-IIIa, and may interact with other adhesion proteins via antagonism of a putative RGD binding site.

The compounds of this invention are compounds of formula (I): 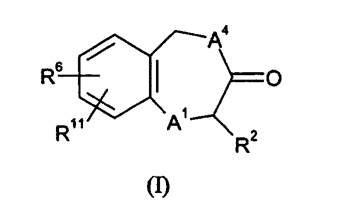 wherein
A¹ is O, S, N-R¹ or CHR¹;
A⁴ is N-R⁴ or CHR⁴;
R² is CH₂R⁷;
R¹ and R⁴ are H, Q-C₁₋₆alkyl, Q-C₁₋₆oxoalkyl, Q-C₂₋₆alkenyl, Q-C₃₋₄oxoalkenyl, Q-C₃₋₄oxoalkynyl, Q-C₂₋₄alkynyl, C₃₋₆cycloalkyl, Ar or Het, optionally substituted by one or more of R¹¹;
Q is H, C₃₋₆cycloalkyl, Het or Ar;
R⁶ is W-(CR'₂)_{q}-Z-(CR'R¹⁰)ᵣ-U-(CR'₂)ₛ-V-;
R⁷ is -COR⁸, -COCR'₂R⁹, -C(S)R⁸, -S(O)ₘOR', -S(O)ₘNR'R", -PO(OR'), -PO(OR')₂, -B(OR')₂, -NO₂ and Tet;
R⁸ is -OR', -NR'R", -NR'SO₂R', -NR'OR', -OCR'₂C(O)OR', -OCR'₂OC(O)-R', -OCR'₂C(O)NR'₂, CF₃ or AA1;
R⁹ is -OR', -CN, -S(O)ᵣR', S(O)ₘNR'₂, -C(O)R' C(O)NR'₂ or -CO₂R';
R¹⁰ is H, C₁₋₄alkyl or -NR'R";
R¹¹ is H, halo, -OR¹², -CN, -NR'R¹², -NO₂, -CF₃, CF₃S(O)ᵣ-, -CO₂R', -CONR'₂, Q-C₀₋₆alkyl-, Q-C₁₋₆oxoalkyl-, Q-C₂₋₆alkenyl-, Q-C₂₋₆alkynyl-, Q-C₀₋₆alkyloxy-, Q-C₀₋₆alkylamino- or Q-C₀₋₆alkyl-S(O)ᵣ₋;
R¹² is R', -C(O)R', -C(O)NR'₂, -C(O)OR¹⁵, -S(O)ₘR' or S(O)ₘNR'₂;
R¹⁵ is H, C₁₋₆alkyl or Ar-C₀₋₄alkyl;
R' is H, C₁₋₆alkyl, C₃₋₇cycloalkyl-C₀₋₄alkyl or Ar-C₀₋₄alkyl;
R" is R', -C(O)R' or -C(O)OR¹⁵;
R''' is R" or AA2;
AA1 is an amino acid attached through its amino group and having its carboxyl group optionally protected by replacing the OH of the carboxy group with R⁸, and AA2 is an amino acid attached through its carboxyl group, and having its amino group optionally protected by substituting the amino group with R¹²;
U and V are absent or CO, CR'₂, C(=CR'2), S(O)ₙ, O, NR', CR'OR', CR'(OR")CR'₂, CR'₂CR'(OR"), C(O)CR'₂, CR'₂C(O), CONR', NR'CO, OC(O), C(O)O, C(S)O, OC(S), C(S)NR', NR'C(S), S(O)ₙNR', NR'S(O)ₙ, N=N, NR'NR', NR'CR'₂, CR'₂NR', CR'₂O, OCR'₂, C≡C or CR'=CR', provided that U and V are not simultaneously absent;
W is R'R"'N- or 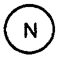 ;

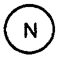 is a nitrogen heterocycle, which may be a saturated or unsaturated stable five-, six- or seven-membered monocyclic ring, or a seven- to ten-membered bicyclic ring containing up to three nitrogen atoms or containing one nitrogen atom and a heteroatom chosen from oxygen and sulfur, substituted in any stable position by R²⁰ or C₁₋₄alkyl substituted by R²⁰, wherein R²⁰ is H, C₁₋₄alkoxy, F, Cl, Br, I, NO₂, NR'₂, OH, CO₂R', CONHR', CF₃, Q-C₀₋₄alkyl, Q-C₁₋₄alkyl-S(O)ᵤ;

Het is an optionally substituted five- or six-membered monocyclic ring, or a nine-or ten-membered bicyclic ring containing one to three heteroatoms chosen from the group of nitrogen, oxygen and sulfur, substituted in any stable position by one to three moieties chosen from R¹¹;

Ar is phenyl or naphthyl, or phenyl or naphthyl substituted by one to three moieties chosen from R¹¹;
Tet is 5-tetrazolyl;
m is 1 or 2;
n is 0 to 3;
q is 0 to 3;
r is 0 to 2;
s is 0 to 2;
t is 0 to 2; and
u is 0, 1 or 2; or
pharmaceutically acceptable salts thereof;
wherein either
(a) Z is non-aromatic Het;
   or
(b) 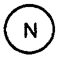 is a 4-substituted, 6-membered nitrogen heterocycle, which may be saturated or unsaturated containing up to three nitrogen atoms or containing one nitrogen atom and a heteroatom chosen from oxygen and sulfur, substituted in any stable position by R²⁰ or C₁₋₄alkyl substituted by R²⁰;
   Z is (CH₂)ₜ;
   V is absent
   U is chosen from NR'CO, CONR', CH₂O, OCH₂, CH₂CH₂, CR'=CR' or C≡C;
   s is 0; and
   q+t+r is 1-3.

In cases wherein the compounds of this invention may have one or more chiral centers, unless specified, this invention includes each unique nonracemic compound which may be synthesized and resolved by conventional techniques. In cases in which compounds have unsaturated carbon-carbon double bonds, both the cis (Z) and trans (E) isomers are within the scope of this invention. In cases wherein compounds may exist in tautomeric forms, such as keto-enol tautomers, such as 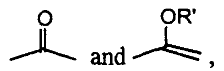 and tautomers of guanidine-type groups, such as 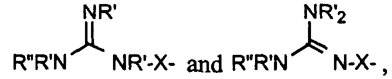 each tautomeric form is contemplated as being included within this invention whether existing in equilibrium or locked in one form by appropriate substitution with R'. The meaning of any substituent at any one occurrence is independent of its meaning, or any other substituent's meaning, at any other occurrence, unless specified otherwise.

Suitably R⁸ is -OR', -OCR'₂C(O)OR, -OCR'₂C(O)NR'₂ or OCR'₂OC(O)-R', preferably -OR'.

Suitably R¹⁰ and R¹¹ are H.

Suitably (CR'R¹⁰)ᵣ-U-(CR'₂)ₛ-V is (CR'R¹⁰)ᵣ-U- or -U-(CR'₂)_{s.}

Suitably U is CO, CONR' or NR'CO.

Preferably, (CR'R¹⁰)ᵣ-U-(CR'₂)ₛ-V is (CH₂)₀₋₂NR'CO, (CH₂)₀₋₂CONR', (CH₂)₀₋₂CO, (CH₂)₀₋₂CH=CH, (CH₂)₀₋₂C≡C, (CH₂)₁₋₃O, or (CH₂)₁₋₅. More preferably, (CR'R¹⁰)ᵣ-U-(CR'₂)ₛ-V is (CH₂)₀₋₂NR'CO or (CH₂)₀₋₂CONR', where R' is H or methyl.

Preferably W is 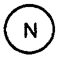 ;

Preferably, Z is piperidinyl, piperazinyl or (CH₂)ₜ. Suitably t is 1.

Particular examples of R⁶ are: 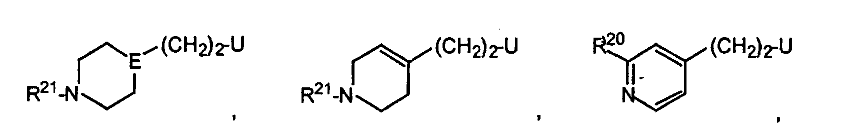 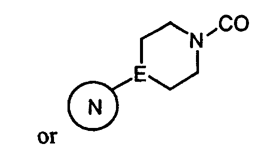 , and R"HN-(CH₂)₅-U wherein E is N or CH , R²⁰ is hydrogen, amino, mono or di-C₁₋₄alkylamino, hydroxy or C₁₋₄alkyl, R²¹ is H or C₁₋₆alkyl and U is NR'CO, CONR', (CH₂)CO, CH=CH, C≡C, CH₂O, OCH₂ and (CH₂)₂.

Preferred illustrative examples of R⁶ are: 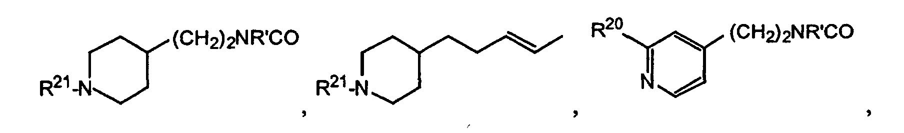 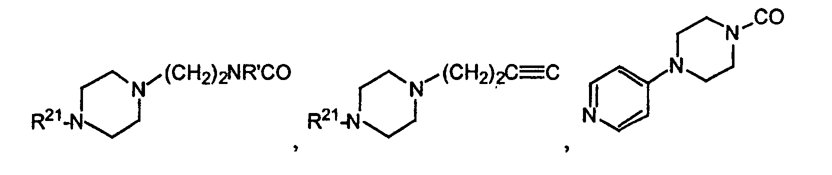 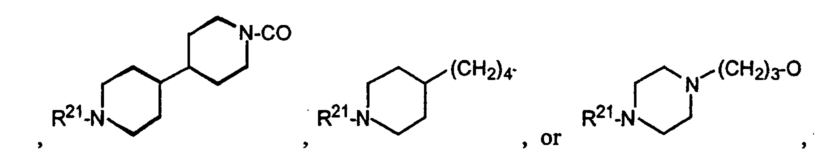 wherein R' and R²¹ are H or C₁₋₄alkyl. Preferably R' is methyl and R²¹ is H.

In a preferred embodiment, A¹ is NR¹, and A⁴ is NR⁴.

In another preferred embodiment, A¹ is CHR¹, and A⁴ is NR⁴.

Preferably R² is CH₂CO₂R', particularly CH₂CO₂H.

Preferably, R¹ and R⁴ are H, C₁₋₄alkyl, Ar-C₁₋₄alkyl or C₃₋₆cycloalkyl-C₁₋₄alkyl. Suitably R¹ is H or methyl and R⁴ is H, methyl, cyclohexylethyl or phenylethyl.

In a more specific preferred embodiment, A¹ is NR^{1'} or CH₂, where R^{1'} is H, C₁₋₄alkyl or C(O)R'; R² is CH₂CO₂H; A⁴ is NR⁴; R⁴ is H, C₁₋₆alkyl, C₃₋₆cycloalkylC₀₋₄alkyl or Ar-C₀₋₄alkyl; Z is a six-membered Het or (CH₂)ₜ; W is R'₂N, or 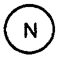 ; (CR'R¹⁰)ᵣ-U-(CR'₂)ₛ-V is (CR'R¹⁰)ᵣ-U- or -U-(CR'₂)ₛ, (*e.g*., V is absent and one of s and r are 0) wherein U is NR'CO, CONR', CR'=CR', C≡C, O, CO or CH₂. Suitably, (CR'R¹⁰)ᵣ-U-(CR'₂)ₛ-V is (CH₂)₀₋₃NR'CO, (CH₂)₀₋₃CONR', where R' is H or methyl, or (CH₂)₀₋₃O. Preferably R' is methyl.

Specific embodiments of this invention, including useful intermediates, are described in Examples 1-43 inclusive.

In the above description of formula (I), W represents a nitrogen-containing group which is capable of making a hydrogen bond. Preferably W is a basic nitrogen moiety. R⁷ represents a group with a non-bonding pair of electrons which is capable of forming a hydrogen bond or chelating with a metal cation. Preferably R⁷ is acidic. It is also preferred that 10-15 (most preferably about 13) intervening covalent bonds via the shortest intramolecular path will exist between the group R⁷ and a terminal basic nitrogen moiety of W for optimal spacing between these groups, and the moieties T, U, V and Z, and the alkyl spacers represented by q, r, s are chosen accordingly. For instance, by way of illustration, but not limitation, when one of R² or R⁴ is (CH₂)₂CO₂H, or preferably CH₂CO₂H, and R⁶ is a substituent in the 7- or 8-position of the benzodiazepine ring system and is W-(CR'₂)_{q}-Z-(CR'R¹⁰)ᵣ-U- (*e.g.*, s is 0 and V is absent), then: when W is 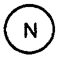 (preferably a 4-substituted six-membered nitrogen heterocycle), and Z is (CH₂)ₜ, and U is chosen from NR'CO, CONR', CH₂O OCH₂, CH₂CH₂, CR'=CR' or C≡C, ('group 1'), suitably q+t+r is 1-3 and preferably q+t+r is 1; when W is 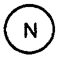 and Z is a six-membered Ar or Het ring (preferably 1,4-disubstituted), and U is 0, CH₂ or CO, q and r are preferably 0; when W is H₂N- and Z is (CH₂)ₜ, and U is chosen from group 1 above, q+r+t is 4-6, preferably 5; when W is H₂N- and Z is a six-membered Ar or Het ring, suitably q+t is 0-2, preferably 1.

Abbreviations and symbols commonly used in the peptide and chemical arts are used herein to describe the compounds of this invention. In general, the amino acid abbreviations follow the IUPAC-IUB Joint Commission on Biochemical Nomenclature as described in *Eur. J. Biochem*., 158, 9 (1984).

Arg refers to arginine, MeArg refers to N^{α}-methyl-arginine, HArg refers to homoarginine, NArg refers to norarginine, (Me₂)Arg refers to N',N"-dimethyl arginine, (Et₂)Arg refers to N',N"-diethyl arginine and Orn refers to omithine. These radicals are suitable components of the substituent R⁶. N^{α}-Substituted derivatives of these amino acid are also useful in this invention. Representative methods for preparing a-substituted derivatives are disclosed in U.S. Patent No. 4,687,758; Cheung *et al., Can. J. Chem.,* 55, 906 (1977); Freidinger *et al., J. Org. Chem., 48,* 77, (1982); and Shuman *et al*., PEPTIDES: PROCEEDINGS OF THE 7TH AMERICAN PEPTIDE SYMPOSIUM, Rich, D., Gross, E., Eds, Pierce Chemical Co., Rockford, III.,617 (1981).

C₁₋₄alkyl as applied herein is meant to include methyl, ethyl, n-propyl, isopropyl, n-butyl, isobutyl and t-butyl. C₁₋₆alkyl additionally includes pentyl, n-pentyl, isopentyl, neopentyl and hexyl and the simple aliphatic isomers thereof. C₀₋₄alkyl and C₀₋₆alkyl additionally indicates that no alkyl group need be present (e.g., that a covalent bond is present).

C₂₋₆alkenyl as applied herein means an alkyl group of 2 to 6 carbons wherein a carbon-carbon single bond is replaced by a carbon-carbon double bond. C₂₋₆alkenyl includes ethylene, 1-propene, 2-propene, 1-butene, 2-butene, isobutene and the several isomeric pentenes and hexenes. Both cis and trans isomers are included.

C₂₋₆ alkynyl means an alkyl group of 2 to 6 carbons wherein one carbon-carbon single bond is replaced by a carbon-carbon triple bond. C₂₋₆ alkynyl includes acetylene, 1-propyne, 2-propyne, 1-butyne, 2-butyne, 3-butyne and the simple isomers of pentyne and hexyne.

C₁₋₄oxoalkyl refers to an alkyl group of up to four carbons wherein a CH₂ group is replaced by a C(O), or carbonyl, group. Substituted formyl, acetyl, 1-propanal, 2-propanone, 3-propanal, 2-butanone, 3-butanone, 1- and 4-butanal groups are representative. C₁₋₆oxoalkyl includes additionally the higher analogues and isomers of five and six carbons substituted by a carbonyl group. C₃₋₆oxoalkenyl and C₃₋-₆oxoalkynyl refers to a C₃₋₆alkenyl or C₃₋₆alkynyl group wherein a CH₂ group is replaced by C(O) group. C₃₋₄oxoalkenyl includes 1-oxo-2-propenyl, 3-oxo-1-propenyl, 2-oxo-3-butenyl and the like.

A substituent on a C₁₋₆alkyl, C₂₋₆alkenyl, C₂₋₆alkynyl or C₁₋₆oxoalkyl group, such as R¹¹, may be on any carbon atom which results in a stable structure, and is available by conventional synthetic techniques.

Q-C₁₋₆ alkyl refers to a C₁₋₆ alkyl group wherein in any position a carbon-hydrogen bond is replaced by a carbon-Q bond. Q-C₂₋₆ alkenyl and Q-C₂₋₆ alkynyl have a similar meaning with respect to C₂₋₆ alkenyl and C₂₋₆ alkynyl.

Ar, or aryl, as applied herein, means phenyl or naphthyl, or phenyl or naphthyl substituted by one to three moieties R¹¹. In particular, R¹¹ may be C₁₋₄alkyl, C₁₋₄alkoxy, C₁₋₄alkthio, trifluoroalkyl, OH, F, Cl, Br or I.

Het, or heterocycle, indicates an optionally substituted five or six membered monocyclic ring, or a nine or ten-membered bicyclic ring containing one to three heteroatoms chosen from the group of nitrogen, oxygen and sulfur, which are stable and available by conventional chemical synthesis. Illustrative heterocycles are benzofuryl, benzimidazole, benzopyran, benzothiophene, furan, imidazole, indoline, morpholine, piperidine, piperazine, pyrrole, pyrrolidine, tetrahydropyridine, pyridine, thiazole, thiophene, quinoline, isoquinoline, and tetra- and perhydro- quinoline and isoquinoline. A six membered ring heterocycle containing one or two nitrogens, such as piperidine, piperazine, tetrahydropyridine and pyridine, are preferred heterocycles for the moiety Z. Any accessible combination of up to three substituents, such as chosen from R¹¹, on the Het ring that is available by chemical synthesis and is stable is within the scope of this invention. A six membered monocyclic ring heterocycle containing one or two nitrogens, such as piperidine, piperazine, tetrahydropyridine and pyridine, are preferred heterocycles for the moiety Z.

C₃₋₇cycloalkyl refers to an optionally substituted carbocyclic system of three to seven carbon atoms, which may contain up to two unsaturated carbon-carbon bonds. Typical of C₃₋₇cycloalkyl are cyclopropyl, cyclobutyl, cyclopentyl, cyclopentenyl, cyclohexyl, cyclohexenyl and cycloheptyl. Any combination of up to three substituents, such as chosen from R¹¹, on the cycloalkyl ring that is available by conventional chemical synthesis and is stable, is within the scope of this invention.

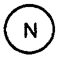 as used herein indicates a nitrogen heterocycle, which may be a saturated or unsaturated stable five-, six- or seven-membered monocyclic ring, or a seven- to ten-membered bicyclic ring containing up to three nitrogen atoms or containing one nitrogen atom and a heteroatom chosen from oxygen and sulfur, and which may be substituted on any atom that results in a stable structure. The nitrogen atom in such ring may be substituted so as to result in a quaternary nitrogen. The nitrogen heterocycle may be substituted in any stable position by R²⁰, for instance H, C₁₋₄alkoxy, F, Cl, Br, I, NO₂, NR'₂, OH, CO₂R', CONHR', CF₃, Q-C₀₋₄alkyl, Q-C₁₋₄alkyl-S(O)ᵤ (e.g., where u is 0, 1 or 2) or C₁₋₄alkyl substituted by any of the aforementioned substituents. Representative of 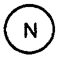 are pyrroline, pyrrolidine, imidazole, imidazoline, imidazolidine, pyrazole, pyrazoline, pyrazolidine, piperidine, piperazine, morpholine, pyridine, pyridinium, tetrahydropyridine, tetrahydro- and hexahydro-azepine, quinuclidine, quinuclidinium, quinoline, isoquinoline, and tetra- and perhydro-quinoline and isoquinoline. In particular, 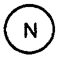 may be pyridyl, pyrolidinyl, piperidinyl, piperazinyl, azetidinyl, quinuclidinyl or tetrahydropyridinyl. 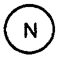 is preferably 4-pyridyl, 4-(2-amino-pyridyl), 4-tetrahydropyridyl, 4-piperidinyl or 4-piperazinyl.

AA1 as referred to herein is an amino acid with its carboxyl group optionally protected, wherein the amino acid may be any of the natural α-amino acids or penicillamine. The unprotected carboxyl group is a free carboxylic acid group. Protecting groups for the carboxyl are esters or amides which are formed, for instance, when the OH of the carboxy group is replaced by R⁸. AA2 is an amino acid, as above, with its amino group optionally protected. Amino protecting groups are well known in the art, for instance, when the amino group is substituted by R¹². An unprotected amino group is a free NH₂ group.

C(O) indicates a carbon doubly bonded to oxygen (*e.g.*, carbonyl), C(S) indicates a carbon doubly bonded to sulfur (e.g., thiocarbonyl).

t-Bu refers to the tertiary butyl radical, Boc refers to the t-butyloxycarbonyl radical, Fmoc refers to the fluorenylmethoxycarbonyl radical, Ph refers to the phenyl radical, Cbz refers to the benzyloxycarbonyl radical, BrZ refers to the o-bromobenzyloxycarbonyl radical, C1Z refers to the o-chlorobenzyloxycarbonyl radical, Bzl refers to the benzyl radical, 4-MBzl refers to the 4-methyl benzyl radical, Me refers to methyl, Et refers to ethyl, Ac refers to acetyl, Alk refers to C₁₋₄alkyl, Nph refers to 1- or 2-naphthyl and cHex refers to cyclohexyl. Tet refers to 5-tetrazolyl.

DCC refers to dicyclohexylcarbodiimide, DMAP refers to dimethylaminopyridine, DIEA refers to diisopropylethyl amine, EDC refers to N-ethyl-N'(dimethylaminopropyl)-carbodiimide.HOBt refers to 1-hydroxybenzotriazole, THF refers to tetrahydrofuran, DIEA refers to diisopropylethylamine, DMF refers to dimethyl formamide, NBS refers to N-bromo-succinimide, Pd/C refers to a palladium on carbon catalyst, PPA refers to 1-propanephosphonic acid cyclic anhydride, DPPA refers to diphenylphosphoryl azide, BOP refers to benzotriazol-1-yloxy-tris(dimethylamino)phosphonium hexafluorophosphate, HF refers to hydrofluoric acid, TEA refers to triethylamine, TFA refers to trifluoroacetic acid, PCC refers to pyridinium chlorochromate.

The compounds of formula (I) are generally prepared by reacting a compound of the formula (XI) with a compound of the formula (XII): 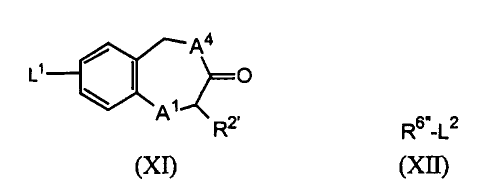 wherein A¹, A⁴, U, V, Z, R', R¹⁰, q, s and r are as defined in formula (I), with any reactive functional groups protected;
L¹ and L² are functional groups which are capable of reacting to form the linkage -(CR'R¹⁰)ᵣ-U-(CR'₂)ₛ-V-;
R^{6"} is W'-(CR'₂)_{q}-Z- and any portion of the group -(CR'R¹⁰)ᵣ-U-(CR'₂)ₛ-V- which is connected to L², with any reactive functional groups protected;
R^{2'} is R² as defined for formula (I) with any reactive group protected; and
W' is W as defined for formula (I) with any basic nitrogen group protected; to form a compound of the formula: 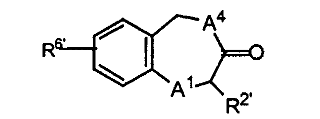 wherein R^{6'} is W'-(CR'₂)_{q}-Z-(CR'R¹⁰)ᵣ-U-(CR'₂)ₛ-V-;
and thereafter removing any protecting groups, and optionally forming a pharmaceutically acceptable salt.

It will be apparent that the precise identity of L¹ and L² will be dependent upon the site of the linkage being formed. General methods for preparing the linkage -(CR'R¹⁰)ᵣ-U-(CR'₂)ₛ-V- are described, for example, in EP-A 0 372 486 and EP-A 0 381 033 and EP-A 0 478 363.

For instance, if V is CONH, L¹ may be -NH₂, L² may be OH (as in an acid) or Cl (as in an acid chloride), and R^{6"} may be W-(CR'₂)_{q}-Z-(CR'R¹⁰)ᵣ-U-(CR'₂)ₛ-C(O), with any functional groups optionally protected. For example, R^{6"} may be (benzyloxycarbonyl-amidino)benzoyl- or (N^{α}-Boc,N^{guan}-Tos)arginyl-. When L² is OH, a coupling agent is used.

Similarly, if V is NHCO, L¹ may be -CO₂H or CO-Cl, L² may be -NH₂, and R^{6"} may be W-(CR'₂)_{q}-Z-(CR'R¹⁰)ᵣ-U-(CR'₂)ₛ-. For example, R^{6"} may be (benzyloxycarbonylamidino)-phenyl, (benzyloxycarbonylamino)methylbenzyl- or 6-(benzyloxycarbonylamino)hexyl-.

Where V is NHSO₂, L¹ may be SO₂Cl, L² may be -NH₂ and R^{6"} may be as above. Where V is SO₂NH, L¹ may be -NH₂ and L² may be SO₂Cl. Methods to prepare such sulfonyl chlorides are disclosed, for instance, in *J. Org. Chem.,* 23, 1257 (1958).

If V is CH=CH, L¹ may be -CHO, L² may be CH=P-Ph₃ and R^{6"} may be W-(CR'₂)_{q}-Z-(CR'R¹⁰)ᵣ-U-(CR'₂)ₛ-. Alternately, L¹ may be CH=P-Ph₃, L² may be CHO, *e.g.*, R^{6"} may be W-(CR'₂)_{q}-Z-(CR'R¹⁰)ᵣ-U-(CR'₂)ₛ₋₁-CHO.

Where V is CH₂CH₂, compounds may be obtained by reduction of a suitably protected compound wherein V is CH=CH.

Where V is CH₂O, CH₂N or C≡C, L¹ may be -OH, -NH or -C≡C H,respectively; L² may be -Br; and R^{6"} may be W-(CR'₂)_{q}-Z-(CR'R¹⁰)ᵣ-U-(CR'₂)ₛ-. For example, R^{6"} may be (benzyloxycarbonylamino)-methylbenzyl- or 2-(N-benzyl-4-piperidinyl)-ethyl. Similarly where U or V is OCH₂, NR'CH₂ or C≡C, L¹ may be -CH₂Br and L² may be -OH, -NH or -C≡C H, respectively, Alternately, when U or V is C≡C, L¹ may be Br, I or CF₃SO₃, L² may be C≡C H and the coupling may be catalyzed by palladium and a base.

Compounds wherein V is CHOHCH₂ may be prepared from a suitably protected compound where V is CH=CH by the procedure disclosed in *J. Org. Chem.,* 54, 1354 (1989).

Compounds wherein V is CH₂CHOH may be obtained from a suitably protected compound where V is CH=CH by hydroboration and basic oxidation as disclosed in *Tet. Lett.,* 31, 231 (1990).

The compounds of formula (XI), are benzodiazepines and benzazepines and are prepared by the general methods illustrated by Schemes 1-8. Representative methods for preparing benzodiazepines are well known in the art (*e.g.,* Hynes, *et al*., *J. Het. Chem.,* 1988, 25, 1173; Muller, *et al*., *Helv. Chim. Acta*., 1982, 65, 2118; Mori, *et al*., *Heterocycles,* 1981, 16, 1491); and WO 93/00095. Scheme 9 is illustrative of a method to prepare benzothiazepines. Benzoxazepines may be prepared in an analogous manner by starting with the compound wherein S is replaced by O. In the Schemes, R^{1"}-R^{7"} indicate R¹-R⁷ or a suitable precursor thereof, wherein any functional groups are protected as known in the art.

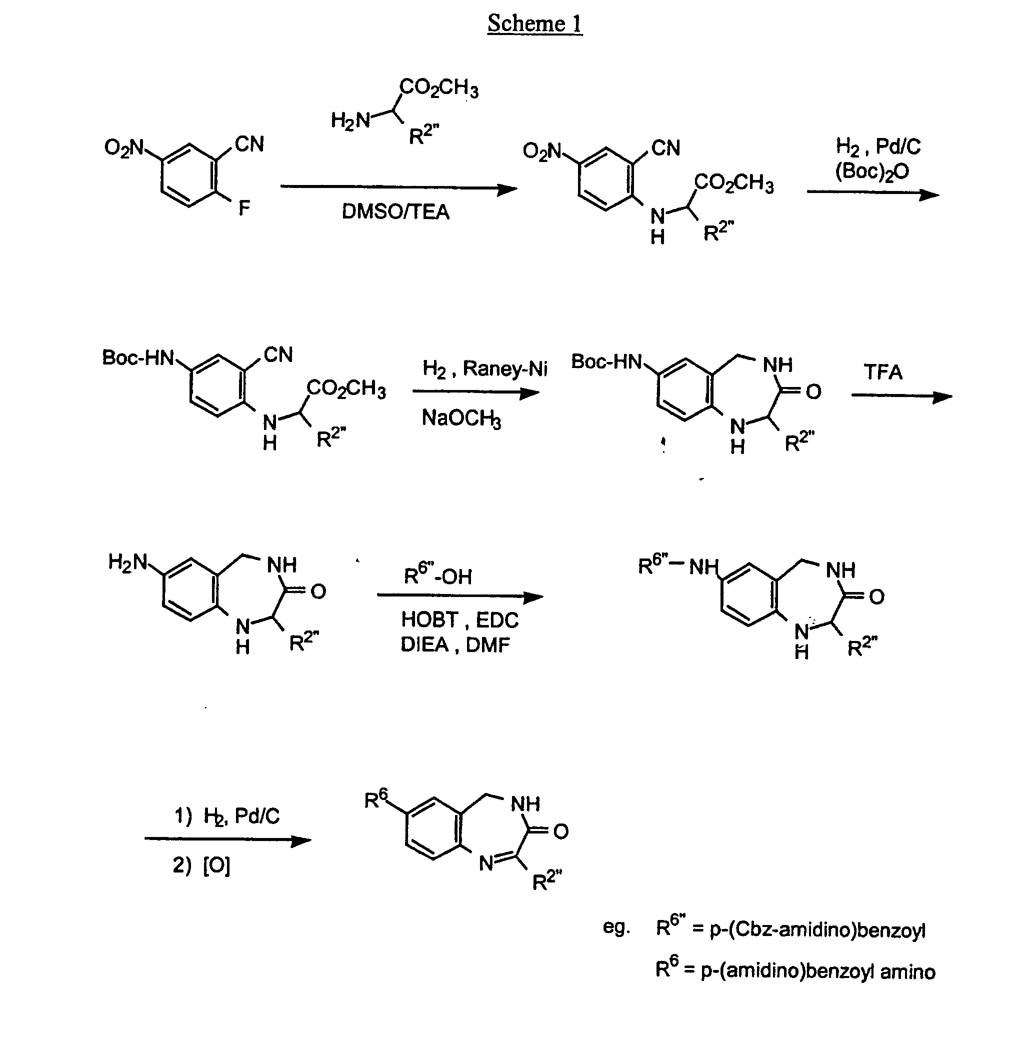

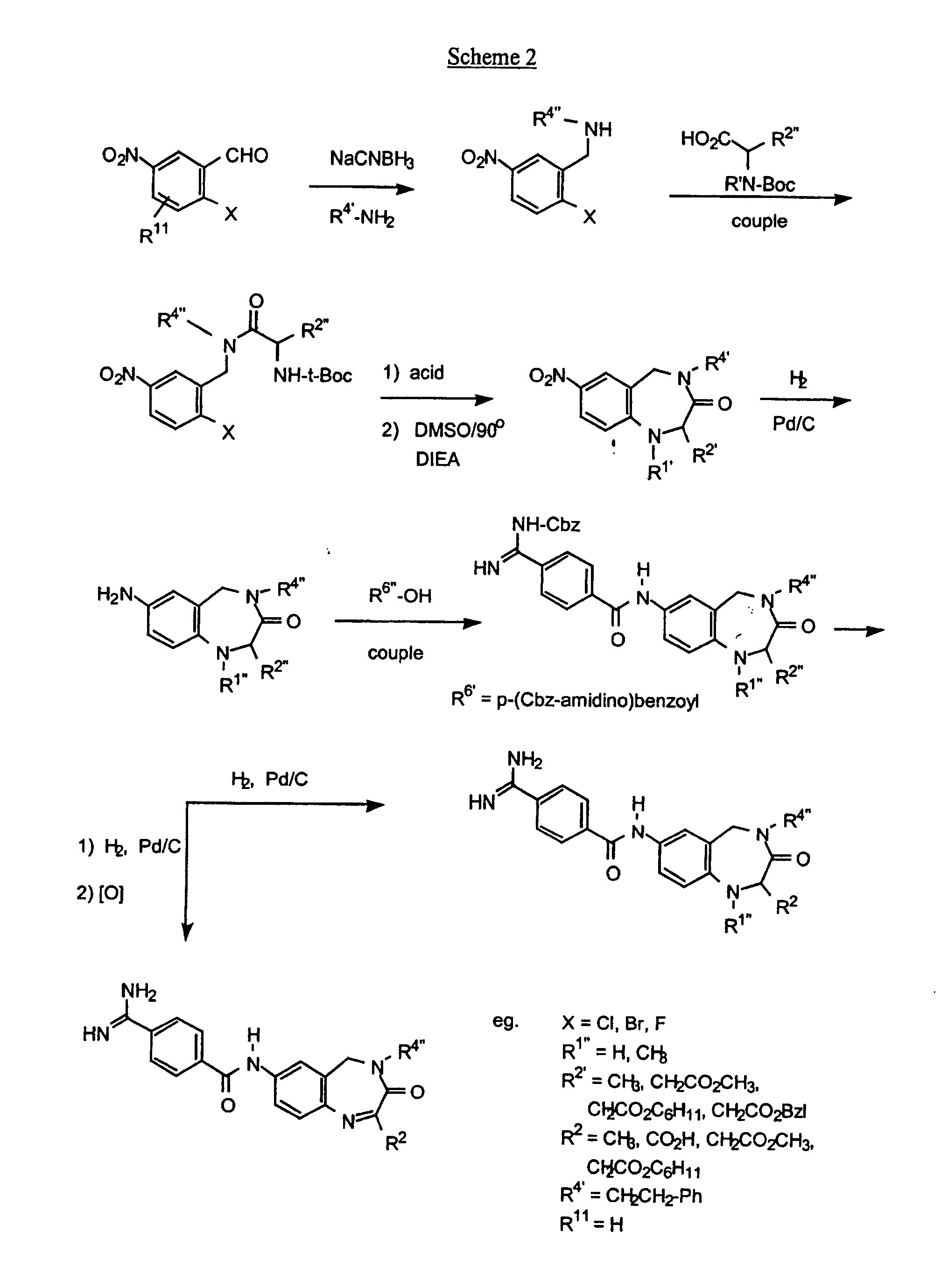

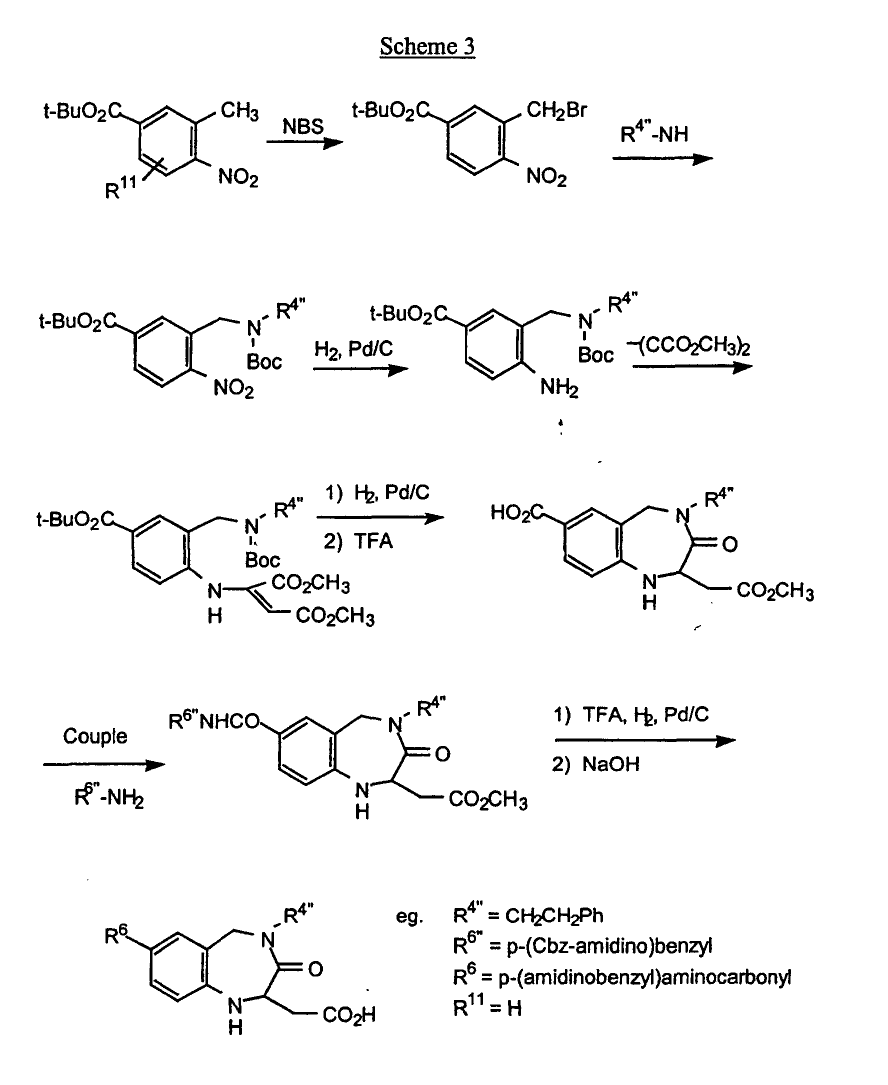

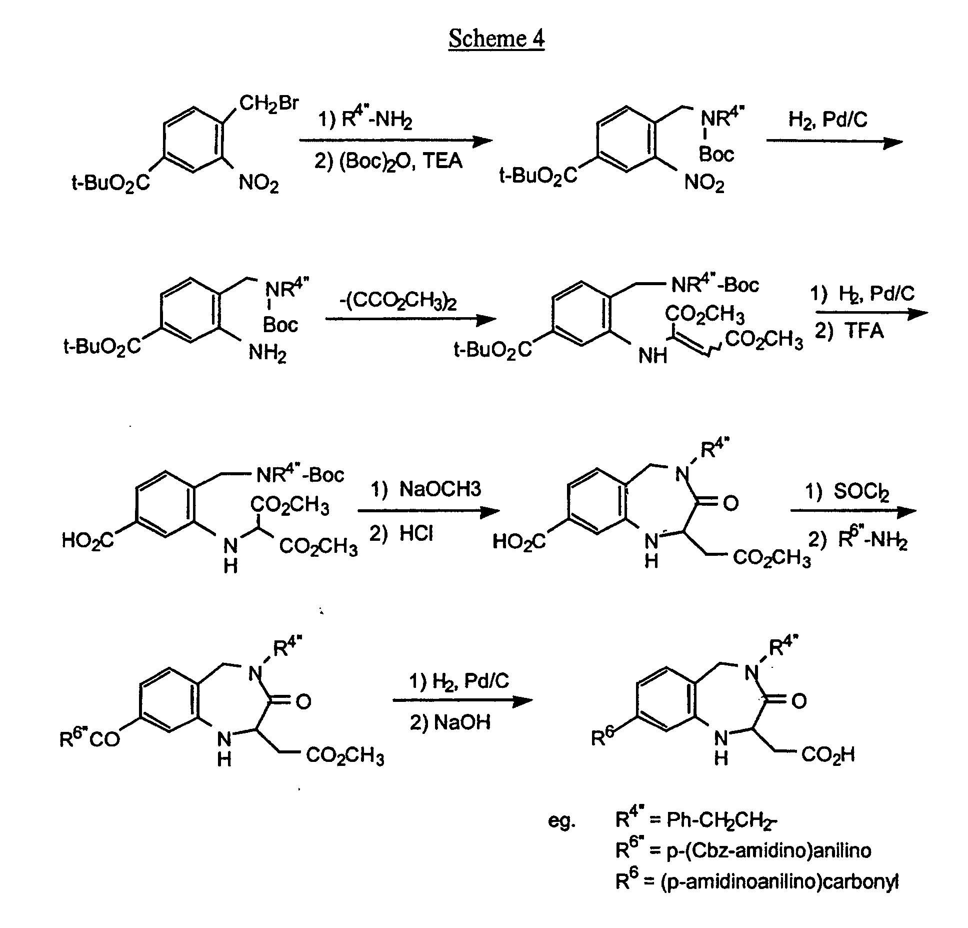

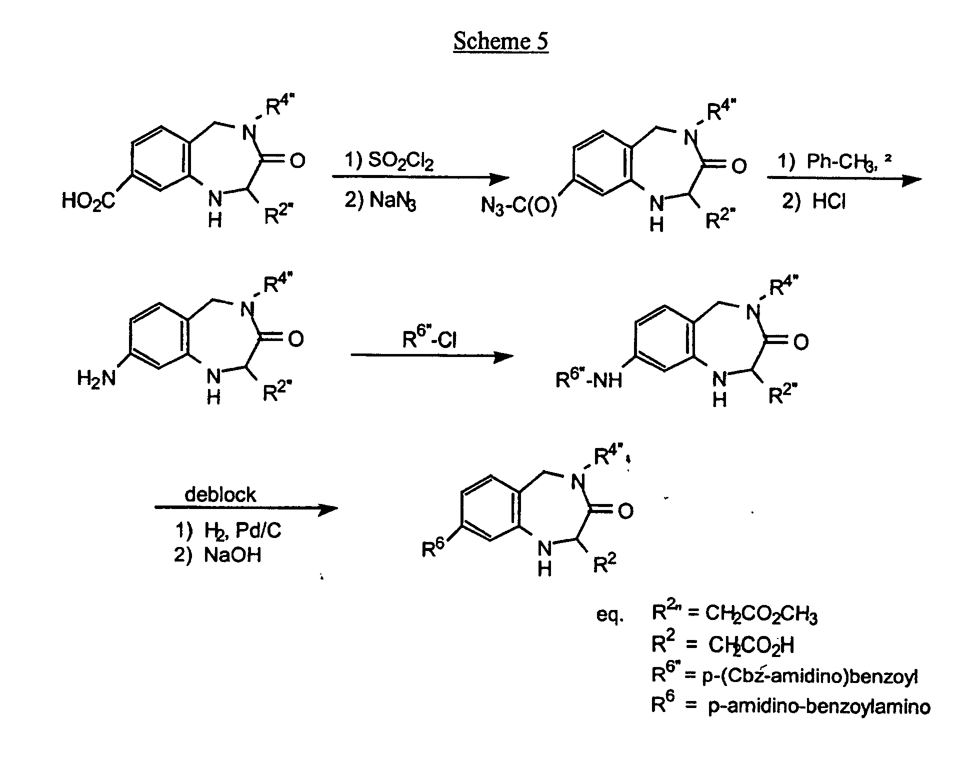

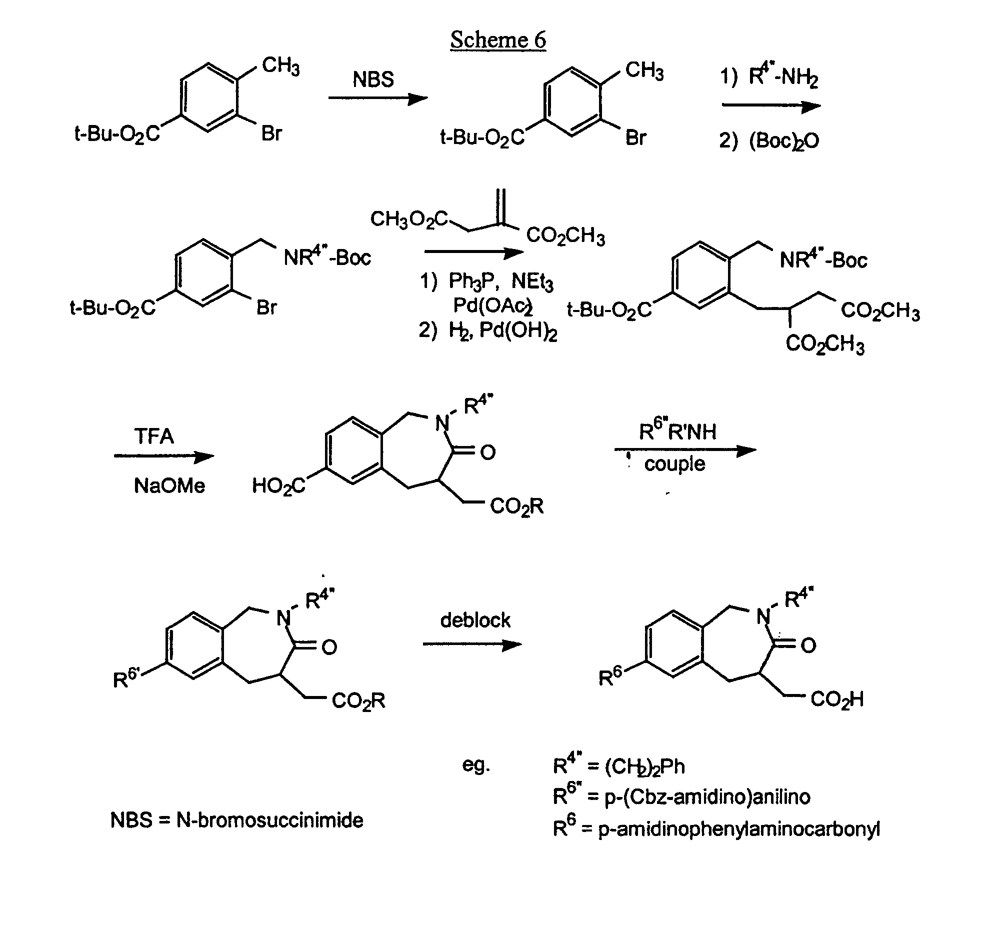

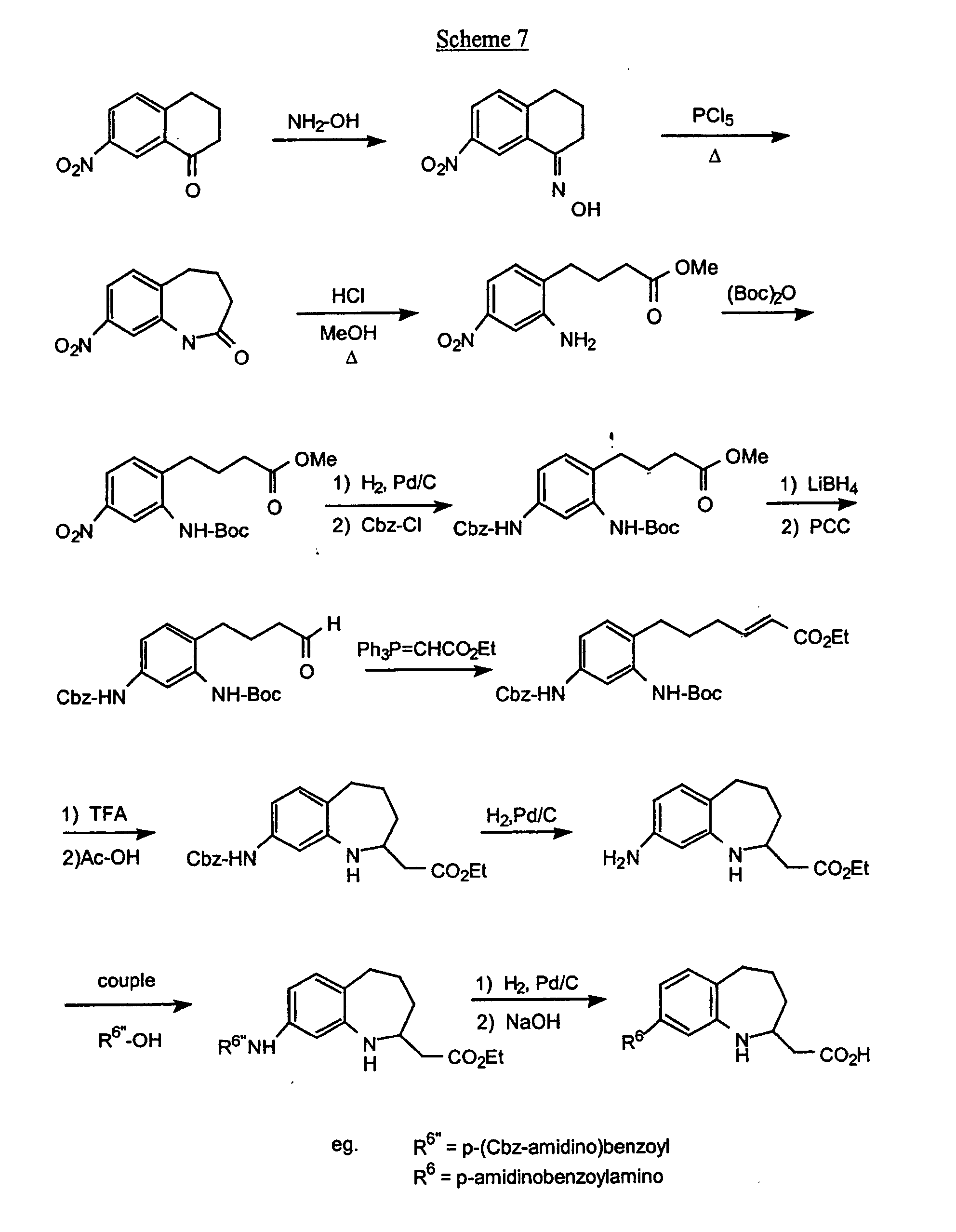

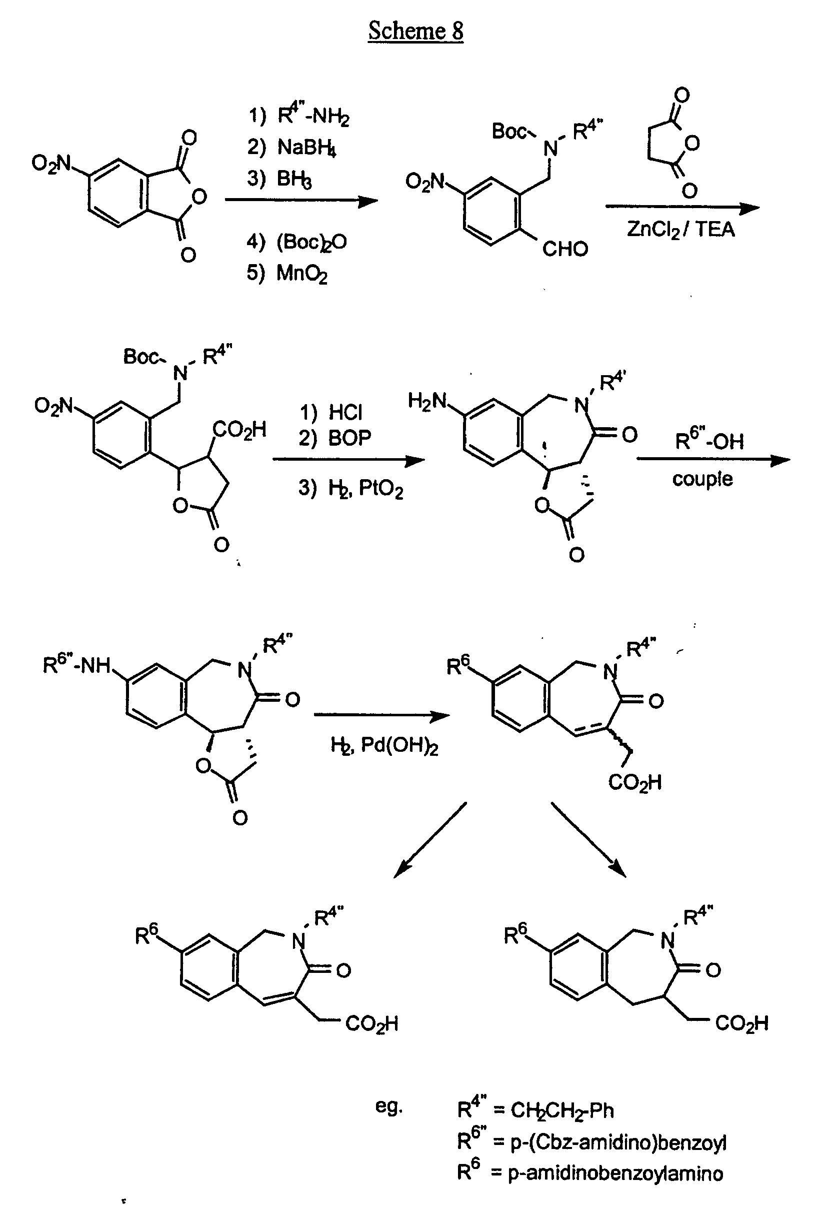

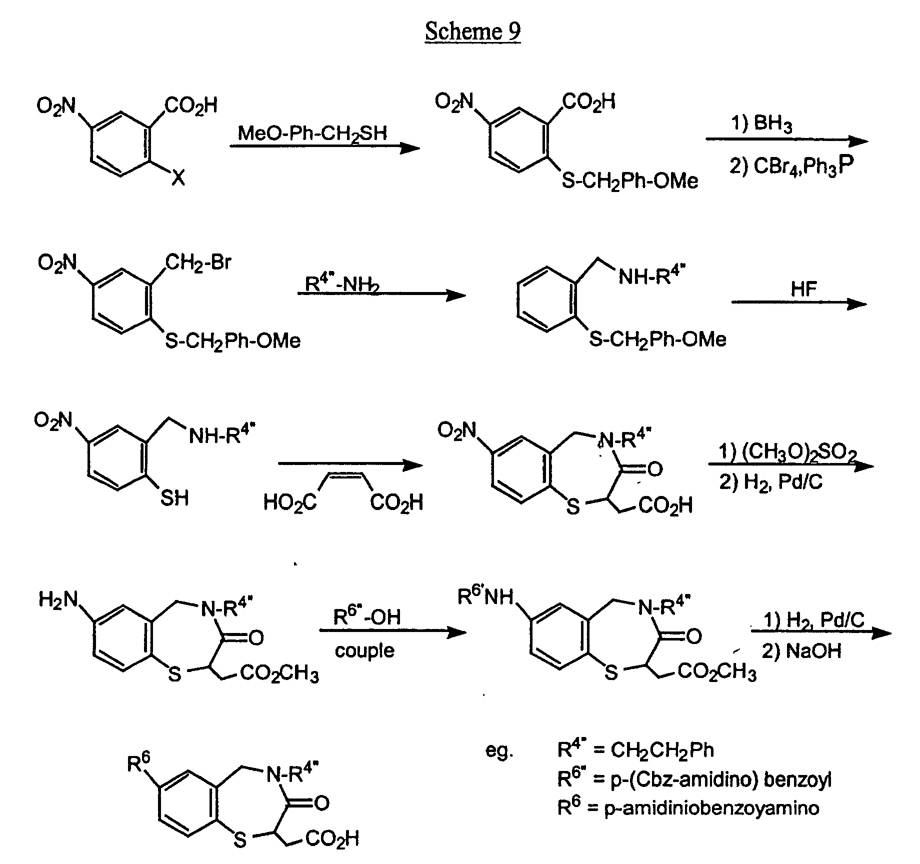

A particularly useful intermediate is the 1,4-benzodiazepine compound of formula (XI), wherein A¹ is NR¹, A⁴ is NR⁴;; L¹ is CHO, CO₂R', Br, I, OH, CF₃SO₃, CH₂-T or NR'R", and T is OH, NHR", Cl, Br or I. In particular, compounds wherein R¹ is H, C₁₋₄alkyl, C₁₋₄oxoalkyl; R² is CH₂CO₂R' and R⁴ is Q-C₁₋₆alkyl are useful. More particularly, compounds wherein R⁴ is H, C₁₋₄alkyl or phenylC₁₋₄alkyl are useful. Other useful intermediates are similarly substituted benzazepine compounds of formula (XI), where A¹ is CHR¹ andA⁴ is NR⁴.

Coupling reagents as used herein denote reagents which may be used to form peptide bonds. Typical coupling methods employ carbodiimides, activated anhydrides and esters and acyl halides. Reagents such as EDC, DCC, DPPA, PPA, BOP reagent, HOBt, N-hydroxysuccinimide and oxalyl chloride are typical.

Coupling methods to form peptide bonds are generally well known to the art. The methods of peptide synthesis generally set forth by Bodansky *et al*., THE PRACTICE OF PEPTIDE SYNTHESIS, Springer-Verlag, Berlin, 1984, Ali *et al*. in *J. Med. Chem*., 29, 984 (1986) and *J. Med. Chem.,* 30, 2291 (1987) are generally illustrative of the technique.

Solution synthesis for the formation of amide or peptide bonds is accomplished using conventional methods used to form amide bonds. Typically, the amine or aniline is coupled via its free amino group to an appropriate carboxylic acid substrate using a suitable carbodiimide coupling agent, such as N,N' dicyclohexyl carbodiimide (DCC), optionally in the presence of catalysts such as 1-hydroxybenzotriazole (HOBt) and dimethylamino pyridine (DMAP). Other methods, such as the formation of activated esters, anhydrides or acid halides, of the free carboxyl of a suitably protected acid substrate, and subsequent reaction with the free amine of a suitably protected amine, optionally in the presence of a base, are also suitable. For example, a protected Boc-amino acid or Cbz-amidino benzoic acid is treated in an anhydrous solvent, such as methylene chloride or tetrahydrofuran(THF), in the presence of a base, such as N-methyl morpholine, DMAP or a trialkylamine, with isobutyl chloroformate to form the "activated anhydride", which is subsequently reacted with the free amine-of a second protected amino acid or aniline.

Compounds of formula (XII) are prepared by conventional methods known in the art from commercially available materials. W is a generally a basic functional group attached to Z, optionally via an alkyl chain, and is protected during the synthesis of R⁶ or is introduced into the molecule after the -(CR'R¹⁰)ᵣ-U-(CR'₂)ₛ-V- linkage has been formed. For example, compounds of formula (XII) or formula (I) wherein W is a suitably substituted R'R"N-, R"R'NC(=NR'), R'₂N(R¹³)C=N-, R"N=(R¹³)C-NR'-, R'₂N(R'₂N)C=N- or R"R'N(R'N=)C-NR', are prepared by conventional methods including those disclosed in EP-A 0 372 486, EP-A 0 381 033 or EP-A 0 478 363.

Compounds of formula (XII) wherein W is 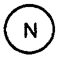 are prepared, *inter alia*, by methods disclosed in EP-A 0 478 363.

Compounds wherein W is R'₂N(R'₂N)C=N-X- or R"R'N(R'N=)C-NR'-X-, and X is O are prepared, *inter alia*, by methods disclosed in *J. Org. Chem.,* 51, 5047 (1986).

Compounds wherein W is R'₂N(R'₂N)C=N-X- or R"R'N(R'N=)C-NR'-X-, and X is N=CR', are prepared, *inter alia,* by methods disclosed in United States Patent 3,714,253 and *Eur. J. Med. Chem.-Chim. Ther.,* 20, 25 (1985).

Compounds wherein W isR'₂N(R'₂N)C=N-X- or R"R'N(R'N=)C-NR'-X-, and X is C(O), are prepared, *inter alia,* by methods disclosed in United States Patent 3,714,253 and *Can. J. Chem.,* 43, 3103 (1965).

Compounds wherein W is R'ONR'C(=NR')- may be prepared, *inter alia*, by methods disclosed in *J. Het. Chem.,* 16, 1063 (1979) or *J. Het. Chem.,* 26, 125 (1989).

Compounds wherein W is R'₂NR'NC(=NR')- are prepared by conventional methods including those disclosed in *Synthesis,* 583 (1974).

Compounds wherein W is R'R''NR'N- are prepared, *inter alia,* by methods disclosed in *J. Prakt. Chem.,* 36, 29 (1967).

Compounds wherein W is R'R''NR'NCO- are prepared, *inter alia,* by methods disclosed in *Bull. Chem. Soc. Jpn*., 43, 2257 (1970).

Compounds wherein W is R''R'NC(=NR')Y, and Y is S, are prepared, *inter alia,* by methods disclosed in *Chem. Lett.,* 1379 (1986).

Compounds of formula (XII) or formula (I), wherein W is R"R'NC(=NR')Y and Y is O, are prepared by conventional methods including those disclosed in Japanese Patent 2022751.

Useful intermediates of formula (XII) include compounds of the formula W'-(CR'₂)_{q}-Z-(CR'R¹⁰)ᵣ-U-(CR'₂)ₛ-L², wherein Z, R', R", R¹⁰, U, q, r, and s are as defined for formula (I); L² is CHO, CO₂R', C≡C-H, OH, Cl, Br, I, CH₂-T or NR'R", and T is CF₃SO₃, OH, NHR", Cl, Br or I; and W' is W with any reactive basic nitrogen group protected as herein described by RP, a nitrogen protecting group. R'SO₂, R'OCO and R'CO (*e.g.*, Tos, Boc, Cbz or acetyl) are typical nitrogen protecting groups. Particular examples of such intermediates are: 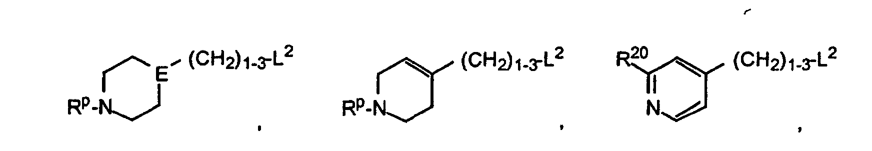 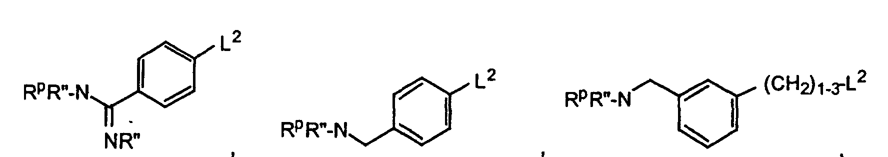 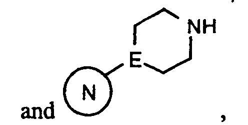 wherein E is N or CH, R²⁰ is hydrogen, amino, mono or di-C₁₋-₄alkylamino, hydroxy or C₁₋₄alkyl.

The reactive functional groups of the sidechains of each synthetic fragment are suitably protected as known in the art. Suitable protective groups are disclosed in Greene, PROTECTIVE GROUPS IN ORGANIC CHEMISTRY, John Wiley and Sons, New York, 1981. For example, the Boc, Cbz, phthaloyl or Fmoc group may be used for protection of an amino or amidino group. The Boc group is generally preferred for protection of an α-amino group. A t-Bu, cHex or benzyl ester may be used for the protection of the side chain carboxyl. A benzyl group or suitably substituted benzyl group (*e.g*., 4-methoxy-benzyl or 2,4-dimethoxy-benzyl) is used to protect the mercapto group or the hydroxyl group. The tosyl group may be used for protection of the imidazolyl group and tosyl or nitro group for protection of the guanidino group. A suitably substituted carbobenzyloxy group or benzyl group may be also be used for the hydroxyl group or amino group. Suitable substitution of the carbobenzyloxy or benzyl protecting groups is ortho and/or para substitution with chloro, bromo, nitro or methyl, and is used to modify the reactivity of the protective group. Except for the Boc group, the protective groups for the amino moiety are, most conveniently, those which are not removed by mild acid treatment. These protective groups are removed by such methods as catalytic hydrogenation, sodium in liquid ammonia or HF treatment, as known in the art.

Modification of amino groups especially on the six-membered ring of the bicyclic system, may be accomplished by alkylation, sulfonylation, cyanation or acylation as is generally known in the art.

Acid addition salts of the compounds are prepared in a standard manner in a suitable solvent from the parent compound and an excess of an acid, such as hydrochloric, hydrobromic, sulfuric, phosphoric, acetic, maleic, succinic or methanesulfonic. The acetate salt form is especially useful. Certain of the compounds form inner salts or zwitterions which may be acceptable. Cationic salts are prepared by treating the parent compound with an excess of an alkaline reagent, such as a hydroxide, carbonate or alkoxide, containing the appropriate cation; or with an appropriate organic amine. Cations such as Li+, Na+, K+, Ca++, Mg++ and NH₄+ are specific examples of cations present in pharmaceutically acceptable salts.

This invention provides a pharmaceutical composition which comprises a compound according to formula (I) and a pharmaceutically acceptable carrier. Accordingly, the compounds of formula (I) may be used in the manufacture of a medicament. Pharmaceutical compositions of the compounds of formula (I) prepared as hereinbefore described may be formulated as solutions or lyophilized powders for parenteral administration. Powders may be reconstituted by addition of a suitable diluent or other pharmaceutically acceptable carrier prior to use. The liquid formulation may be a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

Alternately, these compounds may be encapsulated, tableted or prepared in a emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

For rectal administration, the compounds of this invenfion may also be combined with excipients such as cocoa butter, glycerin, gelatin or polyethylene glycols and molded into a suppository.

The compounds of this invention may be used *in vitro* to inhibit the aggregation of platelets in blood and blood products, *e.g.,* for storage, or for *ex vivo* manipulations such as in diagnostic or research use.

A method of inhibiting platelet aggregation and clot formation in a mammal, especially a human, comprises the internal administration of a compound of formula (I) and a pharmaceutically acceptable carrier. Indications for such therapy include acute myocardial infarction (AMI), deep vein thrombosis, pulmonary embolism, dissecting anurysm, transient ischemia attack (TIA), stroke and other infarct-related disorders, and unstable angina. Chronic or acute states of hyper-aggregability, such as disseminated intravascular coagulation (DIC), septicemia, surgical or infectious shock, post-operative and post-partum trauma, cardiopulmonary bypass surgery, incompatible blood transfusion, abruptio placenta, thrombotic thrombocytopenic purpura (TTP), snake venom and immune diseases, are likely to be responsive to such treatment. In addition, the compounds of this invention may be useful in a method for the prevention of metastatic conditions, the prevention or treatment of fungal or bacterial infection, inducing immunostimulation, treatment of sickle cell disease, and the prevention or treatment of diseases in which bone resorption is a factor.

The compound is administered either orally or parenterally to the patient, in a manner such that the concentration of drug in the plasma is sufficient to inhibit platelet aggregation, or other such indication. The pharmaceutical composition containing the compound is administered at a dose between about 0.2 to about 50 mg/kg in a manner consistent with the condition of the patient. For acute therapy, parenteral administration is preferred. For persistent states of hyperaggregability, an intravenous infusion of the compound in 5% dextrose in water or normal saline is most effective, although an intramuscular bolus injection may be sufficient.

For chronic, but noncritical, states of platelet aggregability, oral administration of a capsule or tablet, or a bolus intramuscular injection is suitable. The compound is administered one to four times daily at a level of about 0.4 to about 50 mg/kg to achieve a total daily dose of about 0.4 to about 200 mg/kg/day.

A method for inhibiting the reocclusion of an artery or vein following fibrinolytic therapy comprises internal administration of a compound of formula (I) and a fibrinolytic agent. It has been found that administration of a compound in fibrinolytic therapy either prevents reocclusion completely or prolongs the time to reocclusion.

When used in the context of this invention the term fibrinolytic agent is intended to mean any compound, whether a natural or synthetic product, which directly or indirectly causes the lysis of a fibrin clot. Plasminogen activators are a well known group of fibrinolytic agents. Useful plasminogen activators include, for example, anistreplase, urokinase (UK), pro-urokinase (pUK), streptokinase (SK), tissue plasminogen activator (tPA) and mutants, or variants, thereof, which retain plasminogen activator activity, such as variants which have been chemically modified or in which one or more amino acids have been added, deleted or substituted or in which one or more or functional domains have been added, deleted or altered such as by combining the active site of one plasminogen activator with the fibrin binding domain of another plasminogen activator or fibrin binding molecule. Other illustrative variants include tPA molecules in which one or more glycosylation sites have been altered. Preferred among plasminogen activators are variants of tPA in which the primary amino acid sequence has been altered in the growth factor domain so as to increase the serum half-life of the plasminogen activator. tPA Growth factor variants are disclosed, *e.g.,* by Robinson *et al*., EP-A 0 297 589 and Browne *et al*., EP-A 0 240 334. Other variants include hybrid proteins, such as those disclosed in EP 0 028 489, EP 0 155 387 and EP 0 297 882. Anistreplase is a preferred hybrid protein for use in this invention. Fibrinolytic agents may be isolated from natural sources, but are commonly produced by traditional methods of genetic engineering.

Useful formulations of tPA, SK, UK and pUK are disclosed, for example, in EP-A 0 211 592, EP-A 0 092 182 and U.S. Patent 4,568,543. Typically the fibrinolytic agent may be formulated in an aqueous, buffered, isotonic solution, such as sodium or ammonium acetate or adipate buffered at pH 3.5 to 5.5. Additional excipients such as polyvinyl pyrrolidone, gelatin, hydroxy cellulose, acacia, polyethylene, glycol, mannitol and sodium chloride may also be added. Such a composition can be lyophilized.

The pharmaceutical composition may be formulated with both the compound of formula (I) and fibrinolytic in the same container, but formulation in different containers is preferred. When both agents are provided in solution form they can be contained in an infusion/injection system for simultaneous administration or in a tandem arrangement.

Indications for such therapy include myocardial infarction, deep vein thrombosis, pulmonary embolism, stroke and other infarct-related disorders. The compound is administered just prior to, at the same time as, or just after parenteral administration of tPA or other fibrinolytic agent. It may prove desirable to continue treatment with the compound for a period of time well after reperfusion has been established to maximally inhibit post-therapy reocclusion. The effective dose of tPA, SK, UK or pUK may be from 0.5 to 5 mg/kg and the effective dose of the compound may be from about 0.1 to 25 mg/kg.

For convenient administration of the inhibitor and the fibrinolytic agent at the same or different times, a kit is prepared, comprising, in a single container, such as a box, carton or other container, individual bottles, bags, vials or other containers each having an effective amount of the inhibitor for parenteral administration, as described above, and an effective amount of tPA, or other fibrinolytic agent, for parenteral administration, as described above. Such kit can comprise, for example, both pharmaceutical agents in separate containers or the same container, optionally as lyophilized plugs, and containers of solutions for reconstitution. A variation of this is to include the solution for reconstitution and the lyophilized plug in two chambers of a single container, which can be caused to admix prior to use. With such an arrangement, the fibrinolytic and the compound may be packaged separately, as in two containers, or lyophilized together as a powder and provided in a single container.

When both agents are provided in solution form, they can be contained in an infusion/injection system for simultaneous administration or in a tandem arrangement. For example, the platelet aggregation inhibitor may be in an i.v. injectable form, or infusion bag linked in series, via tubing, to the fibrinolytic agent in a second infusion bag. Using such a system, a patient can receive an initial bolus-type injection or infusion, of the compound inhibitor followed by an infusion of the fibrinolytic agent.

The pharmacological activity of the compounds of this invention is assessed by their ability to inhibit the binding of ³H-SK&F 107260, a known RGD-fibrinogen antagonist, to the GPIIbIIIa receptor; their ability to inhibit platelet aggregation, *in vitro,* and their ability to inhibit thrombus formation *in vivo.*

### Inhibition of RGD-mediated GPIIb-IIIa binding

Inhibition of RGD-mediated GPIIb-IIIa binding was demonstrated by assessing the ability of compounds to inhibit the binding of ³H-SK&F 107260, a known RGD-fibrinogen antagonist, to the GPIIbIIIa receptor according to the procedure disclosed in WO 93/00095 (PCT/US/92/05463.

### Inhibition of Platelet Aggregation

Inhibition of platelet aggregation was demonstrated following the procedure disclosed in WO 93/00095 (PCT/US/92/05463.

The compounds of this invention inhibit the aggregation of human platelets stimulated with ADP with IC50 of about 0.001 to about 150 µM. Preferred compounds have IC50 of less than 0.1 µM.

To assess the stability of the compounds to plasma proteases, the compounds were incubated for 3 h (rather than 3 min) in the PRP prior to addition of the agonist.

### In Vivo Inhibition of Platelet Aggregation

*In vivo* inhibition of thrombus formation is demonstrated by recording the systemic and hemodynamic effects of infusion of the compounds into anesthetized dogs according to the methods described in Aiken *et al*., *Prostaglandins,* 19, 629 (1980).

The examples which follow are provided to illustrate how to make and use the compounds of this invention. Many other embodiments will be readily apparent and available to those skilled in the art.

### EXAMPLES

In the Examples, all temperatures are in degrees Centigrade. Mass spectra were performed using fast atom bombardment (FAB) or electro-spray (ES) ionization. Melting points were taken on a Thomas-Hoover capillary melting point apparatus and are uncorrected.

NMR were recorded at 250 MHz using a Bruker AM 250 spectrometer, unless otherwise indicated. Chemical shifts are reported in ppm (□) downfield from tetramethylsilane. Multiplicities for NMR spectra are indicated as: s=singlet, d=doublet, t=triplet, q=quartet, m=multiplet, dd=doublet of doublets, dt=doublet of triplets etc. and br indicates a broad signal. J indicates the NMR coupling constant in Hertz.

Diazald® is N-methyl-N-nitroso-p-toluene sulfonamide, and is a registered trademark of the Alrich Chemical Co., Milwaukee, Wisconsin. Celite® is filter aid composed of acid washed diatomaceous silica, and is a registered trademark of Mansville Corp., Denver, Colorado. Florisil® is an activated magnesium silicate chromatographic support and is a registered trademark of Floridon Co., Pittsburgh, Pennsylvania. Analtech silica gel GF and EM silica gel thin layer plates were used for thin layer chromatography. Both flash and gravity chromatography were carried out on Merck 60 (230-400 mesh) silica gel. ODS refers to an octadecylsilyl derivatized silica gel chromatographic support. AN/W-TFA indicates an isocratic eluant system of the indicated percentage of acetonitrile in water with 0.1% TFA. 5µ Apex-ODS indicates an octadecylsilane derivatized silica gel support, having a nominal particle size of 5µ, made by Jones Chromatography, Littleton, Colorado. YMC ODS-AQ® is an ODS chromatographic support and is a registered trademark of YMC Co. Ltd., Kyoto, Japan. PRP-1® is a polymeric (styrene-divinyl benzene) chromatographic support and is a registered trademark of Hamilton Co., Reno, Nevada.

The following methods are illustrative of the manner of making certain useful intermediates for the preparation of the compounds of this invention.

### Preparation 1

### Preparation of 3-(4-pyridyl)propanamine

a) N-[3-(4-pyridyl)propyl]phthalimide
   Diethyl azodicarboxylate (4.4 g, 25.4 mmol) was dissolved in dry THF and added dropwise to a solution of 3-(4-pyridyl)propanol (3.18 g, 23.18 mmol), triphenylphosphine (12.3 g, 46.9 mmol) and phthalimide (3.6 g, 24.5 mmol) in dry THF (50 mL). The mixture was stirred at RT for 3.5 h, concentrated and the residue taken up in ether and filtered. The filter cake was taken up in hexane/ethyl acetate/chloroform (40:30:30) and flash chromatographed (silica gel, 30:35:35 hexane:ethyl acetate:chloroform) (3L) to yield the title compound (3.3 g, 54%). ¹H NMR (90 MHz, CDCl₃) δ 8.44 (m, 2H), 7.71 (m, 4H), 7.13 (d, 2H), 3.70 (t, 2H), 2.65 (t, 2H), 2.05 (m, 2H)
b) 3-(4-pyridyl)propanamine
   The compound of Preparation 1(a) (2.1 g, 7.7 mmol) was dissolved in ethanol, treated with anhydrous hydrazine (0.3 mL, 9.5 mmol), stirred overnight and concentrated. The residue was treated with hydrazine in ethanol overnight, concentrated and the residue taken up in chloroform, filtered and concentrated to give the title compound (1.1 g, quant).
   ¹H NMR (90 MHz, CDCl₃) δ 8.45 (d, 2H), 7.77 (t, 1H), 7.40 (m, 2H), 7.12 (d, 2H), 3.38 (q, 2H), 2.60(t, 2H), 1.86 (m, 2H).

### Preparation 2

### Preparation of 5-(benzyloxycarbonylamino)pentanamine

To a suspension of 1,5-diaminopentane dihydrochloride (2.9 g, 16.7 mmol) in THF (100 mL) was added 20% aqueous sodium hydroxide (10.0 g, 50 mmol) at 0°C. A solution of benzyl chloroformate (1.7 g, 10 mmol) in THF (10 mL) was added rapidly dropwise and the resulting mixture stirred at RT. After 1 h, the organic layer was separated and concentrated to give a residue which was dissolved in ethyl acetate (200 mL). This solution was extracted with water (3 x 50 mL.), dried (sodium sulfate) and concentrated. The residue was redissolved in ether (50 mL) and 1.0 M ethereal hydrogen chloride (3.0 mL) was added. The resulting precipitate was filtered, suspended in CH₂Cl₂ (75 mL) and shaken with 5% sodium hydroxide (15 mL). The organic layer was washed with water (75 mL), dried (sodium sulfate) and concentrated to give the title compound (0.25 g, 30%): ¹H NMR (400 MHz, CDCl₃) δ 7.15-7.4 (m, 5H), 5.15-5.4 (m, 1H), 5.05 (s, 2H), 3.05-3.2 (m, 2H), 2.55-2.7 (m, 2H), 1.2-1.5 (m, 6H).

### Preparation 3

### Preparation of 4-[N-(t-butoxycarbonyl-N-(propyl)-aminomethyl]aniline

a) 4-[N-(t-butoxycarbonyl-N-(propyl)aminomethyl]-1-nitrobenzene
   A solution of N-(propyl)aminomethyl-1-nitrobenzene (1.8 g, 9.27 mmol), triethylamine (1.41 g, 13.9 mmol) and di-t-butyl dicarbonate (2.8 g, 13 mmol) in THF (60 mL) was stirred for 1 h at RT, concentrated and the residue stirred in water (100 mL) for 1 h. The aqueous phase was decanted and the residue dissolved in ethyl acetate (300 mL). The solution was extracted with water (3 x 100 mL) and brine (100 mL), dried (sodium sulfate) and concentrated to yield the title compound (2.65 g, 95%). ¹H NMR (400 MHz, CDCl₃) δ 8.2 (d, 2H), 7.4 (d, 2H), 4.5 (m, 2H), 3.15 (d, 2H), 1.3-1.6 (m, 9H), 0.9 (t, 3H).
b) 4-[N-(t-butoxycarbonyl)-N-(propyl)aminomethyl]aniline
   A solution of the compound of Preparation 3(a) (2.7 g, 9.2 mmol) in ethanol (100 mL) containing 10% palladium on carbon (0.4 g) was hydrogenated at 2.76 bar. After 30 min, the mixture was filtered and concentrated to give the title compound (2.4 g., 100%). ¹H NMR (400 MHz, CDCl₃) δ 7.0-7.3 (m, 3H), 6.6 (d, 1H), 4.3 (m, 2H), 3.6 (br, 2H), 3.1 (m, 2H), 1.4-1.6 (m, 9H), 0.9 (t, 3H).

### Preparation 4

### Preparation of 3-[4-[N-(t-butoxycarbonyl)piperi-dinyl]]propanamine

a) 3-[4-[N-(t-butoxycarbonyl)piperidinyl]]propanol
   3-(4-Pyridyl)propanol (10.40 g, 75.8 mmol) was dissolved in glacial acetic acid, platinum oxide (1 g) was added and the mixture was shaken was hydrogenated ( 3.45 bar) for 6 h. The mixture was filtered through Celite®, concentrated and concentrated twice from toluene. The residue was dried under reduced pressure, dissolved in CH₂Cl₂ (150 mL) and treated with triethylamine (10.2 g, 100.7 mmol) and then with di-t-butyl dicarbonate (18.0 g, 82.5 mmol). The reaction was stirred for 2 h, concentrated and the residue was taken up in ethyl acetate and washed sequentially with water, 1N hydrochloric acid (2x), water and 1N sodium bicarbonate (2x). The organic phase was dried (magnesium sulfate), filtered and concentrated and the residue was purified by flash chromatography (silica gel, chloroform; 5:95 methanol:chloroform) to give the title compound (12.87 g, 68.7%): TLC R_{f} 0.78 (silica 9:1 chloroform:methanol); ¹H NMR (400 MHz, CDCl₃) δ 4.08 (m, 2H), 3.65 (t, 2H), 2.64 (t, 2H), 1.80-0.90 (m), 1.43 (s, 9H).
b) N-[3-[4-[N-(t-butoxycarbonyl)piperidinyl]]propyl]-phthalimide
   The compound of Preparation 4(a) (2.0 g, 8.22 mmol) was dissolved in dry THF (100 mL) with phthalimide (1.52 g, 10.34 mmol) and triphenylphosphine (2.22 g, 8.46 mmol). Diethyl azidodicarboxylate (1.65 g; 9.53 mmol) was dissolved in dry THF and slowly added to the alcohol reaction mixture. The reaction was left stirring at room temperature for 3 days. The solvents were evaporated under vacuum. The residue was taken up in hexane:ethyl acetate (9:1) and flash chromatographed (silica gel, 10%; 20% ethyl acetate/hexane) to yield the title compound (2.0 g, 60.6%). ¹H NMR (90 MHz, CDCl₃) δ 8.05-7.65 (m, 4H), 4.40 (br. d., 2H), 3.72 (t, 2H), 2.78 (t, 2H), 1.0-2.0 (m), 1.50 (s., 9H)
c) 3-[4-[N-(t-butoxycarbonyl)piperidinyl]]propanamine
   The compound of Preparation 4(b) (2.0g, 5.42 mmol) was dissolved in ethanol (50 mL). Anhydrous hydrazine (excess) was added and the solution was stirred overnight, concentrated and the residue taken into chloroform and filtered. The filtrate was concentrated to yield the title compound (863.5 mg, 65.7%). ¹H NMR (250 MHz, CDCl₃) δ 4.05 (br. d., 2H), 3.00 (t, 2H), 2.66 (t, 2H), 2.2-0.90 (m), 1.45 (s, 9H); TLC Rf 0.63 (3:1:1 n-BuOH:HOAc:water).

### Preparation 5

### Preparation of 2-[1-[4-(benzyloxycarbonyl)piperazinyl]]-ethanamine; and 2-[1-[4-(benzyloxycarbonyl)piperazinyl]]-N-methyl-ethanamine

a) 2-[1-[4-(benzyloxycarbonyl)piperazinyl]]ethanamine
   2-(1-Piperazinyl)ethylamine was protected according to the general procedure of *Tet. Lett*., 27, 4391 (1986). A solution of t-butylchlorodiphenylsilane (7.8 mL, 30 mmol) in acetonitrile (10 mL) was added dropwise to a solution of 2-(1-piperazinyl)ethylamine (4.0 mL, 31 mmol) and triethylamine (6.3 mL, 45 mmol) in acetonitrile (20 mL) stirred at 10°C. The mixture was stirred 2 h at RT, cooled to 10°C and treated with triethylamine (6.3 mL, 45 mmol) and a solution of benzyl chloroformate (4.3 mL, 30 mmol) in CH₂Cl₂ (10 mL). The mixture was stirred at RT for 2 h, filtered and concentrated. The residue was stirred in 80% acetic acid (100 mL) overnight, poured into brine and washed with ethyl acetate. The aqueous phase was basified with saturated sodium carbonate, extracted with ethyl acetate and the organic phase was dried (magnesium sulfate), filtered and concentrated to give the title compound (1.1 g, 14%). ¹NMR (400 MHz, CDCl₃) δ 2.43 (6H, m), 2.80 (2H, m), 3.52 (4H, m), 5.14 (2H, s), 7.38 (5H, s).
b) 2-[1-[4-(benzyloxycarbonyl)piperazinyl]]-N-methyl-ethanamine
   Formic acid (0.5 mL, 12.2 mmol) was added dropwise via syringe to acetic anhydride (1.0 mL, 9.8 mmol) at 10°C. The mixture was stirred for 10 min and heated to 50°C for 2 h. The mixture was cooled to RT, anhydrous THF (10 mL) was added followed by the compound of Preparation 5(a) (1.0 g, 3.8 mmol) dissolved in THF. The mixture was stirred for 2.5 h at RT, concentrated and the residue was dissolved in THF (25 mL) and cooled to 10°C. 1.0M Boron methyl sulfide (1 mL, 10 mmol) dissolved in THF (10 mL) was added dropwise at 10°C. The mixture was stirred until gas evolution ceased and was heated to reflux for 2 h. The mixture was cooled, treated carefully with saturated methanolic hydrogen chloride (20 mL) and heated to reflux for 1 h. The mixture was concentrated to yield the title compound (400 mg, 40%). MS(ES) m/e 278.0 [M+H]⁺; 1NMR (400 MHz, CDCl₃) δ 2.68-3.92 (15H, m), 5.15(2H, s), 7.37 (5H, s)

### Preparation 6

### Preparation of 6-(t-butoxycarbonylamino)hexylamine

A solution of 1,6-diaminohexane (14 g, 0.12 mol) in ethanol (500 mL) was mixed with a solution of di-t-butyl carbonate (23g, 0.1 mol) in ethanol (500 mL) and stirred overnight. The mixture was filtered, concentrated and the residue was shaken with a mixture of ether (200 mL) and water (50 mL). The aqueous phase was extracted twice with ether (50 mL) and the organic phase was extracted sequentially with brine, cold 0.4N hydrochloric acid (150 mL) and water (2 x100 mL). The pH of the acidic extract was brought to 12-13 with 1N sodium hydroxide and it was extracted twice with ether(50 mL). The organic phase was washed with brine, dried (sodium sulfate) and concentrated to yield the title compound (13.16 g, 49%). TLC Rf 0.53 (Kieselgel 60 F₂₅₄, 15:3:2 2-butanol:formic acid:water); MS(ES) m/e 217.0; ¹NMR (400 MHz, CDCl₃): δ 4.57 (br s, 1H), 3.10-3.12 (m 2H), 2.68-2.71 (m 2H), 1.84 (br s 2H), 1.44-1.48 (br s 9H), 1.32-1.34 (br s 8H).

### Preparation 7

### Preparation of 4-(t-butoxycarbonylamino)amine

Using the procedure of Preparation 6, except substituting 1,4-diaminobutane for 1,6-diaminohexane, gave the title compound (2.5 g, 26%): TLC Rf 0.39 (Kieselgel 60 F₂₅₄, 15:3:2 2-butanol:formic acid:water); MS(ES) m/e 189.0.

### Preparation 8

### Preparation of 2-(amino)pyridine-4-ethanamine dihydrochloride

a) methyl 2-(acetamido)pyridine-4-carboxylate
   To 2-(acetamido)pyridine-4-carboxylic acid (25 g, 139 mmol) was added a saturated solution of anhydrous hydrogen chloride in methanol (600 mL). The cloudy suspension was stirred for 16 h at RT and refluxed for 24 h. The resulting solution was concentrated, dissolved in chloroform, washed with saturated sodium bicarbonate, brine, dried (magnesium sulfate) and concentrated to give the title compound as a white solid (17.86 g, 84%). TLC R_{f} 0.55 (5% methanol/chloroform); ¹H NMR (CDCl₃) δ 3.96 (3H, s), 4.80 (2H, s),7.12 (1H, J=5Hz, d), 7.19 (1H, s), 8.10 (1H, d, J=5Hz).
b) 2-(amino)pyridine-4-methanol
   To the compound of Preparation 8(a) (15.14 g, 100 mmol) in dry THF (300 mL) was added dropwise, with stirring at 0°C under argon, a solution of 1N lithium aluminum hydride in THF (200 mL, 200 mmol) over 1 h. After the addition, the ice bath was removed and the reaction was stirred at RT for 1 h. The reaction was cooled to 0°C and carefully quenched with ethyl acetate (50 mL) followed by water (8 mL), 15% sodium hydroxide (8 mL) and water (22 mL). After 15 min, the resulting suspension was filtered and the filter was rinsed thoroughly with a solution of 20% methanol/chloroform (200 mL). The filtrate was concentrated and the residue was purified by flash chromatography (silica gel, 10% methanol/chloroform) to obtain the title compound (9.60 g, 77%) as a white solid. TLC R_{f} 0.17 (5% methanol/chloroform). ¹H NMR (CD₃OD) δ 4.55 (2H, s), 4.88 (3H, s), 6.62 (1H, d, J=5Hz), 6.65 (1H, s), 7.88 (1H, d, J=5Hz).
c) 2-amino-4-(bromomethyl)pyridine
   To the compound of Preparation 8(b) (9.40 g, 76 mmol) was added 48% hydrobromic acid (75 mL). The solution was heated to 100°C under argon for 16 h, concentrated and dried under vacuum to give the title compound as an off-white solid which was used in the next reaction without purification.
d) methyl 3-[(2-(amino)pyrid-4-yl]-2-(methoxycarbonyl)-propionate
   To a stirred solution of dimethyl malonate (20 mL, 175 mmol) and sodium methoxide (25 wt% in methanol, 35 mL, 162 mmol) in dry methanol (100 mL) at 0°C was added the compound of Preparation 8(c) (18.67 g, 70 mmol) in one portion. After 5 min, the reaction was allowed to warm to RT. The mixture was stirred for 4 h, acidified with acetic acid and concentrated. The residue was dissolved in chloroform, washed with saturated sodium bicarbonate, brine, dried (magnesium sulfate), and concentrated. The residue was purified by flash chromatography (silica gel, 3% methanol/chloroform) to give the title compound (11.00 g, 66%) as an oil which solidified under vacuum. TLC Rf 0.46 (5% methanol: chloroform). ¹H NMR (CDCl₃) δ 3.10 (2H, d, J=8Hz), 3.69 (1H, t), 3.73 (6H, s), 4.51 (2H, br s), 6.38 (1H, s), 6.50 (1H, d, J=5Hz), 8.01 (1H, d, J=5Hz).
e) 2-(amino)pyridine-4-propionic acid hydrochloride
   To the compound of Preparation 8(d) (11.0 g, 46 mmol) was added concentrated hydrochloric acid (100 mL). The reaction was heated at 100°C for 16 h and concentrated to give the title compound (9.36 g, 100%) as a white solid.
f) methyl 2-(amino)pyridine-4-propionate
   To the compound of Preparation 8(e) (8.36 g, 41 mmol) was added saturated solution of anhydrous hydrogen chloride in methanol (300 mL). The mixture was stirred for 3 d at RT, concentrated, and dried under vacuum. The remaining solid was dissolved in chloroform, washed with saturated sodium carbonate, brine, dried (magnesium sulfate) and concentrated to give the title compound (7.40 g, 100%). TLC R_{f} 0.44 (5% methanol/chloroform). ¹H NMR (CDCl₃) δ 2.72 (4H, m), 3.70 (3H, s), 4.69 (2H, br s), 6.38 (1H, s), 6.52 (1H, d, J=5Hz), 8.01 (1H, d, J=5Hz).
g) methyl 2-(acetamido)pyridine-4-propionate
   To a stirred solution of the compound of Preparation 8(f) (7.40 g, 41 mmol) in THF (200 mL) was added sodium bicarbonate (4.5 g, 53.6 mmol) followed by acetic anhydride (5 mL, 53 mmol). After stirring for 16 h, the reaction was diluted with ethyl acetate, washed with saturated sodium carbonate, brine, dried (magnesium sulfate) and concentrated. The residue was purified by flash chromatography (silica gel, 3% methanol/chloroform) to yield the title compound (9.02 g, 98%). TLC R_{f} 0.27 (60% ethyl acetate/hexane). ¹H NMR (CDCl₃) δ 2.22 (3H, s), 2.70 (2H, t), 3.0 (2H, t), 3.72 (3H, s), 6.98 (1H, d, J=5Hz), 8.20 (1H, s), 8.24 (1H, d, J=5Hz), 9.88 (1H, br s).
h) 2-(acetamido)pyridine-4-propionic acid
   To a stirred solution of the compound of Preparation 8(g) (9.0 g, 40 mmol) in dioxane (150 mL) was added 1N sodium hydroxide (80 mL) dropwise. After stirring for 5 h, the reaction was made acidified with 1N hydrochloric acid (80 mL) and the resulting solution was concentrated to a small volume to precipitate the product, which was filtered off, rinsed with a small volume of cold water and dried under vacuum to give the title compound (7.83 g, 94%). TLC R_{f} 0.32 (95:4:1 chloroform:methanol:acetic acid). MS(DCI/NH₃) m/e 209.1 [M+H]⁺; ¹H NMR (D₆-DMSO) δ 2.08 (3H, s), 2.56 (2H, t), 3.36 (2H, br s), 6.97 (1H, d, J=5Hz), 7.97 (1H, s), 8.18 (1H, d, J=5Hz).
i) 2-[(2-(acetamido)pyrid-4-yl]-N-(methoxycarbonyl)ethanamine
   To a stirred solution of the compound of Preparation 8(h) (4,75 g, 23 mmol) and triethylamine (3.8 mL, 27 mmol) in dry toluene (150 mL) was added diphenylphosphorylazide (5.7 mL, 26 mmol). The reaction was stirred at RT for 15 min, then heated to 80°C with a reflux condenser attached, behind a safety shield. Vigorous gas evolution occurred after ∼10 min. The reaction was stirred an additional 30 min, the mixture became clear and gas evolution ceased. Methanol (5 mL, 123 mmol) was added and the reaction was allowed to stir at 80°C for an additional 4 h. The mixture was concentrated and the residue was purified by flash chromatography (silica gel, 3% methanol/chloroform) to yield the title compound (3.92 g, 72%). TLC R_{f} 0.42 (5% methanol/chloroform). MS(DCI/NH₃) m/e 238.1 [M+H]⁺; ¹H NMR (CDCl₃) δ 2.23 (3H, s), 2.82 (2H, t), 3.39 (2H, m), 3.68 (3H, s), 4.81 (2H, s), 7.02 (1H, d, J=5Hz), 8.03 (1H, s), 8.22 (1H, d, J=5Hz).
j) 2-(amino)pyridine-4-ethanamine
   To the compound of Preparation 8(i) (3.80 g, 16 mmol) was added a solution of 6N hydrochloric acid (150 mL). The reaction was heated at 110°C for 24 h, concentrated and dried under vacuum to give the title compound (3.37 g, 100%) as a white solid. MS(DCI) m/e 138.1 [M+H]⁺.

### Preparation 9

### Preparation of 2-(hydroxy)pyridine-4-ethanamine hydrochloride

a) 2-[(2-methoxy)pyrid-4-yl]-N-(methoxycarbonyl)ethanamine
   Using the procedure of Preparation 8(i), except substituting 2-(methoxy)pyridine-4-propionic acid for the compound of Preparation 8(h), gave the title compound. TLC R_{f} 0.24 (30% ethyl acetate/hexane). MS(DCI/NH₃) m/e 211.1 [M+H]⁺; ¹H NMR (CDCl₃) δ 2.80 (2H, t), 3.49 (2H, m), 3.72 (3H, s), 3.96 (3H, s), 4.82 (1H, br s), 6.66 (1H, s), 6.79 (1H, d, J=5Hz), 8.17 (1H, d, J=5Hz).
b) 2-(hydroxy)pyridine-4-ethanamine hydrochloride
   Using the procedure of Preparation 8(j), except substituting the compound of Preparation 9(a) for the compound of Preparation 8(i), gave the title compound. MS(DCI/NH₃) m/e 139.1 [M+H]⁺.

### Preparation 10

### Preparation of 4-[2-[N-(t-butoxycarbonyl)aminoethyl]] piperidine

a) 4-[[2-(t-butoxycarbonyl)amino]ethyl]pyridine
   A solution of pyridine-4-ethanamine (1.54 g, 12.6 mmol) in dichloromethane (15 mL) was treated at 0°C with di-t-butyl dicarbonate (2.75 g, 12.6 mmol) and triethylamine (1.76 mL, 12.6 mmol), and stirred at RT for 3 d. The mixture was diluted with chloroform, washed with 5% sodium carbonate, dried (magnesium sulfate) and concentrated. The residue was purified by flash chromatography (silica gel, 80% ethyl acetate/hexane) to give the title compound 1.01 g (36%). ¹H NMR (90 MHz, CDCl₃) δ 8.53 (d, 2H, J=4.5 Hz), 7.17 (d, 2H, J=4.5 Hz), 5.77 (br s, 1H), 3.40 (d oft, 2H, J=6.0, 7.5 Hz), 2.83 (t, 2H, J=7.5 Hz), 1.45 (s, 9H).
b) 4-[2-[N-(t-butoxycarbonyl)aminoethyl]]piperidine
   A mixture of the compound of Preparation 10(a) (1.01 g, 4.54 mmol) and platinum oxide (150 mg) in ethanol was shaken under hydrogen at RT for 4 h. Additional 1N hydrochloric acid (4 mL) and platinum oxide (100 mg) was added and the reaction mixture shaken with hydrogen at RT for 1 h and then allowed to sit at neutral pH (adjusted with 5% sodium carbonate) under argon for 18 h. The catalyst was removed by filtration through Celite® and the filtrate was concentrated. The residue was concentrated twice from methanol:toluene, taken into chloroform, filtered and concentrated to give the title compound which was used without purification.

### Preparation 11

### Preparation of N-methyl-2-(1-methyl-1,2,5,6-tetrahydropyrid-4-yl)ethanamine, dihydrochloride

a) N-(t-butoxycarbonyl)-N-methyl-2-(1-methyl-pyrid-4-yl)ethanamine
   A mixture of N-methyl-2-(pyrid-4-yl)ethanamine (4.1 g, 30 mmol), di-t-butyl dicarbonate (26.2 g, 120 mmol) and triethylamine (15.2 g, 150 mmol) in dichloromethane (100 mL) was stirred for 16 h, concentrated, and the residue was diluted with water and extracted with dichloromethane. The organic phase was washed, dried (magnesium sulfate) and concentrated. The residue was chromatographed (silica gel, 5% methanol:dichloromethane) to give the title compound (4 g, 56%).
b) N-(t-butoxycarbonyl)-N-methyl-2-(1-methyl-4-pyridinium)ethanamine iodide
   A mixture of the compound of Preparation 11(a) (2 g, 10 mmol) and iodomethane (21.2 g, 150 mmol) in acetonitrile (30 mL) was heated to reflux for 2 h. The mixture was concentrated and the residue was dissolved in dichloromethane, decolorized with charcoal, filtered and the filtrate was concentrated. The residue was treated with ether to give the title compound (3.0 g, 79%) as a yellow solid. MS(ES) m/e 251 [M-I]⁺.
c) N-(t-butoxycarbonyl)-N-methyl-2-(1-methyl-1,2,5,6-tetrahydropyrid-4-yl)ethanamine, dihydrochloride
   The compound of Preparation 11(b), 0.95 g, 25 mmol) was dissolved in methanol (30 mL), treated with sodium borohydride (0.38 g, 10 mmol) and stirred for 2 h. The mixture was concentrated, dissolved in water and extracted with dichloromethane. The organic phase was dried (magnesium sulfate), concentrated and the residue was treated with 4N hydrogen chloride in dioxane (5 mL) overnight. The mixture was concentrated and the residue was azeotroped with toluene. The resulting solid was triturated with ether to give the title compound (0.5 g, 89%).

### Preparation 12

### Preparation of 3-[N-(t-butoxycarbonyl)piperidin-4-yl]propionaldehyde

To a stirred solution of oxalyl chloride (2 mL, 22 mmol) in dry dichloromethane (50 mL) at -78°C under argon, was added dropwise a solution of dimethylsulfoxide (3.4 mL, 44 mmol) in dichloromethane (10 mL). After 2 min, a solution of 3-[N-(t-butoxycarbonyl)piperidin-4-yl]propanol (4.86 g, 20 mmol) in dichloromethane (10 mL) was added dropwise over 5 min. After stirring an additional 15 min at -78°C, triethylamine (14 mL) was added dropwise. After 5 min, the reaction became a thick white slurry and was allowed to warm to RT and stirred for 16 h. The reaction mixture was washed with cold 1N hydrochloric acid and with brine, dried (magnesium sulfate) and concentrated. The residue was purified by flash chromatography (silica gel, 30% ethyl acetate/hexane) to yield the title compound (4.18g, 87%). TLC R_{f} 0.40 (30% ethyl acetate/hexane); ¹H NMR (CDCl₃) δ 0.95-1.80 (7H, m), 1.48 (9H, s), 2.40-2.90 (4H, m), 4.14 (2H, br dt), 9.88 (1H, t).

### Preparation 13

### Preparation of 2-[2-(amino)pyrid-4-yl]-N-methyl-ethanamine

a) 2-[2-(amino)pyrid-4-yl]-N-formyl-N-methyl-ethanamine
   To a stirred solution of the compound of Preparation 8(j) (1.5 g, 7.1 mmol) in 1N sodium hydroxide (14.3 mL), water (15 mL) and THF (30 mL) was added dropwise a solution of 4-nitrophenyl formate (1.3 g, 7.8 mmol) in THF (15 mL). After stirring for 16 h, the reaction was concentrated and the residue was purified by flash chromatography (silica gel, 15:85:0.1 methanol/chloroform:ammonium hydroxide) to give the title compound (1.14 g, 96%) as a slightly yellowish solid. TLC R_{f} 0.32 (90:10:0.1, chloroform:methanol:ammonium hydroxide); ¹H NMR (CDCl₃) δ 2.77 (2H, t), 3.57 (2H, dt), 4.84 (2H, br s), 6.47 (1H, s), 6.57 (1H, d, J=6Hz), 6.94 (1H, br s), 8.0 (1H, d, J=6Hz), 8.21 (1H, s).
b) 2-[2-(amino)pyrid-4-yl]-N-methyl-ethanamine
   To a stirred solution of the compound of Preparation 13(a) (0.5 g, 3 mmol) in dry THF (30 mL) was added dropwise, at 0°C under argon, a solution of 1N lithium aluminum hydride in THF (20 mL, 20 mmol). The reaction was allowed to warm to RT and was heated to reflux for 16 h. After cooling to 0°C, the reaction was carefully quenched with ethyl acetate (2 mL), water (0.8 mL), 15% sodium hydroxide (0.8 mL) and water (2.3 mL). The resulting suspension was stirred for 15 min, filtered through Celite®, and the filtrate was concentrated. The residue was purified by flash chromatography (silica gel, 75:25:0.1 chloroform:methanol:ammonium hydroxide) to afford the title compound (0.30 g, 66%). TLC R_{f} 0.16 (80:20:0.1, chloroform:methanol:ammonium hydroxide); ¹H NMR (CDCl₃) δ 2.45 (3H, s), 2.82 (4H, dt), 4.95 (3H, br s), 6.53 (1H, s), 6.57 (1H, d, J=6Hz), 7.87 (1H, d, J=6Hz).

### Preparation 14

### Preparation of 4-[N-(t-butoxycarbonyl)piperazin-1-yl]but-1-yne

A mixture of butyn-1-yl-4-tosylate (0.4 g, 1.7 mmol), N-(t-butoxycarbonyl)piperazine (0.28 g, 1.5 mmol) and sodium bicarbonate (0.15 g, 1.7 mmol) in dimethylformamide (7 mL) was stirred and heated to 80°C. The mixture was concentrated and the residue was partitioned between dichloromethane and water. The organic phase was washed with brine, dried (magnesium sulfate) and concentrated to give the title compound (0.38 g) as an oil: ¹H NMR (400 MHz, CDCl₃) δ 3.45 (m, 4H), 2.60 (t, 2H), 2.42 (m, 4H), 2.38 (t, 2H), 1.99 (s, 1H), 1.98 (s, 9H).

### Preparation 15

### Preparation of N-(t-butoxycarbonyl)-4,4'-bipiperidine

4,4'-Bipiperidine dihydrochloride (2.5 g, 10 mmol) was dissolved in water (10 mL) and treated with 5N sodium hydroxide to pH 8-9, diluted to 120 mL with ethanol and stirred at RT. The resulting mixture was treated with di-t-butyl dicarbonate (2.4 g, 11 mmol) in ethanol (80 mL) in one portion and the mixture stirred at RT, with periodic additions of 5N sodium hydroxide to maintain a pH of 8-9. After 5 h, the mixture was concentrated and the residue was dissolved in a mixture of 1:1 ether:water (100 mL) and the pH adjusted to 12 with 5N sodium hydroxide. The aqueous phase was extracted with ether and the organic phase was washed sequentially with brine, dilute citric acid, and water. The aqueous phase was adjusted to pH 12-13 with 5N sodium hydroxide and extracted with ether. The organic phase was washed with brine, dried (sodium sulfate), concentrated to a clear oil and dried *in vacuo* to give the title compound (1.7 g, 63%) as a solid. TLC R_{f} 0.4 (Kieselgel 60 F₂₅₄, 15:3:2 2-butanol:formic acid:water); MS (ES) m/e 268.3 [M+H]⁺.

### Preparation 16

### Preparation of (R,S)-8-[[[4-(aminoiminomethyl)phenyl]amino]-carbonyl]-1,2,4,5-tetrahydro-3-oxo-4-(2-phenylethyl)-3H-1,4-benzodiazepine-2-acetic acid, dihydrochloride

a) t-butyl 3-nitro-4-[N-(2-phenylethyl)aminomethyl]benzoate, and t-butyl 3-nitro-4-[N-(t-butoxycarbonyl)-N-(2-phenylethyl)aminomethyl]benzoate.
   A solution of t-butyl 4-bromomethyl-3-nitrobenzoate [prepared by the method in *Int. J. Peptide Res*., 36, 31 (1990)] (1.6 g, 0.005 mol) in CH₂Cl₂ (10 mL) was added dropwise, over 15 min, to a solution of phenethylamine (1.89 g, 0.015 mol) in CH₂Cl₂ (50 mL). The mixture was stirred at room temperature under argon for 24 h and concentrated *in vacuo*. The residue was dissolved in THF (50 mL) and treated with a solution of triethylamine (2.5 g, 0.025 mol) and di-t-butyl dicarbonate (4.4 g, 0.02 mol) in THF (50 mL). The resulting mixture was stirred overnight at room temperature under argon and concentrated *in vacuo*. The residue was dissolved in ethyl acetate, washed with water and dried with sodium sulfate. The organic phase was concentrated *in vacuo* and the residue was triturated with ethyl acetate:hexane (15:85) to yield the title compound (0.87 g, 37%). Mp 110-113°C.
   The filtrate was chromatographed (silica gel, ethyl acetate:hexane 15:85) to yield additional compound (0.5 g, 21%). Mp 113-115°C.
b) t-butyl 3-amino-4-[N-(t-butoxycarbonyl)-N-(2-phenylethyl)aminomethyl]benzoate
   A mixture of the compound of Preparation 16(a) (1.3 g, 0.0028 mol), ethanol (125 mL) and 10% Pd/C (0.32 g) was shaken under a hydrogen atmosphere ( 2.76 bar) for 50 min. The mixture was filtered and the filtrate was concentrated *in vacuo* to give the title compound. Mp 105-106°C.
c) t-butyl (E/Z)-3-[2-(1,4-dimethoxy-1,4-dioxo-2-butenyl)amino]-4-[N-(t-butoxycarbonyl)-N-(2-phenylethyl)aminomethyl]benzoate
   A solution of the compound of Preparation 16(b) (1.15 g, 0.0027 mol) in methanol (50 mL) was treated with dimethyl acetylenedicarboxylate (0.45 g, 0.0032 mol) and the resulting solution was heated to reflux under argon for 1 h. The mixture was concentrated *in vacuo* and the residue was chromatographed (silica gel, ethyl acetate:hexane 20:80) to give the title compound (1.3 g, 85%).
d) t-butyl (R,S)-3-[2-(1,4-dimethoxy-1,4-dioxo-2-butyl)-amino]-4-[N-(t-butoxycarbonyl)-N-(2-phenylethyl)aminomethyl]-benzoate
   A solution of the compound of Preparation 16(c) (1.3 g, 0.0023 mol) in methanol (100 mL) containing 10% Pd/C (0.38 g) was shaken in a hydrogen atmosphere (2.76 bar) for 4.5 h. The mixture was filtered and the filtrate concentrated *in vacuo* to yield the title compound.
e) methyl (R,S)-8-carboxy-1,2,4,5-tetrahydro-3-oxo-4-(2-phenylethyl)-3H-1,4-benzodiazepine-2-acetate
   A solution of the compound of Preparation 16(d) (1.3 g, 0.003 mol) in CH₂Cl₂ (50 mL) and TFA (50 mL) was kept at room temperature under argon overnight. The mixture was concentrated *in vacuo* to give a residue containing crude (R,S)-3-[2-(1,4-dimethoxy-1,4-dioxo-2-butyl)amino]-4-[N-(2-phenylethyl)aminomethyl]benzoic acid. The residue was dissolved in anhydrous methanol (70 mL), treated with methanolic sodium methoxide (1.6 mL, 0.007 mol) and heated to reflux for 8 h. The mixture was kept at room temperature for 14 h, treated with 1N HCl in diethyl ether (7.5 mL), concentrated *in vacuo*, treated with methanol (3 x 20 mL) and concentrated *in vacuo*. The residue was dissolved in methanol:CH₂Cl₂:acetic acid (10:90:0.4, 15 mL), filtered and chromatographed (silica gel, methanol:CH₂Cl₂:acetic acid 10:90:0.4) to give the title compound (0.66 g, 70%).
   The solid was dissolved in CH2C12 (30 mL) and treated with 1N HCl in diethyl ether (3 mL) to give methyl (R,S)-8-carboxy-1,2,4,5-tetrahydro-3-oxo-4-(2-phenylethyl)-3H-1,4-benzodiazepine-2-acetate hydrochloride.

### Preparation 17

### Preparation of (R,S)-8-[[[4-(aminoiminomethyl)phenyl]-methylamino]carbonyl]-1,2,4,5-tetrahydro-3-oxo-4-(2-phenylethyl)-1H-1,4-benzodiazepine-2-acetic acid

a) 4-(aminomethyl)benzonitrile
   A solution of 4-aminobenzonitrile (5.91 g, 50 mmol) and TFA (0.19 mL, 2.5 mmol) in dry, distilled triethylorthoformate (100 mL) was heated to reflux under argon. After 0.5 h at reflux, the yellow solution was concentrated to dryness *in vacuo* to leave a yellow oil which crystallized on drying in high vacuum. The solid was dissolved in absolute EtOH (100 mL), cooled to 0°C under argon, and NaBH4 (5.68 g, 150 mmol) was added. The mixture was allowed to warm to room temperature over 0.5 h and heated to reflux. After 1 h, the thick mixture was concentrated *in vacuo*, and the residue was partitioned between H₂O (100 mL) and Et₂O (100 mL). The layers were separated, and the aqueous layer was extracted with Et₂O. The combined organic layers were dried (MgSO₄) and concentrated to a yellow solid. The solid was purified by chromatography (silica gel, 30% EtOAc/hexane) to yield the title compound as a faintly yellow solid (6.10 g, 92%). TLC R_{f} 0.45 (silica gel, 30% EtOAc/hexane); ¹H NMR (250 MHz, CDCl₃) δ 7.43 (d, 2H), 6.55 (d, 2H), 4.39 (br s, 1H), 2.88 (s, 3H); IR (CHCl₃) 3460, 2220, 1610, 1528, 1337, 1176, 823 cm⁻¹; MS(ES) m/e 133.0 [M+H]⁺.
b) 4-aminomethyl-(N-t-benzyloxycarbonyl)benzamidine
   A solution of trimethylaluminum in toluene (2.0M, 51 mL, 102 mmol) was added over 4 min to a suspension of powdered NH₄Cl (5.46 g, 102 mmol) in dry toluene (51 mL) in a flame-dried flask at 0°C under argon. The ice bath was then removed and the reaction was allowed to stir at room temperature until gas evolution ceased (1 h). The compound of Preparation 17(a) (4.49 g, 34 mmol) was added, and the reaction was warmed to 80°C (oil bath). Gas evolution occurred on warming. After 23 h at 80°C, the reaction was cooled to room temperature and poured into a stirred slurry of silica gel (170 g) in CHCl₃ (500 mL), causing a significantly exothermic reaction. The resulting mixture was stirred for 0.5 h, then was filtered, and the filter pad was washed with MeOH (1 L). The filtrate was concentrated to a yellow solid which was dried under high vacuum at 50-60°C for 0.5 h. The resulting material was used without further purification.
   The material was dissolved in THF (136 mL) and H₂O (34 mL), and the solution was cooled to 0°C. 5N NaOH (20 mL, 100 mmol) was added dropwise, and the resulting two-phase mixture was cooled for an additional 5 min. Benzyl chloroformate (4.85 mL, 34 mmol) was added dropwise, and the reaction was stirred at 0°C for 0.5 h. The THF was removed *in vacuo* and the residue was extracted with CH₂Cl₂, followed by EtOAc. The combined organic extracts were dried (Na₂SO₄) and concentrated to an off-white solid. Chromatography (silica gel, 3:2 EtOAc:hexane) gave the title compound as an off-white solid (6.50 g, 67%). For characterization purposes, a small sample was recrystallized from EtOAc/hexane. Mp 141-143°C; TLC R_{f} 0.49 (silica gel, 3:2 EtOAc:hexane); ¹H NMR (400 MHz, CDCl₃) δ 7.77 (d, 2H), 7.45 (m, 2H), 7.27-7.38 (m, 3H), 6.56 (d, 2H), 5.20 (s, 2H), 4.28 (br s, 1H), 2.88 (s, 3H); IR (CHCl₃) 3500, 3450, 3310, 1648, 1608, 1575, 1493, 1263, 1141 cm⁻¹; MS(ES) m/e 284.2 [M+H]⁺.
c) methyl (R,S)-8-[[[4-[N-(benzyloxycarbonyl)-aminoiminomethyl]phenyl]methylamino]carbonyl]-1,2,4,5-tetrahydro-3-oxo-4-(2-phenylethyl)-3H-1,4-benzodiazepine-2-acetate
   The compound of Preparation 16(e) (382.4 mg, 1.0 mmol) was refluxed with SOCl₂ (10 mL) for 15 min, and the solution was concentrated to dryness *in vacuo*. The residue was dissolved in dry toluene (5 mL), reconcentrated *in vacuo* to remove residual SOCl₂, and dissolved in dry CH₂Cl₂ (2.5 mL). This solution was added dropwise over 5 min to a well-stirred solution of the compound of Preparation 17(b) (0.85 g, 3.0 mmol) and dry pyridine (0.40 mL, 5 mmol) in dry CH₂Cl₂ (30 mL) at 0°C under argon. The resulting orangish-yellow mixture was warmed to room temperature, stirred for 1 h, diluted with EtOAc (100 mL) and washed with 5% aq. NaHCO₃. The solution was dried (Na₂SO₄), concentrated, and purified by chromatography (silica gel, 9:1 EtOAc/toluene) to yield the title compound (441.6 mg, 68%). TLC R_{f} 0.38 (silica gel, 9:1 EtOAc:toluene); ¹H NMR (400 MHz, CDCl₃) δ 7.71 (d, J=8.5 Hz, 2H), 7.02-7.46 (m, 12H), 6.62 (d, J=1.4 Hz, 1H), 6.56 (d, J=7.8 Hz, 1H), 6.38 (dd, J=7.8, 1.4 Hz, 1H), 5.19 (s, 2H), 5.18 (d, J=16.8 Hz, 1H), 4.85-4.93 (m, 1H), 4.17 (d, J=5.3 Hz, 1H), 3.71 (s, 3H), 3.57-3.70 (m, 2H), 3.53 (d, J=16.8 Hz, 1H), 3.44 (s, 3H), 2.90 (dd, J=16.1, 6.9 Hz, 1H), 2.63-2.79 (m, 2H), 2.58 (dd, J=16.1, 6.3 Hz, 1H); IR (CHCl₃) 3160-3540 (br), 3490, 3300, 1733, 1655, 1616, 1577, 1500, 1443, 1380, 1271, 1149, 1110 cm⁻¹; MS(ES) m/e 648.4 [M+H]⁺.
   The following Examples illustrate the manner of making the pharmacologically active compounds and compositions of this invention. The following Preparations illustrate procedures and intermediates in the preparation of compounds of this invention.

### Preparation A

### Preparation of (R,S)-7-[[[4-(aminoiminomethyl)phenyl]-methylamino]carbonyl]-2,3,4,5-tetrahydro-3-oxo-2-(2-phenylethyl)-1H-2-benzazepine-4-acetic acid

a) t-butyl 3-bromo-4-methylbenzoate
   Dimethylformamide dimethyl acetal (48 mL, 200 mmol) was added dropwise over 15 min to a suspension of 3-bromo-4-methylbenzoic acid (85%; 10.75 g, 42.5 mmol) in toluene (100 mL) at 70°C. The reaction was stirred at 70-80°C for an additional 0.5 h, then was cooled, washed sequentially with water and 5% sodium bicarbonate, and dried (sodium sulfate). The mixture was filtered through a pad of silica gel, and the filter pad was washed with toluene. The filtrate was concentrated to afford the title compound (8.0 g, 69%) as a yellow oil. TLC (toluene) R_{f} 0.68; ¹H NMR (250 MHz, CDCl₃) δ 8.13 (d, J=1.7 Hz, 1H), 7.81 (dd, J=7.9, 1.7 Hz, 1H), 7.27 (d, J=7.9 Hz, 1H), 2.44 (3H), 1.59 (s, 9H); IR (CCl₄) 1715, 1368, 1297, 1255, 1170, 1123, 1115 cm⁻¹; MS(ES) m/e 273.0 [M+H]⁺, 271.0 [M+H]⁺, 214.8 (M+H-56)⁺.
b) t-butyl 3-bromo-4-(bromomethyl)benzoate
   A mixture of the compound of A(a) (1.36 g, 5 mmol), N-bromosuccinimide (0.98 g, 5.5 mmol), and benzoyl peroxide (61 mg, 0.25 mmol) in carbon tetrachloride (25 mL) was heated at reflux. After 4 h, the mixture was cooled and filtered, and the filtrate was concentrated. The resulting material was used without purification. TLC R_{f} 0.39 (5:95 ethyl acetate:hexane).
c) t-butyl 3-bromo-4-[[(2-phenylethyl)amino]methyl]-benzoate
   The compound of A(b) was dissolved in dry ether (25 mL), and phenethylamine (1.9 mL, 15 mmol) was added. The addition was slightly exothermic, and the reaction became cloudy. The reaction was stirred at RT overnight, then was diluted with ether (75 mL) and was washed sequentially with 5% sodium bicarbonate and brine (25 mL). Drying (magnesium sulfate), concentration, and silica gel chromatography (33% ethyl acetate/hexane) gave the title compound (1.26 g, 65%) as a pale yellow oil. TLC R_{f} 0.42 (30:70 ethyl acetate:hexane); ¹H NMR (250 MHz, CDCl₃) δ 8.12 (d, J=1.5 Hz, 1H), 7.87 (dd, J=7.9, 1.5 Hz, 1H), 7.40 (d, J=7.9 Hz, 1H), 7.10-7.40 (m, 5 H), 3.90 (s, 2H), 2.70-3.00 (m, 4H), 1.59 (s, 9H); IR (CCl₄) 1717, 1368, 1294, 1253, 1170, 1118 cm⁻¹; MS(ES) m/e 390.0 [M+H]⁺.
d) t-butyl 3-bromo-4-[[[N-(2-phenylethyl)-N-t-butoxycarbonyl]amino]methyl]benzoate
   Di-t-butyl dicarbonate (845 mg, 3.88 mmol) was added all at once to a solution of the compound of A(c) (1.26 g, 3.23 mmol) in chloroform (16 mL) at RT. The reaction was stirred at RT for 1.5 h, then at reflux for 0.5 h. Concentration and chromatography (silica gel, 10% ethyl acetate/hexane) gave the title compound (1.57 g, 99%) as a colorless oil which solidified *in vacuo*. TLC R_{f} 0.49 (10:90 ethyl acetate:hexane); ¹H NMR (250 MHz, CDCl₃) Rotameric mixture, ratio ca. 1.5 : 1; δ 8.13 (d, J=1.5 Hz, 1H), 7.88 (dd, J=8.1, 1.5 Hz, 1H), 7.05-7.40 (m, 6H), 4.30-4.60 (m, 2H), 3.30-3.55 (m, 2H), 2.74-2.93 (m, 2H), 1.20-1.80 (m, 18 H); IR (CCl₄) 1717, 1698, 1367, 1295, 1251, 1165, 1120 cm⁻¹; MS(ES) m/e 490.2 [M+H]⁺, 436.0.
e) methyl 3-methoxycarbonyl-4-[[5-(t-butoxycarbonyl)-2-[[N-(2-phenylethyl)-N-t-(butoxycarbonyl)]amino]methyl]-phenyl]-2-butenoate and methyl 3-methoxycarbonyl-4-[[5-(t-butoxycarbonyl)-2-[[N-(2-phenylethyl)-N-t-butoxycarbonyl)]amino]methyl]phenyl]-3-butenoate.
   A mixture of the compound of A(d) (1.34 g, 2.73 mmol), dimethyl itaconate (648 mg, 4.10 mmol), palladium (II) acetate (30.7 mg, 0.14 mmol), tri-*o*-tolylphosphine (83.2 mg, 0.27 mmol), dry triethylamine (0.76 mL, 5.46 mmol), and dry acetonitrile (27 mL) was deoxygenated through a single evacuation/argon purge cycle, then was heated at reflux under argon. After 6 h, the reaction was cooled, and more palladium (II) acetate (30.7 mg, 0.14 mmol) and tri-o-tolylphosphine (83.2 mg, 0.27 mmol) were added. The mixture was deoxygenated through three evacuation/argon purge cycles, then was heated at reflux under argon overnight (16.5 h). The reaction was concentrated, and the residue was dissolved in ether and washed with water and brine. Drying (magnesium sulfate), concentration, and chromatography (silica gel, 15% ethyl acetate/hexane; then 40% ethyl acetate/hexane) gave the crude title compound as a yellow oil. The residue was rechromatographed (silica gel, 25% ethyl acetate/hexane)to yield the title compound (1.36 g, 88%) as a light yellow oil. This material was used without separation of the isomeric reaction products.
f) methyl 3-methoxycarbonyl-4-[5-(t-butoxycarbonyl)-2-[[[N-(2-phenylethyl)-N-t-butoxycarbonyl]amino]methyl]-phenyl]butanoate
   The compound of A(e) (1.18 g, 2.08 mmol) was dissolved in anhydrous methanol (21 mL), and palladium (II) hydroxide on carbon (0.21 g) was added. The resulting mixture was shaken at RT under H₂ ( 3.24 bar) for 2 h, then was filtered through Celite®. The filtrate was concentrated and resubmitted to the same reaction conditions. After another 5.5 h, the mixture was filtered as before, and the filtrate was concentrated to afford the title compound (1.13 g, 95%) as a colorless oil.
g) methyl (R,S)-7-carboxy-2,3,4,5-tetrahydro-3-oxo-2-(2-phenylethyl)- 1H-2-benzazepine-4-acetate
   TFA (11 mL) was added all at once to a cloudy solution of the compound of A(f) (1.30 g, 2.28 mmol) in dry CH₂Cl₂ (11 mL) at 0°C under argon. The resulting light yellow solution was warmed to RT, stirred for 2 h, and concentrated. The residue was concentrated once from 1,2-dichloroethane to remove residual TFA and give methyl 3-methoxycarbonyl-4-[5-carboxy-2-[[N-(2-phenylethyl)amino]methyl]phenyl]butanoate as a pale green oil.
   The oil was dissolved in anhydrous methanol (11 mL), and the solution was cooled to 0°C under argon. Freshly prepared 1.0 M sodium methoxide/methanol (11 mL, 11 mmol) was added and the ice bath was removed. The yellow solution was allowed to warm to RT over 5 min and heated to reflux under argon. After 3 h, the reaction was cooled in ice and quenched with glacial acetic acid (1.3 mL, 22.8 mmol). The reaction was diluted with ethyl acetate (100 mL) and washed with water. The combined aqueous layers were back-extracted with ethyl acetate, and the combined ethyl acetate layers were washed with brine, dried (magnesium sulfate), and concentrated to a pale green residue. Chromatography ((silica gel, 10% methanol/chloroform/0.1% acetic acid) gave the title compound as a yellow foam. Crystallization from methanol containing a little chloroform gave the title compound (528.2 mg, 61%) as an off-white solid. mp 214-216°C; TLC R_{f} 0.49 (10:90 methanol:chloroform); ¹H NMR (400 MHz, 10% CD₃OD/CDCl₃) δ 7.72-7.78 (m, 2H), 7.11-7.22 (m, 3H), 7.01-7.08 (m, 3H), 5.21 (d, J=16.8 Hz, 1H), 3.70-3.92 (m, 3H), 3.72 (s, 3H), 3.5-3.66 (m, 1H, partially obscured by water peak), 3.03 (app dd, 1H), 2.97 (dd, J=16.7, 8.7 Hz, 1H), 2.86 (dd, J=17.3, 13.6 Hz, 1H), 2.76 (t, J=7.3 Hz, 2H), 2.43 (dd, J=16.7, 5.3 Hz, 1H); MS(ES) m/e 404.0 (M+Na)⁺, 382.4 [M+H]⁺, 350.2 (M+H-CH₃OH)⁺.
h) methyl (R,S)-7-[[[4-[N-(benzyloxycarbonyl)-aminoiminomethyl]phenyl]methylamino]carbonyl]-2,3,4,5-tetrahydro-3-oxo-2-(2-phenylethyl)-1H-2-benzazepine-4-acetate
   The compound of A(g) (117.9 mg, 0.31 mmol) was refluxed with thionyl chloride (3 mL) for 15 min and the yellow solution was concentrated. The residue was concentrated from dry toluene (3 mL) to remove residual thionyl chloride, and the resulting material was dissolved in dry CH₂Cl₂ (0.5 mL). This solution was added dropwise over 1-2 min to a solution of 4-(methylamino)-(N-benzyloxycarbonyl)benzamidine (263 mg, 0.93 mmol) and anhydrous pyridine (0.125 mL, 1.55 mmol) in dry CH₂Cl₂ (10 mL) at 0°C under argon. The resulting yellow mixture was warmed to RT and stirred for 0.5 h, diluted with ethyl acetate, and washed with 5% sodium bicarbonate. Drying (sodium sulfate), concentration, and chromatography (silica gel, 10% ethyl acetate/toluene) gave the title compound (174.8 mg, 87%) as a faintly yellow oil. TLC R_{f} 0.45 (9:1 toluene:ethyl acetate). ¹H NMR (400 MHz, CDCl₃) δ 7.73 (app d, 2H), 7.00-7.45 (m, 13H), 6.89 (app d, 1H), 6.74 (d, J=7.8 Hz, 1H), 5.19 (s, 2H), 5.05 (d, J=16.6 Hz, 1H), 3.52-3.77 (m, 4H), 3.69 (s, 3H), 3.48 (s, 3H), 2.93 (dd, J=16.8, 8.4 Hz, 1H), 2.83 (dd, J=17.3, 3.9 Hz, 1H), 2.60-2.77 (m, 3H), 2.34 (dd, J=16.8, 5.4 Hz, 1H); IR (CHCl₃) 3490, 3300, 1731, 1650, 1614, 1495, 1437, 1360, 1284 (shoulder), 1264, 1143 cm⁻¹; MS(ES) m/e 647.2 [M+H]⁺.
i) methyl (R,S)-7-[[[4-(aminoiminomethyl)phenyl]methylamino]-carbonyl]-2,3,4,5-tetrahydro-3-oxo-2-(2-phenylethyl)- 1H-2-benzazepine-4-acetate
   10% Palladium on carbon (58 mg, 0.054 mmol) was added carefully to a solution of the compound of A(h) (174.8 mg, 0.27 mmol) and TFA (0.021 mL, 0.27 mmol) in ethyl acetate/methanol (1:1, 9 mL), and the mixture was stirred briskly under hydrogen (balloon pressure). After 1.5 h, the mixture was filtered through Celite®, and the filter pad was washed thoroughly with ethyl acetate and methanol. Concentration gave the title compound.
j) (R,S)-7-[[[4-(aminoiminomethyl)phenyl]methylamino)-carbonyl)-2,3,4,5-tetrahydro-3-oxo-2-(2-phenylethyl)- 1H-2-benzazepine-4-acetic acid
   The compound of A(i) was dissolved in methanol (9 mL), and 1.0 N sodium hydroxide (0.81 mL, 0.81 mmol) was added. The solution was stirred at RT overnight, then was concentrated. The residue was dissolved in water/acetonitrile (3 mL), cooled to 0°C, and acidified with TFA (0.21 mL, 2.7 mmol). The faintly yellow solution was concentrated and the residue was purified by reversed-phase flash chromatography (C-18 silica gel, 25% AN/W-TFA). Concentration and lyophilization gave the title compound (123 mg, 67%) as a colorless powder. HPLC k' = 1.84 (PRP-1®, 30% AN/W-TFA); ¹H NMR (400 MHz, CD₃OD) δ 7.67 (app d, 2H), 7.40 (app d, 2H), 6.96-7.20 (m, 8H), 5.13 (d, J=16.8 Hz, 1H), 3.96 (d, J=16.8 Hz, 1H), 3.66-3.81 (m, 2H), 3.45-3.58 (m, 1H), 3.49 (s, 3H), 2.92 (app dd, 1H), 2.77 (dd, J=17.0, 9.1 Hz, 1H), 2.54-2.68 (m, 3H), 2.38 (dd, J=17.0, 4.8 Hz, 1H); MS(ES) m/e 499.0 [M+H]⁺; Anal.
   (C₂₉H₃₀N₄O₄·1.5(CF₃CO₂H)·0.5 H₂O) calcd: C, 56.64; H, 4.83; N, 8.26. found: C, 56.77; H, 5.05; N, 8.35.

### Preparation B

### Preparation of (R,S)-8-[[[4-(aminoiminomethyl)phenyl]-methylamino]carbonyl]-2,3,4,5-tetrahydro-1-methyl-3-oxo-4-(2-phenylethyl)-1H-1,4-benzodiazepine-2-acetic acid

a) t-butyl 4-[N-(t-butoxycarbonyl)-N-(phenylethyl)aminomethyl]-3-(methylamino)benzoate
   Formic acid (0.166 g, 3.6 mmol) was added slowly to acetic anhydride (0.36 g, 3.5 mmol) at 0°. This solution was stirred at 0° for 10 min, then at 55°C for two hours, cooled to RT, and diluted with THF (15 mL). To this solution was added dropwise a solution of the compound of Preparation 16(b) (0.5 g, 1.17 mmol) in THF (3 mL). The solution was stirred 3 h and concentrated to give a colorless oil. The residue was redissolved in THF (20 mL), cooled to 0°C and 2.0M borane-methyl sulfide in THF was added (0.22 g, 2.9 mmol, 1.45 mL). After gas evolution had stopped, the solution was heated at reflux 3 h, cooled to RT and methanol (5.0 mL) was added. The resulting solution was refluxed 1 h and concentrated to give the title compound (0.5 g, 98%) as a colorless oil. ¹H NMR (400 MHz, CDCl₃) δ 7.1-7.3 (m, 5H), 7.05 (d, 2H), 7.0 (d, 1H), 5.5 (b, 1H), 4.3 (s, 2H), 3.3 (t, 2H), 2.9 (s, 3H), 2.65 (t, 2H), 1.6 (s, 9H), 1.45 (s, 9H); TLC R_{f} 0.75 (1:4 ethyl acetate:hexane).
b) t-butyl (E/Z)-4-[N-(t-butoxycarbonyl)-N-(2-phenylethyl)aminomethyl]-3-[N-[2-(1,4-dimethoxy-1,4-dioxo-2-butenyl)]-N-(methyl)amino]benzoate
   Dimethyl acetylenedicarboxylate (0.34 g, 2.4 mmol) was added to a solution of the compound of B(a) (1.0 g, 2.7 mmol) in methanol (50 mL). The solution was heated to reflux for 1 h, treated with dimethyl acetylenedicarboxylate (0.17 g, 1.2 mmol) and heated at reflux for an additional 1 h. The mixture was cooled, filtered, concentrated and the residual yellow oil was chromatographed (silica gel, 25% ethyl acetate/hexane) to give the title compound (0.5 g, 80%). (1:1;E:Z) ¹H NMR (400 MHz, CDCl₃) δ 7.9 (d, 2H), 7.7 (s, 2H), 7.3-7.1 (m, 12H), 6.2 (s, 1H), 5.2 (s, 1H), 4.7 (d, 2H), 4.3-(m, 2H), 3.9 (s, 3H), 3.85 (s, 3H), 3.8 (s, 3H), 3.7 (s, 3H), 3.6-3.2 (m, 8H), 3.0 (b, 3H), 2.9 (b, 3H), 1.6 (s, 18H), 1.4 (d, 18H).
c) t-butyl 4-[N-(t-butoxycarbonyl)-N-(2-phenylethyl)-aminomethyl]-3-[N-[2-(1,4-dimethoxy-1,4-dioxo-2-butyl)]-N-(methyl)amino]benzoate
   A solution of the compound of B(b) in methanol (50 mL) containing 10% Pd/carbon (0.5 g) was hydrogenated (3.10 bar) at RT. After 4 h, the suspension was filtered and concentrated to give a pale yellow oil. The residue was purified by chromatography (silica gel, 1:4 ethyl acetate:hexane) to give the title compound as a pale yellow oil (0.17 g, 40%). ¹H NMR (400 MHz, CDCl₃) δ 7.8 (s, 1H), 7.7 (d, 1H), 7.0-7.3 (m, 6H), 4.4-4.6 (m, 2H), 4.05 (m, 1H), 3.7 (s, 3H), 3.6 (s, 3H), 3.2-3.5 (m, 2H), 3.0 (dd, 1H), 2.7-2.9 (m, 3H), 2.65 (s, 3H), 1.6 (s, 9H), 1.4 (m, 9H).
d) methyl (R,S)-8-carboxy-1-methyl-2,3,4,5-tetrahydro-3-oxo-4-(phenylethyl)-1H-1,4-benzodiazepine-2-acetate
   TFA (50 mL) was added to a solution of the compound of B(c) (0.17 g, 0.3 mmol) in CH₂Cl₂ (10 mL). The mixture was stirred for 4 h and concentrated to yield 4-[N-(2-phenylethyl)aminomethyl]-3-[N-[2-(1,4-dimethoxy-1,4-dioxo-2-butyl)]-N-(methyl)amino]-benzoate as a yellow solid.
   The yellow solid was dissolved in methanol (20 mL) and treated with 25% sodium methoxide in methanol (0.2 mL, 0.87 mmol). The mixture was heated to 50°C for 2 h, cooled and treated with 1M hydrogen chloride in ether (2 mL). The mixture was concentrated to give the title compound as a yellow solid (0.12 g, 94%). ¹H NMR (400 MHz, CD₃OD) δ 7.55 (s, 1H), 7.5 (d, 1H), 7.1 (m, 4H), 7.0 (m, 2H), 5.2 (d, 1H), 4,8 (m, 1H), 4.1(m, 2H), 3.7 (s, 3H), 3.5 (m, 1H), 3.0 (dd, 1H), 2.8 (t, 2H), 2.7 (dd, 1H), 2.6 (s, 3H).
e) methyl (R,S)-8-[[[4-[N-(benzyloxycarbonyl)-aminoiminomethyl]phenyl]methylamino]carbonyl]-2,3,4,5-tetrahydro-1-methyl-3-oxo-4-(2-phenylethyl)-1H-1,4-benzodiazepine-2-acetate
   Using the procedure of Preparation A(h), except substituting the compound B(d) for the compound A(g), the title compound was prepared (0.040 g, 35%). ¹H NMR (400 MHz, CDCl₃) δ 7.75 (d, 2H), 7.25-7.45 (m, 6H), 7.0-7.2 (m, 6H), 6.95 (s, 1H), 6.7 (m, 2H), 5.2 (s, 2H), 5.0 (d, 1H), 4.6 (m, 1H), 3.9 (m, 1H), 3.65 (m, 4H), 3.6 (m, 1H), 3.5 (s, 3H), 2.9 (dd, 1H), 2.75 (t, 2H), 2.55 (dd, 1H), 2.5 (s, 3H).
f) methyl (R,S)-8-[[[4-(ammoiminomethyl)phenyl]methylamino]-carbonyl]-2,3,4,5-tetrahydro-1-methyl-3-oxo-4-(2-phenylethyl)- 1H-1,4-benzodiazepine-2-acetate
   A solution of the compound of B(e) (0.04 g, 0.06 mmol) in methanol (25 mL) containing 1.0M hydrogen chloride in ether (0.6 mL) was treated with 10% palladium on carbon (0.06 g) and the mixture was shaken in a hydrogen atmosphere ( 2.76 bar) for 1 h. The mixture was filtered and concentrated to yield the title compound.
g) (R,S)-8-[[[4-(aminoiminomethyl)phenyl]methylamino]-carbonyl]-2,3,4,5-tetrahydro-1-methyl-3-oxo-4-(2-phenylethyl)-1H-1,4-benzodiazepine-2-acetic Acid
   Using the procedure of Preparation A(j), except substituting the compound B(f) for the compound A(i), gave a residue which was purified by HPLC (YMC-ODS AQ, 33% AN/W-TFA) to yield the title compound (0.012 g, 35%). ¹H NMR (400 MHz, CD₃OD) δ 7.7 (d, 2H), 7.45 (d, 2H), 7.05-7.15 (m, 3H), 7.0 (d, 2H), 6.9 (m, 2H), 6.8 (d, 1H), 5.1 (dd, 1H), 4.7 (m, 1H), 4.0 (d, 1H), 3.9 (m, 1H), 3.55 (m, 1H), 3.5 (s, 3H), 2.85 (dd, 1H), 2.7 (t, 2H), 2.5 (dd, 1H), 2.4 (s, 3H); MS(ES) m/e 514.2 [M+H]⁺.

### Examples 1 and 2 and Preparation C

a) Using the procedure of Preparation 17(c), except substituting 2-(4-pyridyl)ethylamine, 2-(2-pyridyl)ethylamine and the compound of Preparation 1(b) for 4-(methylamino)-(N-benzyloxycarbonyl)benzamidine, gave the following title compounds:
   1(a) methyl (R,S)-2,3,4,5-tetrahydro-3-oxo-4-(2-phenylethyl)-8-[[[2-(4-pyridyl)ethyl]amino]carbonyl]-1H-1,4-benzodiazepine-2-acetate. HPLC RT 17.8 min (YMC ODS-AQ, 6 x 250 mm, 1.5 mL/min, gradient, A:acetonitrile B:water-0.1% trifluoracetic acid, 10-80% A during 20 min, UV detections at 220 nm); ¹H NMR (250 MHz, CDCl₃) δ 8.5 (d, 2H), 7.1-7.3 (m, 7H), 6.95 (s, 1H), 6.8 (s, 2H), 6.05 (m, 1H), 5.25 (d, 1H), 4.5 (m, 1H), 4.25 (m, 1H), 3.75 (s, 3H), 3.69-3.7 (m, 3H), 2.95-3.0 (m, 2H), 2.8 (m, 2H), 2.65 (d, 2H).
      Preparation C methyl (R,S)-2,3,4,5-tetrahydro-3-oxo-4-(2-phenylethyl)-8-[[[2-(2-pyridyl)ethyl]amino]carbonyl]-1H-1,4-benzodiazepine-2-acetate. HPLC RT 18.3 min (YMC ODS-AQ, 6 x 250 mm, 1.5 mL/min, gradient, A:acetonitrile B:water-0.1% tridfluoracetic acid, 10-80% A during 20 min, UV detections at 220 nm).
   2(a) methyl (R,S)-2,3,4,5-tetrahydro-3-oxo-4-(2-phenylethyl)-8-[[[3-(4-pyridyl)propyl]amino]carbonyl]-1H-1,4-benzodiazepine-2-acetate. This compound was purified by flash chromatography (silica gel, chloroform, 5:95 methanol:chloroform). ¹H NMR (250 MHz, CDCl₃/CD₃OD) δ 8.48 (d, 2H), 7.46-6.68 (m, 12H), 6.17 (m, 1H), 5.28 (d, 1H), 4.98 (q, 1H), 4.32 (d, 1H), 3.90-3.55 (m), 3.72 (s, 3H), 2.98 (dd, 1H), 2.88-2.50 (m), 1.95 (m).
b) Using the procedure of Preparation A(j), except substituting the compounds of Examples 1(a) and 2(a) for the compound A(i), gave the following title compounds.
   1(b) (R,S)-2,3,4,5-tetrahydro-3-oxo-4-(2-phenylethyl)-8-[[[2-(4-pyridyl)ethyl]amino]carbonyl]-1H-1,4-benzodiazepine-2-acetic acid. ¹H NMR(DMSO d₆/TFA, 360 MHz) δ 8.8 (d, 2H), 8.35 (m, 1H), 7.95 (d, 2H), 7.2 (m, 2H), 7.1 (m, 2H), 7.0 (m, 2H), 6.84 (d, 1H), 5.35 (d, 1H), 4.95 (d, 1H), 3.95 (d, 1H), 3.6 (m, 4H), 3.12 (m, 2H), 2.73 (m, 2H), 2.62 (m, 2H); MS(ES) m/e 473 [M+H]⁺.
   2(b) (R,S)-2,3,4,5-tetrahydro-3-oxo-4-(2-phenylethyl)-8-[[[3-(4-pyridyl)propyl]amino]carbonyl]-1H-1,4-benzodiazepine-2-acetic acid This compound was purified by chromatography (Sephadex LH20, water). MS(ES) m/e 487 [M+H]⁺; HPLC RT 10.10 min (PRP-1®, 4.6 x 250 mm, 1.5 mL/min, gradient, A:acetonitrile B:water/0.1% TFA, 20-50% A during 20 min, UV detection at 220 and 250 nm); TLC Rf 0.47 (silica, 3:1:1 n-BuOH:HOAc:H₂O); Anal. (C₂₈H₃₀N₄O₄•3H₂O) calcd: C, 62.21; H, 6.71; N, 10.36. found: C, 62.21; H, 6.00; N, 10.36.

### Example 3

a) Using the procedure of Preparation 17(c) and Preparation A(h), except substituting the compound of Preparation 2 for 4-(methylamino)-(N-benzyloxycarbonyl)benzamidine, gave the following title compound:
   3(a) methyl (R,S)-8-[[[5-aminopentyl]amino]carbonyl]-2,3,4,5-tetrahydro-3-oxo-4-(2-phenylethyl)-1H-1,4-benzodiazepine-2-acetate. TLC R_{f} 0.45 (silica, 1:20 CH₂Cl₂:methanol); ¹H NMR (400 MHz, CD₃OD) δ 6.9-7.4 (m, 13H), 5.4 (d, 1H), 5.1 (m, 1H), 5.05 (s, 2H), 3.87 (d, 1H), 3.7 (s, 3H), 3.6.-3.8 (m, 2H), 3.1 (t, 2H), 2.95 (dd, 1H), 2.75 (t, 2H), 2.65 (dd, 1H), 1.45-1.65 (m, 4H), 1.3-1.4 (m, 2H).
b) Using the procedure of Preparation A(j), except substituting the compound of Example 3(a) for the compound A(i) gave the following title compound:
   3(b) (R,S)-8-[[(5-aminopentyl)amino]carbonyl]-2,3,4,5-tetrahydro-3-oxo-4-(2-phenylethyl)-1H-1,4-benzodiazepine-2-acetic acid . ¹H NMR (400 MHz, CD₃OD) δ 7.05-7.25 (m, 5H), 7.0 (s, 1H), 6.95 (s, 2H), 5.45 (d, 1H), 5.1 (m, 1H), 3.9 (d, 1H), 3.8 (m, 1H), 3.7 (m, 1H), 3.4 (t, 2H), 2.95-2.85 (m, 3H), 2.7-2.8 (m, 2H), 2.55-2.65 (dd, 1H); MS(ES) m/e 453.4 [M+H]⁺.

### Preparation D

### Preparation of (R,S)-8-[[[4-(Propyl)aminomethyl)phenyl]-amino]carbonyl]-2,3,4,5-tetrahydro-3-oxo-4-(2-phenylethyl)-1H-1,4-benzodiazepine-2-acetic Acid

a) methyl (R,S)-8-[[[N-(t-butoxycarbonyl)-4-(propyl)-aminomethyl)phenyl]amino]carbonyl]-2,3,4,5-tetrahydro-3-oxo-4-(2-phenylethyl)-1H-1,4-benzodiazepine-2-acetate
   Using the procedure of Preparation 17(c), except substituting the compound of Preparation 3(b) for 4-(methylamino)-(N-benzyloxycarbonyl)benzamidine gave the title compound. ¹H NMR (400 MHz, CDCl₃) δ 7.6 (d, 2H), 7.0-7.3 (m, 9H), 6.9 (d, 1H), 5.3 (d, 1H), 5.0 (m, 1H), 4.4 (m, 2H), 4.35 (d, 1H), 3.7 (s, 3H), 3.6-3.7 (m, 2H), 3.0-3.2 (m, 2H), 2.95 (dd, 1H), 2.75 (m, 2H), 2.65 (dd, 1H), 1.5-1.7 (m, 9H), 0.9 (t, 3H)
b) methyl (R,S)-8-[[[4-(propyl)aminomethyl)phenyl]amino]-carbonyl]-2,3,4,5-tetrahydro-3-oxo-4-(2-phenylethyl)-1H-1,4-benzodiazepine-2-acetate
   The compound D(a) (350 mg, 0.56 mmol) was dissolved in a mixture of CH₂Cl₂ (25 mL) and TFA (5 mL). After 1 h, the mixture was concentrated to give the title compound.
c) (R,S)-8-[[[4-(propyl)aminomethyl)phenyl]amino]carbonyl]-2,3,4,5-tetrahydro-3-oxo-4-(2-phenylethyl)-1H-1,4-benzodiazepine-2-acetic Acid
   Using the procedure of Preparation A(j), except substituting the compound D(b) for the compound A(i) gave the title compound (142 mg, 40%). ¹H NMR (400 MHz, CD₃OD) δ 7.8 (d, 2H), 7.45 (d, 2H), 7.0-7.25 (m, 8H), 5.5 (d, 1H), 5.1 (m, 1H), 4.2 (s, 2H), 3.9 (d, 1H), 3.8 (m, 1H), 3.7 (m, 1H), 2.9-3.1(m, 3H), 2.75 (m, 2H), 2.65 (dd, 1H), 1.7 (m, 2H), 1.0 (t, 3H); MS(ES) m/e 515.2 [M+H]⁺.

### Example 4

a) Using the procedure of Preparation D(a)-(c), except substituting the compound of Preparation 4(c) for the compound of Preparation 3(b), gave:
   4(a) (R,S)-8-[[[3-[4-[N-(t-butoxycarbonyl)-piperidinyl]propyl]amino]carbonyl]-2,3,4,5-tetrahydro-3-oxo-4-(2-phenylethyl)-1H-1,4-benzodiazepine-2-acetic acid
b) Using the procedure of Preparation D(b), except substituting the compound of Example 4(a) for the compound D(a), and purifying the residue with chromatography (Sephadex LH20) gave:
   4(b) (R,S)-8-[[[3-[4-piperidinyl]propyl]amino]carbonyl]-2,3,4,5-tetrahydro-3-oxo-4-(2-phenylethyl)-lH-1,4-benzodiazepine-2-acetic acid . ¹H NMR (250 MHz, DMSO-d6) δ 7.40-6.70 (m, 8H), 5.38 (dd, 1H), 4.99 (m, 1H), 4.29 (s, 1H), 4.00 (dd, 1H), 3.70-2.90 (m), 2.90-2.20 (m), 1.72 (d, 2H), 1.48 (m, 3H), 1.17 (m, 4H); TLC R_{f} 4.90 (silica gel, 3:1:1 n-BuOH:HOAc:water); HPLC RT 9.82 min (PRP-1®, 4.6 x 250 mm, 20-50% AN/W-TFA, gradient 20 minutes @1.5 mL/min, UV detection at 220 and 250 nm); MS(ES) m/e 493.2 [M+H]⁺; Anal. (C₂₈H₃₆N₄O₄•HOAC•2H₂O) calcd: C, 61.21; H, 7.53; N, 9.52. found: C, 61.56; H, 7.17, N, 9.91.

### Examples 5, 6 and 7

a) Using the procedure of Preparation 17(c), except substituting 4-(aminomethyl)pyridine, 2-(1H-imidazol-1-yl)ethanamine (Syn. Comm., 21, 535 (1991)) and 3-(1H-imidazol-1-yl)propanamine for 4-(methylamino)-(N-benzyloxycarbonyl)]benzamidine and substituting triethylamine for pyridine, gave:
   5(a) methyl (R,S)-2,3,4,5-tetrahydro-3-oxo-4-(2-phenylethyl)-8-[[[4-(pyridyl)methyl]amino]carbonyl]-1H-1,4-benzodiazepine-2-acetate
   6(a) methyl (R,S)-2,3,4,5-tetrahydro-8-[[[2-(1H-imidazol-1-yl)ethyl]amino]carbonyl]-3-oxo-4-(2-phenylethyl)-1H-1,4-benzodiazepine-2-acetate. MS(ES) m/e 476.2 [M+H]⁺, ¹NMR (400 MHz, MeOD) δ 2.66-2.76 (3H, dd+t), 2.93 (1H, dd), 3.58-3.70 (7H, m), 3.92 (1H, d), 4.30 (2H, t), 5.06 (1H, m), 5.37 (1H, d), 6.90-7.25 (10H, m), 7.92(1H, s).
   7(a) methyl (R,S)-2,3,4,5-tetrahydro-8-[[[3-(1H-imidazol-1-yl)propyl]amino]carbonyl]-3-oxo-4-(2-phenylethyl)-1H-1,4-benzodiazepine-2-acetate. MS(ES) m/e 490.2 [M+H]⁺, ¹NMR (400 MHz, MeOD) δ 2.08 (2H, m), 2.62-2.77 (3H, dd+t), 2.93 (1H, dd), 3.34 (2H, t), 3.61 (1H, m), 3.67-3.77 (4H, s+m), 3.86 (1H, d), 4.08 (2H, t), 5.08 (1H, m), 5.39 (1H, d), 6.90-7.23 (11H, m), 7.75 (1H, bs).
b) Using the procedure of Preparation A(j), except substituting the compounds of Examples 5, 6 and 7(a) for the compound A(i), gave the title compounds:
   5(b) (R,S)-2,3,4,5-tetrahydro-3-oxo-4-(2-phenylethyl)-8-[[[4-(pyridyl)methyl]amino]carbonyl]-1H-1,4-benzodiazepine-2-acetic acid : HPLC RT 8.2 min (YMC ODS-AQ, 50 x 250 mm, 80 mL/min, 30% AN/W-TFA, UV detection at 220 nm); MS(ES) m/e 459.2 [M+H]⁺; ¹NMR (400 MHz, DMSO) δ 2.55 (1H, dd), 2.67 (2H, t), 2.78 (1H, dd, J₁=9 Hz, J₂=17 Hz), 3.58 (2H, m), 4.00 (1H, d, J=17 Hz), 4.65 (2H, d, J=6 Hz), 5.03 (1H, m), 5.44 (1H, d, J=17 Hz), 7.00-7.29 (10H, m), 7.80 (2H, d, J=5 Hz), 8.78 (1H, bs), 9.10 (1H, t, J₁=6 Hz, J₂=12 Hz). Anal.(C₂₆H₂₆N₄O₄·1.5C₂HF₃O₂·0.5 H₂O) calcd: C, 54.55; H, 4.50; N, 8.77. found: C, 54.32; H, 4.61; N, 8.89.
   6(b) (R,S)-2,3,4,5-tetrahydro-8-[[[2-(1H-imidazol-1-yl)ethyl]amino]carbonyl]-3-oxo-4-(2-phenylethyl)-1H-1,4-benzodiazepine-2-acetic acid . HPLC RT 6.8 min (YMC ODS-AQ, 50 x 250 nm, 80 mL/min, 30% AN/W-TFA, UV detection at 220 nm); MS(ES) rule 462.2 [M+H]⁺; 1NMR (400 MHz, DMSO-d) δ 2.55 (1H, dd), 2.66 (2H, t), 2.78 (1H, dd), 3.55 (2H, t), 3.65 (2H, t), 4.00 (1H, d), 4.35 (2H, t), 5.00 (1H, m), 5.38 (1H, d), 6.00 (1H, bs), 6.87 (1H, d), 7.00 (1H, s), 7.07 (1H, d,), 7.13-7.28 (5H, m), 7.63 (1H, s), 7.73 (1H, s), 8.45 (1H, t), 9.09 (1H, s). Anal. (C₂₅H₂₇N₅O₄·1.5(C₂HF₃O₂)H₂O) calcd: C, 51.69; H, 4.73; N, 10.79. found: C, 51.46; H, 4.66; N, 10.70.
   7(b) (R,S)-2,3,4,5-terrahydro-8-[[[3-(1H-imidazol-1-yl)propyl]amino]carbonyl]-3-oxo-4-(2-phenylethyl)-1H-1,4-benzodiazepine-2-acetic acid . HPLC RT 8.6 min (YMC ODS-AQ, 50 x 250 nm, 80 mL/min, 30% AN/W-TFA, UV detection at 220 nm); MS(ES) m/e 476.2 [M+H]⁺; ¹NMR (400 MHz, DMSO-d) δ 2.06 (4H, bt), 2.51 (1H, dd), 2.66 (4H, bt), 2.77 (1H, dd), 3.22 (4H, m), 3.58 (4H, bt), 4.00 (1H, d), 4.20 (4H, t), 5.00 (1H, m), 5.40 (1H, d), 6.92 (1H, d), 7.05 (2H, m), 7.15 (2H, m), 7.23 (2H, m), 7.68 (1H, s), 7.80 (1H, s), 8.37 (1H, t), 9.12 (1H, s); Anal. (C₂₆H₂₉N₅O₄·1.75C₂HF₃O₂) calcd: C, 52.48; H, 4.59; N, 10.37. found: C, 52.61; H, 4.98; N, 10.38.

### Example 8-9

a) Using the procedure of Example 24(a) and 19(f), except substituting the compounds of Preparation 5(a) and (b) for 4-(aminomethyl)pyridine, and the procedure of Preparation B(f), except substituting palladium hydroxide for palladium on carbon, gave:
   8(a) methyl (R,S)-2,3,4,5-tetrahydro-3-oxo-4-(2-phenylethyl)-8-[[[2-(1-piperazinyl)ethyl]amino]carbonyl]-1H-1,4-benzodiazepine-2-acetate. HPLC RT 5.3 min (YMC ODS-AQ, 6 x 250 mm, 1.5 mL/min, 30% AN/W-TFA, UV detection at 220 nm).
   9(a) methyl (R,S)-2,3,4,5-tetrahydro-3-oxo-4-(2-phenylethyl)-8-[[[2-(1-piperazinyl)ethyl]methylamino]carbonyl]-1H-1,4-benzodiazepine-2-acetate
b) Using the procedure of Preparation A(j), except substituting the compounds of Example 8(a) and 9(a) for the compound of A(i) gave:
   8(b)(1) (R,S)-2,3,4,5-tetrahydro-3-oxo-4-(2-phenylethyl)-8-[[[2-(1-piperazinyl)ethyl]amino]carbonyl]-lH-1,4-benzodiazepine-2-acetic acid . HPLC RT 7.4 min (YMC ODS-AQ, 50 x 250 mm, 80 mL/min, 22% AN/W-TFA, UV detection at 220 nm); MS(ES) m/e 480.2 [M+H]⁺; ¹NMR (400 MHz, DMSO-d) δ 2.48-2.60 (1H, dd), 2.70 (2H, t), 2.82 (1H, dd), 3.20 (2H, bs), 3.89 (8H, bs), 3.58 (4H, m), 4.05 (1H, d), 5.00 (1H, t), 5.40 (1H, d), 6.97 (1H, d), 7.11 (2H, m), 7.18 (3H, m), 7.26 (1H, m), 8.50 (1H, bs). Anal. (C₂₆H₃₃N₅O₄·2.5(C₂HF₃O₂)·2(H₂O)) calcd: C,46.39; H, 4.96; N, 8.73. found: C, 46.35; H, 4.88; N, 8.90.
   9(b) (R,S)-2,3,4,5-tetrahydro-3-oxo-4-(2-phenylethyl)-8-[[[2-(1-piperazinyl)ethyl]methylamino]carbonyl]-1H-1,4-benzodiazepine-2-acetic acid . HPLC RT 9.8 min (YMC ODS-AQ, 50 x 250 nm, 80 mL/min, 25% AN/W-TFA, UV detection at 220 nm); MS(ES) m/e 494.2 [M+H]⁺; ¹NMR (400 MHz, DMSO-d) δ 2.52 (1H, dd), 2.68 (2H, m), 2.75 (1H, dd), 2.92 (3H, s), 2.94-3.30 (8H, m), 3.50-4.10 (7H, m), 5.03 (1H, m), 5.40 (1H, d), 6.49 (1H, d), 6.57 (1H, s), 7.02 (1H, d), 7.10-7.25 (5H, m); Anal.(C₂₇H₃₅N₅O₄·2.75 C₂HF₃O₂) calcd: C, 48.36; H, 4.75; N, 8.68. found: C, 48.23; H, 4.86; N, 8.61.

### Examples 10-11

a) Using the procedure of Preparation 17(c) and the procedure of 8(b), except substituting the compounds of Preparation 6 and 7 for the compound A(i) and deleting pyridine, gave:
   10(a) methyl (R,S)-8-[[[6-aminohexyl]amino]carbonyl]-2,3,4,5-tetrahydro-3-oxo-4-(2-phenylethyl)-1H-1,4-benzodiazepine-2-acetate. TLC Rf 0.35 (Kieselgel 60 F₂₅₄, 15:3:2 2-butanol:formic acid:water); MS(ES) m/e 467.
   11(a) methyl (R,S)-8-[[[4-aminobutyl]amino]carbonyl ]-2,3,4,5-tetrahydro-3-oxo-4-(2-phenylethyl)-1H-1,4-benzodiazepine-2-acetate. MS(ES) m/e 453.2.
b) Using the procedure of Preparation A(j), except substituting the compounds of Examples 10(a) and 11(a) for A(i) and substituting lithium hydroxide for sodium hydroxide, gave:
   10(b) (R,S)-8-[[[6-aminohexyl]amino]carbonyl]-2,3,4,5-tetrahydro-3-oxo-4-(2-phenylethyl)-1H-1,4-benzodiazepine-2-acetic acid. MS(ES) m/e 467. Anal. (C26H34N4O4. 1.4 C₂HO₂F₃) calcd: C, 55.13 ; H, 5.55; N, 8.8. found: C, 55.24; H, 5.70; N, 8.9.
   11(b) (R,S)-8-[[[4-aminobutyl]amino]carbonyl]-2,3,4,5-tetrahydro-3-oxo-4-(2-phenylethyl)-1H-1,4-benzodiazepine-2-acetic acid. MS(ES) m/e 439.2. Anal. (C₂₄H₃₀N₄O_{4.}1.5 H₂O.0.1 HCl) calcd: C, 61.03; H, 7.02; N, 11.62; Cl, 1.10. found: C, 61.44; H, 7.11; N, 11.94; Cl, 0.76.

### Preparation E

### Preparation of (R,S)-2,3,4,5-tetrahydro-3-oxo-4-(2-phenylethyl)-8-[[[3-(4-pyrazolyl)propyl]amino]carbonyl]-1H-1,4-benzodiazepine-2-acetic acid

a) methyl (R,S)-2,3,4,5-tetrahydro-3-oxo-4-(2-phenylethyl)-8-[[[3-(4-pyrazolyl)propyl]amino]carbonyl]-1H-1,4-benzodiazepine-2-acetate
   The compound of Preparation 16(e) (776 mg, 2.0 mmol) dissolved in DMF (30 mL) was treated with 3,3-DCC (458 mg, 2.2 mmol), 1-HOBT (285 mg, 2.1 mmol) and adjusted to pH 7 with triethylamine. The mixture was treated with 2-(4-pyrazolyl)ethanamine dihydrochloride (300 mg, 2.4 mmol) (*J. Am. Chem. Soc.,* 75, 4048 (1953)), stirred at RT for 48 h, concentrated and the residue was purified by flash chromatography (silica gel, step gradient, chloroform to 95:5 chloroform:methanol) to yield the title compound (1 g, 90%). MS ES [M+H]⁺= 490; ¹H NMR (250 MHz) δ 7.44 (s, 2H), 7.29-7.19 (m, 5H), 7.18-7.09 (m, 3H), 5.30 (dd, 2H J=7.5 Hz), 4.98-4.95 (m, 1H), 3.73 (s, 3H), 3.70-3.60 (m, 3H), 3.43 (d, 2H, J=7.5 Hz), 3.00 (dd, 2H, J=7.0 Hz) 2.77-2.65 (m, 2H), 2.63 (t, 2H, J=7 Hz),1.88 (p, 2H, J=7.5).
b) (R,S)-2,3,4,5-tetrahydro-3-oxo-4-(2-phenylethyl)-8-[[[3-(4-pyrazolyl)propyl]amino]carbonyl]- 1H-1,4-benzodiazepine-2-acetic acid
   Using the procedure of Preparation A(j), except substituting the compound E(a) for the compound A(i), gave the title compound. MS(ES) 476 [M+H]⁺; ¹H NMR (400 MHz) δ 7.66 (s, 2H), 7.21-7.13 (m, 5H), 7.00-6.96 (m, 3H), 5.47 (d, 1H, J=16 Hz), 5.10 (m, 1H), 3.70 (d, 1H, J=16 Hz), 3.80-3.71 (m, 1H), 3.70-3.60 (m, 1H), 3.32 (t, 2H, J=1.6 Hz), 2.95 (dd, 2H, J=8 Hz), 2.75 (m, 2H), 2.66 (t, 2H, J=8 Hz), 1.89 (p, 2H, J=8 Hz).

### Examples 12 and 13

a) Using the procedure of Preparation E(a), except substituting histamine and 2-(1-methyl-1H-imidazol-5-yl)ethanamine for 2-(4-pyrazolyl)ethanamine dihydrochloride and substituting 1-(3-dimethylaminopropyl)-3-ethylcarbodiimide hydrochloride for 1,3-dicyclohexylcarbodiimide gave:
   12(a) methyl (R,S)-2,3,4,5-tetrahydro-8-[[[2-(1H-imidazol-4-yl)ethyl]amino]carbonyl]-3-oxo-4-(2-phenylethyl)-1H-1,4-benzodiazepine-2-acetate
   13(a) methyl (R,S)-2,3,4,5-tetrahydro-8-[[[2-(1-methyl-1H-imidazol-5-yl)ethyl]amino]carbonyl]-3-oxo-4-(2-phenylethyl)-1H-1,4-benzodiazepine-2-acetate
b) Using the procedure of Preparation A(j), except substituting the compounds of Examples 12 and 13(a) for the compound A(i), gave:
   12(b) (R,S)-2,3,4,5-tetrahydro-8-[[[2-(1H-imidazol-4-yl)ethyl]amino]carbonyl]-3-oxo-4-(2-phenylethyl)-lH-1,4-benzodiazepine-2-acetic acid. MS(ES) m/e 462.2 [M+H]⁺; HPLC k' 3.2 (YMC ODS AQ, 22% AN/W-TFA, UV detection at 220 nm); TLC R_{f} 0.85 (silica gel, 15:5:10:10 pyridine:acetic acid:butanol:water). Anal. (C₂₅H₂₇N₅O₄·1.25 H₂O) calcd: C, 62.03; H, 6,14; N, 14.47. found: C, 61.89; H, 6.37; N, 14.20.
   13(b) (R,S)-2,3,4,5-tetrahydro-8-[[[2-(1-methyl-1H-imidazol-5-yl)ethyl]amino]carbonyl]-3-oxo-4-(2-phenylethyl)-1H-1,4-benzodiazepine-2-acetic acid. MS(ES) m/e 476 [M+H]⁺; HPLC k' 2.03 (YMC ODS AQ, 25% AN/W-TFA, UV detection at 220 nm); TLC R_{f} 0.90 (silica gel, 15:5:10:10 pyridine:acetic acid:butanol:water); Anal. (C₂₆H₂₉N₅O₄·5 HCl· 0.5 H₂O) calcd: C, 46.83; H, 5.29; N, 10.50. found: C, 47.20; H, 5.70; N, 10.47.

### Example 14

### Preparation of(R,S)-8-[[[2-(4-pyridyl)ethyl]methyl-amino]carbonyl]-2,3,4,5-tetrahydro-3-oxo-4-(2-phenylethyl)-1H-1,4-benzodiazepine-2-acetic acid

a) methyl (R,S)-8-[[[2-(4-pyridyl)ethyl]methylamino]-carbonyl]-2,3,4,5-tetrahydro-3-oxo-4-(2-phenylethyl)-1H-1,4-benzodiazepine-2-acetate
   Using the procedure of Example 12(a), except substituting N-methyl-2-(4-pyridyl)ethanamine for histamine and deleting the diisopropylethylamine, gave the title compound. HPLC RT 16.6 min (YMC ODS-AQ, 6 x 250 mm, 1.5 mL/min, gradient, A:acetonitrile B:water-0.1% trifluoracetic acid, 10-80% A during 20 min, UV detection at 220 nm; MS(ES) m/e 487 [M+H]⁺.
b) (R,S)-8-[[[2-(4-pyridyl)ethyl]methylamino]carbonyl]-2,3,4,5-tetrahydro-3-oxo-4-(2-phenylethyl)-1H-1,4-benzodiazepine-2-acetic Acid
   Using the procedure of Preparation A(j), except substituting the compound of Example 14(a) for the compound A(i), gave the title compound. ¹H NMR(DMSO d₆/TFA, 360 MHz) δ 8.8 (br, 2H), 8.0 (br, 1H), 7.7 (br, 1H), 7.15 (m, 5H), 6.0 (br, 1H), 6.3 (br, 2H), 5.35 (d, 1H), 5.0 (m, 1H), 3.95 (d, 1H), 3.75 (br, 2H), 3.6 (m, 3H), 3.15 (br, 2H), 2.9 (br, 3H), 2.75 (m, 1H), 2.65 (m, 2H); MS(ES) m/e 487 [M+H]⁺.

### Example 15

### Preparation(R,S)-2,3,4,5-tetrahydro-3-oxo-4-(2-phenylethyl)-8-[[[2-(4-piperidinyl)ethyl]amino]carbonyl]-1H-1,4-benzodiazepine-2-acetic acid

a) methyl (R,S)-2,3,4,5-tetrahydro-3-oxo-4-(2-phenylethyl)-8-[[[2-(4-piperidinyl)ethyl]amino]carbonyl]-1H-1,4-benzodiazepine-2-acetate
   A solution of the compound of Preparation C (0.45 g, 1 mmol) and 0.6N hydrochloric acid (3.1 mL) in methanol was treated with platinum oxide (90 mg) and hydrogenated (3.10 bar) for 5 h. The mixture was degassed and platinum oxide (74 mg) was added and the mixture was hydrogenated ( 3.10 bar) for 16 h, filtered and concentrated to yield the title compound. MS(ES) m/e 479.2 [M+H]⁺.
b) (R,S)-2,3,4,5-tetrahydro-3-oxo-4-(2-phenylethyl)-8-[[[2-(4-piperidinyl)ethyl]amino]carbonyl]-1H-1,4-benzodiazepine-2-acetic acid bis(trifluoroacetate)
   Using the procedure of Preparation A(j), except substituting the compound of Example 15(a) for A(i), gave the title compound. HPLC RT 16.1 min (YMC ODS-AQ, 6 x 250 mm, 1.5 mL/min, gradient, A:acetonitrile B:water-0.1% trifluoracetic acid, 10-80% A during 20 min, UV detection at 220 nm); ¹H NMR(DMSO d₆/TFA, 360 MHz) δ 8.5 (br, 1H), 8.3 (m, 2H), 7.3 (m, 2H), 7.15 (m, 2H), 7.05 (m, 2H), 6.9 (d, 1H), 5.39 (d, 1H), 5.0 (m, 1H), 4.0 (d, 1H), 3.55 (m, 2H), 3.25 (d, 3H), 2.55-2.8 (m, 3H), 1.2-1.8 (m, 9H); MS(ES) m/e 479 [M+H]⁺.

### Example 16

### Preparation (R,S)-2,3,4,5-tetrahydro-3-oxo-4-(2-phenylethyl)-8-[[[2-(4-piperidinyl)ethyl]methylamino]carbonyl]-1H-1,4-benzodiazepine-2-acetic acid

a) methyl (R,S)-2,3,4,5-tetrahydro-3-oxo-4-(2-phenylethyl)-8-[[[2-(4-piperidinyl)ethyl]methylamino]carbonyl]-1H-1,4-benzodiazepine-2-acetate
   A solution of the compound of Example 14(a) (0.1 g, 0.2 mmol) and 0.6N hydrochloric acid (0.6 mL) in methanol (30 mL) was treated with platinum oxide (5 mg) and hydrogenated ( 3.10 bar) overnight. The mixture was filtered and concentrated to give the title compound. MS(ES) m/e 493 [M+H]⁺.
b) (R,S)-2,3,4,5-tetrahydro-3-oxo-4-(2-phenylethyl)-8-[[[2-(4-piperidinyl)ethyl]methylamino]carbonyl]-1H-1,4-benzodiazepine-2-acetic Acid Bis(trifluoroacetate)
   Using the procedure of Preparation A(j), except substituting the compound of Example 16(a) for A(i), gave the title compound (130 mg, 92%). HPLC RT 16.6 min (YMC ODS-AQ, 6 x 250 mm, 1.5 mL/min, gradient, A:acetonitrile B:water-0.1% TFA, 10-80% A during 20 min, UV detections at 220 nm); ¹H NMR(DMSO d₆/TFA, 360 MHz) δ 8.5 (br, 1H), 8.1 (br, 1H), 7.2 (m, 5H), 70 (d, 1H), 6.5 (s, 1H), 6.45 (d, 1H), 5.4 (d, 1H), 4,95 (m, 1H), 3.95 (d, 1H), 3.6 (br, 2H), 3.44 (br, 1H), 3.09-3.25 (br, 4H), 2.69-2.85 (m, 6H), 1.85 (br, 1H), 1.1-1.86 (m, 8H); MS(ES) m/e 493 [M+H]⁺.

### Example 17

### Preparation (R,S)-2,3,4,5-tetrahydro-3-oxo-4-[2-(cyclohexyl)-ethyl]-8-[[[2-(4-piperidinyl)ethyl]methylamino]carbonyl]-1H-1,4-benzodiazepine-2-acetic acid

a) methyl (R,S)-2,3,4,5-tetrahydro-3-oxo-4-[2-(cyclohexyl)-ethyl]-8-[[[2-(4-pyridyl)ethyl]methylamino]carbonyl]-1H-1,4-benzodiazepine-2-acetate
   A solution of the compound of Example 14(a) (0.6 g, 1.2 mmol) and 0.6N hydrochloric acid (4.0 mL) in methanol (50 mL) was treated with platinum oxide (120 mg) and hydrogenated (3.10 bar) and the mixture was filtered and concentrated to give the title compound. HPLC RT 22 min (YMC ODS-AQ, 6 x 250 mm, 1.5 mL/min, gradient, A:acetonitrile B:water-0.1% TFA, 10-80% A during 20 min, UV detections at 220 nm; MS(ES) m/e 512 [M+H]⁺.
b) (R,S)-2,3,4,5-tetrahydro-3-oxo-4-[2-(cyclohexyl)ethyl]-8-[[[2-(4-pyridyl)ethyl]methylamino]carbonyl]-1H-1,4-benzodiazepine-2-acetic acid bis(trifluoroacetate)
   Using the procedure of Preparation A(j), except substituting the compound of Example 17(a) for A(i), gave the title compound (35 mg, 4%). HPLC RT 19.8 min (YMC ODS-AQ, 6 x 250 mm, 1.5 mL/min, gradient, A:acetonitrile B:water-0.1% trifluoracetic acid, 10-80% A during 20 min, UV detection at 220 nm); ¹H NMR(DMSO d₆/TFA, 360 MHz) δ 8.5 (br, 1H), 8.15 (br, 1H), 7.0 (d, 1H), 6.5 (s, 1H), 6.4 (d, 1H), 5.4 (d, 1H), 4.95 (m, 1H), 3,89 (d, 1H), 3.2-3.5 (br, 7H), 2.8 (br, 7H), 0.75-1.85 (m, 23H); MS(ES) m/e 499 [M+H]⁺.

### Example 18

### Preparation of methyl (R,S)-8-[[2-[4-[1-(methyl)pyridinium]-ethyl]methylamino]carbonyl]-2,3,4,5-tetrahydro-3-oxo-4-(2-phenylethyl)-1H-1,4-benzodiazepine-2-acetate Iodide

The compound of Example 14(a) (0.11 g, 2.2 mmol) dissolved in acetonitrile (10 mL) was treated with iodomethane (0.2 mL, 3.2 mmol) and heated to reflux for 24 h, concentrated *in vacuo* (toluene azeotrope) and triturated with ether to yield the title compound as a yellow solid. ¹H NMR(DMSO d₆/TFA, 250 MHz) δ 8.8 (br, 2H), 8.05 (br, 1H), 7.15 (m, 6H), 6.99 (br, 1H), 6.4 (br, 2H), 5.35 (d, 1H), 5.05 (m, 1H), 4.25 (s, 3H), 3.95 (d, 1H), 3.75 (br, 3H), 3.55 (s, 3H), 2.6-3.15 (m, 11H); MS(ES) m/e 641 [M-H], 515.2 [M+H]⁺.

### Example 19-20

### Preparation (R,S)-2,3,4,5-tetrahydro-3-oxo-4-[2-(cyclohexyl)ethyl]-8-[[[2-(1-methyl-4-piperidinyl)ethyl]methylamino]carbonyl]-1H-1,4-benzodiazepine-2-acetic acid and (R,S)-2,3,4,5-tetrahydro-3-oxo-4-(2-phenylethyl)-8-[[[2-(1-methyl-4-piperidinyl)ethyl]methylamino]carbonyl]-1H-1,4-benzodiazepine-2-acetic acid

a) A solution of the compound of Example 18 (0.3 g, 0.47 mmol) in methanol (35 mL) was treated with platinum oxide (27 mg) and hydrogenated for 8 h, filtered and concentrated to yield a mixture of the following compounds:
   19(a) methyl (R,S)-2,3,4,5-tetrahydro-3-oxo-4-[2-(cyclohexyl)ethyl]-8-[[[2-(1-methyl-4-piperidinyl)ethyl]-methylamino]carbonyl]-1H-1,4-benzodiazepine-2-acetate
   20(a) methyl (R,S)-2,3,4,5-tetrahydro-3-oxo-4-(2-phenylethyl)-8-[[[2-(1-methyl-4-piperidinyl)ethyl]-methylamino]carbonyl]-1H-1,4-benzodiazepine-2-acetate
b) Using the procedure of the Preparation A(j), except substituting the compounds of Examples 19-20(a) for the compound A(i), and purifying the resultant mixture by HPLC (YMC ODS AQ, 50 x 250 mm, 90 mL/min, 27% AN/W-TFA, UV detection at 220 nm) yielded:
   19(b) (R,S)-2,3,4,5-tetrahydro-3-oxo-4-[2-(cyclohexyl)ethyl)-8-[[[2-(1-methyl-4-piperidinyl)ethyl]methylamino]carbonyl]-1H-1,4-benzodiazepine-2-acetic acid: ¹H NMR(DMSO d₆/TFA, 360 MHz) δ 9.14 (br, 1H), 7.0 (d, 1H), 6.5 (s, 1H), 6.45 (d, 1H), 5.4 (d, 1H), 5.0 (m, 1H), 3,89 (d, 1H), 2.9-3.5 (br, 6H), 2.25-2.85 (m, 10H), 1.9 (br, 1H), 0.76-1.55 (m, 19H); MS(ES) m/e 513 [M+H]⁺.
   20(b) (R,S)-2,3,4,5-tetrahydro-3-oxo-4-(2-phenylethyl)-8-[[[2-(1-methyl-4-piperidinyl)ethyl]methylamino]carbonyl]-1H-1,4-benzodiazepine-2-acetic acid: ¹H NMR(DMSO d₆/TFA, 360 MHz) δ 9.1 (br, 1H), 7.2 (m, 5H), 7.0 (d, 1H), 6.5 (s, 1H), 6.45 (d, 1H), 5.4 (d, 1H), 5.0 (m, 1H), 3,95 (m, 1H), 3.55 (m, 9H), 3.4 (br, 2H), 3.2 (br, 2H), 2.85 (s, 3H), 2.65-2.75 (m, 7H), 1.95 (br, 1H), 1.0-1.49(br, 8H); MS(ES) m/e 507 [M+H]⁺.

### Example 21

### Preparation of 7-[3-(4-pyridyl)propyloxy]-2-(2-phenylethyl)-3-oxo-2,3,4,5-tetrahydro-1H-2-benzazepine-4-acetic acid

a) N-(2-phenylethyl)-4-nitrophthalimide
   A mixture of 4-nitrophthalic anhydride (18 g, 93.3 mmol) and phenethylamine (11.5 g, 95 mmol) in toluene (250 mL) was heated to reflux using a Dean-Stark trap to remove water. After 2.5 h, the mixture was concentrated to solid residue, which was triturated and filtered to give the titled compound (24.5 g, 89%): mp 142-3°C).
b) N-(2-phenylethyl)-4-methoxyphthalimide
   A stirred solution of the compound of Example 21(a) (18 g, 0.06 mol) in DMF (85 mL) at 0°C was treated with sodium methoxide (3.6 g, 0.066 mol) portionwise. The dark brown mixture was stirred at RT for 3.5 h, quenched with ice-water, extracted with ethyl acetate, dried (magnesium sulfate) and concentrated. Trituration with ether afforded the title compound (10.9 g, 64%): mp 112-4°C; MS(ES) m/e 282 [M+H]⁺; ¹H NMR (400 MHz, CDCl₃) δ 7.72 (d, J=8.25 Hz, 1H), 7.25-7.30 (m, 6H), 7.14 (dd, J=2.30, 8.25 Hz, 1H), 3.9 (s, 3H), 3.89 (t, J=7.87 Hz, 2H), 2.99 (t, J=7.87 Hz, 2H).
c) 2-hydroxymethyl-4-methoxy-N-(2-phenylethyl)-1-benzamide
   A solution of the compound of Example 21(b) (15 g, 0.05 mol) in isopropanol (300 mL) and water (60 mL) was treated with sodium borohydride (12 g, 0.3 mmol) portionwise. After 3.5 h, the mixture was concentrated to half of the original volume, quenched with 5% sodium bicarbonate solution, extracted with ethyl acetate, dried (sodium sulfate), concentrated to a small volume, and filtered to yield the title compound (10 g, 67%): mp 154-6°C; MS m/e 286 [M+H]⁺; ¹H NMR (400 MHz, CDCl₃) δ 7.39 (d, J=8.25 Hz, 1H), 7.12-7.28 (m, 5H), 6.81 (d, J=2.3 Hz, 1H), 7.35 (dd, J=2.30, 8.25 Hz, 1H), 4.46 (s, 2H), 3.76 (s, 3H), 3.61 (q, J=7.87 Hz, 2H), 2.89 (t, J=7.87 Hz, 2H).
d) 2-hydroxymethyl-4-methoxy-[N-(2-phenylethyl)]benzylamine
   To a solution of borane (800 mL, 1M in THF, 0.8 mol) at RT, a suspension of the compound of Example 21(c) (25 g, 0.088 mol) in THF (250 mL) was added over 0.5 h. The mixture was heated to reflux for 12 h, cooled and treated with 1N hydrochloric acid (150 mL). The mixture was heated for 1.5 h until evolution of hydrogen gas ceased, basified in the cold and extracted with ethyl acetate. The organic phase was dried (sodium sulfate) and concentrated to yield the title compound (24 g, 100%): MS m/e 272.2 [M+H]⁺; ¹H NMR (400 MHz, CDCl₃) δ 7.08-7.29 (m, 5H), 6.90 (d, J=2.62 Hz, 1H), 6.73 (dd, J=2.62, 8.29 Hz, 1H), 4.58 (s, 2H), 3.83 (s, 2H), 3.77 (s, 3H), 3.10 (t, J=6.78 Hz, 2H), 2.89 (t, J=6.78 Hz, 2H).
e) 2-hydroxymethyl-4-methoxy-[N-(2-phenylethyl)-N-(t-butyloxycarbonyl)]benzylamine
   A mixture of Example 21(d) (23 g, 0.085 mol), di-t-butyldicarbonate (19.3 g, 0.089 mmol) and triethylamine (12.3 mL, 0.089 mmol) in CH₂Cl₂ (250 mL) was stirred at RT for 18 h, concentrated and azeotroped twice with CH₂Cl₂. MS(ES) m/e 372.2 [M+H]⁺; ¹H NMR (400 MHz, CDCl₃) 8 7.13-7.28 (m, 5H), 6.90 (d, J=2.53 Hz, 1H), 6.79 (dd, J=2.53, 8.29Hz, 1H), 4.60 (bs, 2H), 4.43 (bs, 2H), 3.43 (s, 3H), 3.36 (t, J=7.90 Hz, 2H), 2.72 (t, J=7.90 Hz, 2H).
f) 5-methoxy-2-[N-(2-phenylethyl)-N-(t-butyloxycarbonyl)aminomethyl]benzaldehyde
   A suspension of the compound of Example 21(e) and MnO₂ (75 g, 0.86 mol) in CH₂Cl₂ (275 mL) was stirred at RT for 18 h. The reaction mixture was filtered and concentrated to give the titled compound (20 g, 70%): MS m/e 370.2 [M+H]⁺; ¹H NMR (400 MHz, CDCl₃) δ 10.16 (s, 1H), 7.34 (d, J=2.14 Hz, 1H), 7.07-7.27 (m, 7H), 4.79 (bs, 2H), 3.81 (s, 3H), 3.32 (bt, 2H), 2.75 (bt, 2H), 1.46 (s, 9H).
g) 2-[5-methoxy-2-[N-(2-phenylethyl)-N-(t-butoxy-carbonyl)aminomethyl]phenyl]-5-oxo-tetrahydro-3-furancarboxylic acid
   To a suspension of zinc chloride (27 g, 0.2 mol; flame-dried at reduced pressure), succinic anhydride (20 g, 0.2 mol), and the compound of Example 21(f) (20 g, 0.05 mol) in CH₂Cl₂ (150 mL), triethylamine (28 mL, 0.2 mol) was added dropwise at 0°C. The mixture was stirred for 18 h, diluted with CH₂Cl₂ (100 mL), washed with cold 1N hydrochloric acid, dried (sodium sulfate) and concentrated. The residue was triturated with ether/CH₂Cl₂ (75 mL, 2:1) and filtered. The filtrate was concentrated to give the title compound (23 g, 87%).
h) trans-3a,5,6,10b-tetrahydro-9-methoxy-5-(2-phenylethyl)-2H-furo[3,2-d][2]benzazepin-2,4(3H)-dione
   A mixture of the compound of Example 21(g) (23 g, 0.05 mol) and hydrogen chloride (200 mL, 4M in dioxane, 0.8 mol) in CH₂Cl₂ (150 mL) was stirred at RT for 18 h and concentrated to give 2-[5-methoxy-2-[N-(2-phenylethyl)aminomethyl]phenyl]-5-oxo-tetrahydro-3-furancarboxylic acid as grayish solid (18 g, 97%): MS(ES) m/e 370.2 [M+H]⁺.
   This solid, triethylamine (14 mL, 0.1 mol), and benzotriazol-1-yloxy-tris(dimethylamino)phosphonium hexafluorophosphate (22 g, 0.05 mol) were dissolved in CH₂Cl₂ (400 mL) and allowed to stir at RT overnight. The mixture was concentrated and the residue was purified by flash column chromatography (silica gel, 0.2% methanol/CH₂Cl₂) to yield the title compound (9.5 g, 55%): MS(ES) m/e 352.2 [M+H]⁺; ¹H NMR (400 MHz, CDCl₃) δ 7.12-7.16 (m, 3H), 6.97-7.00 (m, 3H), 6.91-6.94 (d, J=8.47 Hz, 1H), 6.79 (dd, J=2.75,8.47 Hz, 1H), 5.01 (d, J=17.26 Hz, 1H), 4.92 (d, J=11.48 Hz, 1H), 4.07 (dt, J=7.82 Hz, J=13.57 Hz, 1H), 3.92 (d, J=17.26 Hz, 1H), 3.85 (m, 1H), 3.79 (s, 3H), 3.52 (m, 1H), 3.25 (dd, J=17.61 Hz, J=11.76, 1H), 2.78 (m, 2H), 2.65 (dd, J=17.26 Hz, J=7.26 Hz, 1H).
i) ethyl 7-methoxy-2-(2-phenylethyl)-3-oxo-2,3,4,5-tetrahydro-1H-2-benzazepine-4-acetate
   A solution of the compound of Example 21(h) (0.123 g, 0.35 mmol) in 3N hydrochloric acid (15 mL) in ethanol (50 mL) was treated with palladium hydroxide (120 mg) and hydrogenated ( 3.45 bar) for 4 h. The mixture was filtered and the filtrate was concentrated. The residue was azeotroped with ethanol and toluene. The residue was dissolved in ethanol (75 mL) and heated to reflux in the presence of 4 M hydrogen chloride/dioxane (2 mL) for 2 h. The solution was concentrated to give the title compound (0.11 g, 83%): MS(ES) m/e 382 [M+H]⁺; ¹H NMR (400 MHz, CDCl₃) δ 7.08-7.26 (m, 5H), 6.90 (d J=8.31 Hz, 1H), 6.63 (dd, J=2.41, 8.28 Hz, 1H), 6.60 (d, J=2.41 Hz, 1H), 5.11 (d, J=16.47 Hz, 1H), 4.16 (q, J=7.10 Hz, 2H), 3.76 (s, 3H), 3.72 (d, J=16.47 Hz, 1H), 3.63 (m, 3H), 2.97 (m, 2H), 2.82 (m, 1H), 2.75 (t, J=7.30 Hz, 2H), 1.27 (t, J=7.10 Hz, 3H).
j) ethyl 7-hydroxy-2-(2-phenylethyl)-3-oxo-2,3,4,5-tetrahydro-1H-2-benzazepine-4-acetate
   To a solution of the compound of Example 21(i) (0.11 g, 0.29 mmol) and ethanethiol (1 mL, 13.5 mmol) in CH₂Cl₂ (20 mL) at 0°C was added aluminum chloride (0.2 g, 1.5 mmol). The mixture was warmed to RT over 1 h, diluted with CH₂Cl₂, washed with cold 3N hydrochloric acid, dried (sodium sulfate), and concentrated to give the title compound (0.08 g, 74%): MS(ES) m/e 368.2 [M+H]⁺.
k) ethyl 7-[3-(4-pyridyl)propyloxy]-2-(2-phenylethyl)-3-oxo-2,3,4,5-tetrahydro-1H-2-benzazepine-4-acetate
A mixture of the compound of Example 21(j) (20 mg, 0.05 mmol), 3-(4-pyridyl)propyl chloride hydrochloride (50 mg, 0.2 mmol), sodium iodide (3 mg), and sodium hydride (12 mg, 0.5 mmol) was heated at 90°C for 18 h. The mixture was quenched with ice water, extracted with ethyl acetate, dried (sodium sulfate) and concentrated to give the title compound (0.01 g, 42%): MS(ES) m/e 486 [M+H]⁺.
1) 7-[3-(4-pyridyl)propyloxy]-2-(2-phenylethyl)-3-oxo-2,3,4,5-tetrahydro-1H-2-benzazepine-4-acetic acid

A mixture the compound of Example 21(k) and 1N sodium hydroxide solution in methanol is stirred at RT for 5 h and acidified with 1N hydrochloric acid to give the title compound.

### Example 22

### Preparation of 7-[3-(4-piperidinyl)propyloxy]-2-(2-phenylethyl)-3-oxo-2,3,4,5-tetrahydro-1H-2-benzazepine-4-acetic acid

Using the procedure of Example 15, except substituting the compound of Example 21(1) for the compound of Preparation C gives the title compound.

### Example 23

### Preparation of 8-[1-[4-(4-pyridyl)piperazinyl]carbonyl]-2,3,4,5-tetrahydro-3-oxo-4-(2-phenylethyl)-1H-1,4-benzodiazepine-2-acetic acid

Using the procedure of Example 14, except substituting 1-(4-pyridyl)piperazine for N-methyl-2-(4-pyridyl)ethanamine, gave the title compound: HPLC RT 16.6 min (YMC ODS-AQ, 6 x 250 mm, 1.5 mL/min, gradient, A:acetonitrile B:water-0.1% TFA, 10-80% A in 20 min, UV detection at 220 nm).

### Example 24

### Preparation of 2-[3-(4-piperidinyl)propyloxy]-7-oxo-6,7,8,9-tetrahydro-5H-benzocycloheptene-6-acetic acid; and 3-[3-(4-piperidinyl)propyloxy]-7-oxo-6,7,8,9-tetrahydro-5H-benzocycloheptene-6-acetic acid

a) methyl 2-methoxy-7-oxo-6,7,8,9-tetrahydro-5H-benzocycloheptene-6-acetate, and methyl 3-methoxy-7-oxo-6,7,8,9-tetrahydro-5H-benzocycloheptene-6-acetate
   Using the general method of *J*. *Org. Chem., 57,* 1429 (1992), except substituting 2-methoxy-benzosuberone (*J. Org. Chem.* 47, 5201 (1982)) for benzosuberone, and separating the resulting mixture of isomers by chromatography, gives the title compounds.
b) 2-[3-(4-piperidinyl)propyloxy]-7-oxo-6,7,8,9-tetrahydro-5H-benzocycloheptene-6-acetic acid, and 3-[3-(4-piperidinyl)propyloxy]-7-oxo-6,7,8,9-tetrahydro-5H-benzocycloheptene-6-acetic acid
   Using the procedures of Examples 21 and 22, except substituting each of the compounds of Example 24(a) for the compound of Examples 21(i), gives the title compounds.

### Example 25

### Preparation of (R,S)-8-[[[2-[4-(2-aminopyridyl)]ethyl]-amino]carbonyl]-2,3,4,5-tetrahydro-3-oxo-4-(2-phenylethyl)-1H-1,4-benzodiazepine-2-acetic acid

Using the procedures of Example 12(a) and Example 10(b), except substituting the compound of Preparation 8(j) for histamine and benzotriazol-1-yloxy-tris(dimethylamino)-phosphonium hexafluorophosphate for 1-(3-dimethylamino-propyl)-3-ethylcarbodiimide hydrochloride, gave the title compound. MS(ES) m/e 488.2 [M+H]⁺; Anal. (C₂₇H₂₉N₅O₄·1.5 CF₃CO₂H·H₂O) calcd: C, 53.35; H, 4.56; N, 10.35. found: C, 53.25; H, 4.84; N,10.35.; HPLC k' 3.88 (PRP-1®, 25% AN/W-TFA).

### Example 26

### Preparation of (R,S)-2,3,4,5-tetrahydro-8-[[[2-[2-(hydroxy)pyrid-4-yl]ethyl]amino]carbonyl]-3-oxo-4-(2-phenylethyl)-1H-1,4-benzodiazepine-2-acetic acid

Using the procedure of Example 25, except substituting the compound of Preparation 9(b) for the compound of Preparation 8 (j), gave the title compound. MS(ES) m/e 489.4 [M+H]⁺; Anal. (C₂₇H₂₈N₄O₅·1.125 H₂O) calcd: C, 63.74; H, 5.74; N, 10.71. found: C, 63.74; H, 5.99; N,11.01.; HPLC k' 3.70 (PRP-1®, 25% AN/W-TFA).

### Example 27

### Preparation of (R,S)-8-[[4-(2-aminoethyl)piperidin-1-yl]carbonyl]-2,3,4,5-tetrahydro-3-oxo-4-(2-phenylethyl)-1H-1,4-benzodiazepine-2-acetic acid

a) methyl (R,S)-8-[[4-[(N-t-butoxycarbonyl)-(2-aminoethyl)]piperidin-1-yl]carbonyl]-2,3,4,5-tetrahydro-3-oxo-4-(2-phenylethyl)-1H-1,4-benzodiazepine-2-acetate
   Using the procedure of Preparation 17(c), except substituting the compound of Preparation 10(b) for the compound A(g), gave the title compound (657 mg, 37%). ¹H NMR (90 MHz, CDCl₃) δ 7.40-7.08 (m, 5H), 6.95-6.50 (m, 3H), 5.50-4.40 (m, 5H), 4.00-2.43 (m, 14H), 3.73 (s, 3H), 1.90-1.00 (m, 5H), 1.43 (s, 9H).
b) (R,S)-8-[[4-(2-aminoethyl)piperidin-1-yl]carbonyl]-2,3,4,5-tetrahydro-3-oxo-4-(2-phenylethyl)-1H-1,4-benzodiazepine-2-acetic acid
   Using the procedures of Preparation D(b) and D(c), except substituting the compound of Example 27(a) for the compound D(a), gave the title compound (160 mg). MS(ES) m/e 479 [M+H]⁺; ¹H NMR (CD₃OD, 250 MHz) δ 7.30-7.08 (m, 5H), 6.98 (d, 1H, J=7.6 Hz), 6.60-6.50 (m, 2H), 5.47 (d, 1H, J=17 Hz), 5.15-5.08 (m, 1H), 3.90-3.58 (m, 3H), 3.89 (d, 1H, J=17Hz), 3.18-2.55 (m, 9H), 1.95-1.52 (m, 5H), 1.35-1.08 (m, 2H); HPLC k' 2.43 (PRP-1®, 25% AN/W-TFA, UV detection at 220 nm); Anal. (C₂₇H₃₄N_{4O}4•1.5 TFA) calcd: C,55.46; H, 5.51; N, 8.62, found C, 55.19; H, 5.68; N, 8.58.

### Example 28

### Preparation of (R,S)-2,3,4,5-tetrahydro-8-[[[2-[1-methyl-1,2,5,6-tetrahydropyrid-4-yl]ethyl]methylamino]carbonyl]-3-oxo-4-(2-phenylethyl)-1H-1,4-benzodiazepine-2-acetic acid

a) methyl (R,S)-2,3,4,5-tetrahydro-8-[[[2-[1-methyl-1,2,5,6-tetrahydropyrid-4-yl]ethyl]methylamino]carbonyl]-3-oxo-4-(2-phenylethyl)-1H-1,4-benzodiazepine-2-acetate
   Using the procedure of Example 12(a), except substituting the compound of Preparation 11(c) for histamine, gave the title compound. MS(ES) m/e 519 [M+H]⁺.
b) (R,S)-2,3,4,5-tetrahydro-8-[[[2-[1-methyl-1,2,5,6-tetrahydropyrid-4-yl]ethyl]-methylamino]carbonyl]-3-oxo-4-(2-phenylethyl)-1H-1,4-benzodiazepine-2-acetic acid
   Using the procedure of Preparation A(j), except substituting the compound of Example 28(a) for the compound A(i), gave the title compound. HPLC RT 15.7 min (YMC ODS-AQ, 6x250 mm, 1.5 mL/min, gradient, A:acetonitrile B:water-0.1% TFA, 10-80% A during 20 min, UV detection at 220 nm); ¹H NMR (DMSO-d₆/TFA, 400 MHz) δ 9.6 (br d, 1H), 7.25-7.15 (m, 5H), 7.0 (d, 1H), 6.49 (s, 1H), 6.45 (s, 1H), 5.5 (s, 1H), 5.4 (d, 1H), 5.0 (m, 1H), 4.0 (d, 1H), 3.75-3.05 (br m, 6H), 2.9-2.16 (br m, 16H); MS(ES) m/e 505 [M+H]⁺.

### Example 29

### Preparation of (R,S)-2,3,4,5-tetrahydro-3-oxo-4-(2-phenylethyl)-8-[4-(piperidin-4-yl)-1(E)-butenyl]-1H-1,4-benzodiazepine-2-acetic acid

a) methyl (R,S)-2,3,4,5-tetrahydro-8-hydroxymethyl-3-oxo-4-(2-phenylethyl)-1H-1,4-benzodiazepine-2-acetate
   Methyl (R,S)-8-carboxy-1,2,3,4-tetrahydro-3-oxo-4-(phenylethyl)- 1H-1,4-benzodiazepine-2-acetate (Preparation 16(e)) (3.8 g, 10 mmol) was treated with thionyl chloride (50 mL) and the resulting suspension was heated at reflux under argon with stirring for 15 min, cooled to RT and concentrated. The residual oil was re-dissolved in toluene (25 mL), concentrated and the residue was dissolved in dry THF (50 mL). The solution was stirred at RT and sodium borohydride (2.0 g, 53 mmol) as added in one portion. After stirring for 16 h, the reaction was carefully quenched at 0°C with 1N hydrochloric acid, basified with 1N sodium carbonate, extracted with ethyl acetate (150 mL), washed with brine, dried (magnesium sulfate) and concentrated. Purification by flash chromatography (silica gel, 1% methanol/chloroform) gave the title compound (1.71 g, 47%) as a solid foam. TLC R_{f}=0.49 (silica gel, 5% methanol/chloroform); ¹H NMR (CDCl₃) δ 2.62 (dd, 1H), 2.77 (m, 2H), 2.97 (dd, 1H), 3.27 (br s, 2H), 3.68 (m, 3H), 3.72 (s, 3H), 4.51 (s, 2H), 4.94 (t, 1H), 5.24 (d, J=16.5 Hz, 1H), 6.56 (s, 1H), 6.62 (d, J=7.4, 1H), 6.80 (d, J=7.7 Hz, 1H), 7.20 (m, 5H).
b) methyl (R,S)-8-bromomethyl-2,3,4,5-tetrahydro-3-oxo-4-(2-phenylethyl)-1H-1,4-benzodiazepine-2-acetate
   To a solution of the compound of Example 29(a) (1.20 g, 3.3 mmol) and carbon tetrabromide (1.4 g, 4.2 mmol) in dry THF (40 mL), stirred at 0°C under argon, was added triphenylphosphine (1.1 g, 4.2 mmol) in one portion. After 10 min, the reaction was allowed to warm to RT and stirred for 3 h. The mixture was concentrated and the residue was purified by flash chromatography (silica gel, 50% ethyl acetate/hexane) to yield the title compound (1.17 g, 83%). TLC R_{f} 0.38 (silica gel, 40% ethyl acetate/hexane); ¹H NMR (CDCl₃) δ 1.26 (1H, s), 2.82(3H, m), 3.11 (1H, dd), 3.71 (2H, m), 3.74 (3H, s), 3.83 (1H, d, J=16.5Hz), 4.37 (2H, s), 4.90 (1H, t), 5.13 (1H, d, J=16.5Hz), 6.82 (2H, s), 6.89 (1H, d), 7.1-7.28 (6H, m).
c) (R,S)-[2-methoxycarbonylmethyl-3-oxo-4-(2-phenylethyl)-2,3,4,5-tetrahydro-1H-1,4-benzodiazepin-8-yl]methyltriphenyl-phosphonium bromide
   To a solution of the compound of Example 29 (b) (1.15 g, 2.67 mmol) in dry THF (30 mL) was added triphenylphosphine (0.71 g, 2.7 mmol). The reaction was refluxed for 4 h, cooled to RT, concentrated and triturated with ether, filtered and dried under vacuum to give the title compound (1.89 g, 100%). ¹H NMR (CDCl₃) δ 2.01 (1H, s), 2.73 (2H, m), 2.90 (1H, dd), 3.08 (1H, dd), 3.54 (1H, m), 3.65 (1H, m), 3.70 (3H, s), 3.74 (1H, d, J=16.7Hz), 4.49 (2H, dt), 5.08 (1H, d, J=15.5Hz), 5.17 (1H, t), 6.40 (1H, d), 6.58 (1H, d, J=11.4Hz), 6.87 (1H, s), 7.18 (5H, m), 7.58-7.80 (15H, m).
d) methyl (R,S)-8-[4-[N-(t-butoxycarbonyl)-piperidin-4-yl]-1-(E and Z)butenyl]-2,3,4,5-tetrahydro-3-oxo-4-(2-phenylethyl)- 1H-1,4-benzodiazepine-2-acetate
   To a solution of compound of Example 29 (c) (1 g, 1.44 mmol) and the compound of Preparation 12(1.75 mmol) in dry THF (30 mL) was added, with stirring under argon, a 60% dispersion of sodium hydride in mineral oil (58 mg, 1.44 mmol) in one portion. After stirring for 16 h, the reaction was concentrated. The residue was purified by flash chromatography (silica gel, 5% ethyl acetate:chloroform) to give a mixture of the title compounds (0.717 g, 86%). Pure olefin isomers in the ratio of approximately (1:2) (Z:E) were obtained by HPLC chromatography (silica gel, 40% ethyl acetate/hexane). E-isomer: ¹H NMR (CDCl₃) δ 1.11 (2H, m), 1.43 (4H, m), 1.45 (9H, s), 1.68 (2H, d, J=12.5Hz), 2.20 (2H, dd), 2.67 (2H, dt), 2.72 (1H, dd), 2.81 (1H, dd), 3.72 (3H, m), 3.74 (3H, s), 4.08 (2H, d, J=13.7Hz), 4.90 (1H, t), 5.18 (1H, d, J=16.5Hz), 6.14 (1H, dt, Jₜᵣₐₙₛ=15.8Hz), 6.24 (1H, d, Jₜᵣₐₙₛ=15.8Hz), 6.67 (1H, s), 6.72 (1H, dd, J=7.8Hz), 6.80 (1H, d, J=7.8Hz), 7.20 (5H, m); MS(ES) m/e 576.2 (M+H)⁺.
e) (R,S)-2,3,4,5-tetrahydro-3-oxo-4-(2-phenylethyl)-8-[4-(piperidin-4-yl)-1(E)-butenyl]-1H-1,4-benzodiazepine-2-acetic acid
Using the procedures of Preparation D(b) and D(c), except substituting the compounds of Example 29(d) for the compound D(a) gave the title compound. MS(ES) m/e 462.2 [M+H]⁺; Anal. (C₂₈F₃₅N₃O₃·1.5 CF₃CO₂H) calcd: C, 58.86; H, 5.82; N, 6.64. found: C, 58.68; H, 5.84; N,6.32; HPLC k' 4.07 (PRP-1®, 35% AN/W-TFA); ¹H NMR (CD₃OD) δ 1.38 (2H, m), 1.45 (2H, m), 1.61 (1H, br s), 1.95 (2H, d, J=13.6Hz), 2.22 (2H, dd), 2.61 (1H, m), 2.77 (2H, m), 2.91 (3H, m), 3.35 (2H, m), 3.67 (2H, m), 3.79 (1H, d, J=16.8Hz), 5.02 (1H, dd), 5.37 (1H, d, J=16.5Hz), 6.15 (1H, dt), 6.26 (1H, d, J=15.9Hz), 6.58 (1H, s), 6.60 (1H, d, J=7.8Hz), 6.81 (1H, d, J=7.8Hz), 7.17 (5H, m).

### Example 30

### (R,S)-2,3,4,5-tetrahydro-3-oxo-4-(2-phenylethyl)-8-[4-(piperidin-4-yl)-1(Z)-butenyl]-1H-1,4-benzodiazepine-2-acetic acid

a) methyl (R,S)-8-[4-[N-(t-butoxycarbonyl)-piperidin-4-yl]-1-(Z)butenyl]-2,3,4,5-tetrahydro-3-oxo-4-(2-phenylethyl)-1H-1,4-benzodiazepine-2-acetate
   The title compound was prepared by chromatography of the mixture of Example 29(d). Z-isomer: MS(ES) m/e 462.2 [M+H]⁺; Anal. (C₂₈H₃₅N₃O₃·1.5 CF₃CO₂H·0.5 H₂O) calcd: C, 57.83; H, 5.73; N, 6.27. found: C, 58.03; H, 5.89; N,6.55.; HPLC k' 4.98 (PRP-1®, 35% CH₃CN/water/0.1% TFA); ¹H NMR (CD₃OD) δ 1.30 (2H, m), 1.41 (2H, dd), 1.58 (1H, br s), 1.82 (2H, d, J=13.1Hz), 2.34 (2H, m), 2.60 (1H, m), 2.76 (2H, m), 2.88 (3H, m), 3.29 (3H, m), 3.69 (2H, m), 3.81 (1H, d, J=16.9Hz), 5.04 (1H, m), 5.40 (1H, d, J=16.6Hz), 5.58 (1H, dt), 6.31 (1H, d, J=11.6Hz), 6.48 (1H, d, J=7.9Hz), 6.50 (1H, s), 6.85 (1H, d, J=7.6Hz), 7.18 (5H, m).
b) (R,S)-2,3,4,5-tetrahydro-3-oxo-4-(2-phenylethyl)-8-[4-(piperidin-4-yl)- 1(Z)-butenyl]-1H-1,4-benzodiazepine-2-acetic acid
   The title compound was prepared in the same manner as in Example 29(e), except substituting the compound of Example 30(a). ¹H NMR (CDCl₃) δ 1.08 (2H, m), 1.41 (4H, m), 1.45 (9H, s), 1.62 (2H, d, J=12.9Hz), 2.31 (2H, dd), 2.64 (2H, dt), 2.72 (1H, dd), 2.82 (2H, dt), 3.03 (1H, dd), 3.73 (3H, m), 3.75 (3H, s), 4.06 (2H, d, J=13.2Hz), 4.93 (1H, t), 5.22 (1H, d, J=16.5Hz), 5.59 (1H, dt, J_{cis}=11.6Hz), 6.27 (1H, d, J_{cis}=11.7Hz), 6.55 (1H, s), 6.63 (1H, d, J=7.7Hz), 6.82 (1H, d, J=7.7Hz), 7.20 (5H, m); MS(ES) m/e 576.2 (M+H)⁺.

### Example 31

### Preparation of (R,S)-2,3,4,5-tetrahydro-3-oxo-4-(2-phenylethyl)-7-[[4-(pyrid-4-yl)piperazin-1-yl]carbonyl]-1H-1,4-benzodiazepine-2-acetic acid

a) methyl (R,S)-2,3,4,5-tetrahydro-3-oxo-4-(2-phenylethyl)-7-[[4-(pyrid-4-yl)piperazin-1-yl]carbonyl]-1H-1,4-benzodiazepine-2-acetate
   Using the procedure of Example 14(a), except substituting 1-(pyrid-4-yl)piperazine for N-methyl-2-(4-pyridyl)ethanamine, gave the title compound. HPLC RT 16.3 min (YMC ODS-AQ, 6x250 mm, 1.5 mL/min, gradient, A:acetonitrile B:water-0.1% TFA, 10-80% A during 20 min, UV detection at 220 nm); MS(ES) m/e 528 [M+H]⁺.
b) (R,S)-2,3,4,5-tetrahydro-3-oxo-4-(2-phenylethyl)-7-[[4-(pyrid-4-yl)piperazin-1-yl]carbonyl]-1H-1,4-benzodiazepine-2-acetic acid
   Using the procedure of Preparation A(j), except substituting the compound of Example 31(a) for the compound A(i), gave the title compound. HPLC RT 14.6 min (YMC ODS-AQ, 6x250 mm, 1.5 mL/min, gradient, A:acetonitrile B:water-0.1% TFA, 10-80% A during 20 min, UV detection at 220 nm); ¹H NMR (DMSO-d₆:TFA, 250 MHz) δ 8.3 (m, 2H), 7.19 (m, 9H), 6.55 (d, 1H), 5.4 (d, 1H), 5.09 (m, 1H), 4.0 (d, 1H), 3.7 (m, 8H), 2.8-2.4 (br m, 6H); MS(ES) m/e 514 [M+H]⁺.

### Example 32

### Preparation of (R,S)-2,3,4,5-tetrahydro-3-oxo-7-[[[2-(piperidin-4-yl)ethyl]methylamino]carbonyl]-1H-2-benzazepine-4-acetic acid

a) t-butyl 3-bromo-4-methylbenzoate
   A mixture of 3-bromo-4-methylbenzoic acid (10.75 g, 50 mmol) in dry ether (50 mL) in a glass bomb was cooled to -30°C and isobutylene gas was bubbled in to give a total reaction volume of approximately 150 mL. Trifluoromethanesulfonic acid (0.22 g, 2.5 mmol) was added dropwise to the stirred mixture, and the bomb was sealed. The reaction was allowed to warm to RT and was stirred for 6 d. The bomb was opened carefully, and 5% sodium bicarbonate (50 mL) was added slowly. The mixture was allowed to stir for 15 min and ether (250 mL) was added. The layers were separated and the organic layer was washed with 5% sodium bicarbonate (2x50 mL) and then with brine (50 mL). Drying (magnesium sulfate) and concentration gave the title compound (12.88 g, 95%) as a yellow oil. TLC R_{f} 0.68 (toluene); ¹H NMR (250 MHz, CDCl₃) δ 8.13 (d, J=1.7 Hz, 1 H), 7.81 (dd, J=7.9, 1.7 Hz, 1 H), 7.27 (d, J=7.9 Hz, 1 H), 2.44 (s, 3 H), 1.59 (s, 9 H); IR (CCl₄) 1715, 1368, 1297, 1255, 1170, 1123, 1115 cm⁻¹; MS(ES) m/e 273.0 [M+H]⁺, 271.0 [M+H]⁺, 214.8 [M+H-C₄H₈]⁺.
b) t-butyl 3-bromo-4-(bromomethyl)benzoate
   A mixture of the compound of Example 32(a)(5.25 g, 19.36 mmol), N-bromosuccinimide (3.79 g, 21.3 mmol), and benzoyl peroxide (0.23 g, 0.97 mmol) in carbon tetrachloride (100 mL) was heated to reflux. After 15 h, the mixture was cooled, filtered, and the filtrate was concentrated. The resulting material was used without purification. TLC R_{f} 0.57 (10% ethyl acetate/hexane).
c) t-butyl 3-bromo-4-[[N-t-butoxycarbonyl-N-methoxycarbonyl]amino]methylbenzoate
   The compound of Example 32(b) and methyl t-butyl iminodicarboxylate potassium salt (prepared according to *J. Chem. Soc. Perkin Trans. I*, 1088, (1988)) (3.10 g, 14.52 mmol) were combined in dry dimethylformamide (20 mL), and the mixture was warmed in an oil bath at 60°C. More methyl t-butyl iminodicarboxylate potassium salt (0.83 g, 3.89 mmol) was added after 20 min and the mixture which was stirred an additional 5 min and concentrated. The residue was partitioned between water (100 mL) and ether (100 mL), and the layers were separated. The aqueous layer was extracted with ether, and the combined organic layers were dried (magnesium sulfate) and concentrated to a brown oil. Chromatography (silica gel, 20% ethyl acetate/hexane) gave the title compound (5.21 g, 61%) as a yellow oil which solidified in vacuum. TLC Rf 0.49 (20% ethyl acetate/hexane); ¹H NMR (250 MHz, CDCl₃) δ 8.15 (d, J=1.6 Hz, 1 H), 7.89 (dd, J=8.1, 1.6 Hz, 1 H), 7.16 (d, J=8.1 Hz, 1 H), 4.95 (s, 2 H), 3.84 (s, 3 H), 1.59 (s, 9 H), 1.41 (s, 9 H); IR (carbon tetrachloride) 1756, 1719, 1369, 1352, 1297, 1255, 1222, 1155, 1113 cm⁻¹; MS(ES) m/e 446 [M+H]⁺, 444 [M+H]⁺, 390 [M+H-C₄H₈]⁺, 388 [M+H-C₄H₈]⁺, 334 [M+H-2xC₄H₈]⁺, 332 [M+H-2xC₄H₈]⁺.
d) t-butyl 3-bromo-4-[(t-butoxycarbonyl)amino] methylbenzoate
   A solution of the compound of Example 32(c) (5.21 g, 11.73 mmol) and 1N sodium hydroxide (11.7 mL, 11.7 mmol) in methanol (105 mL) was stirred at RT. After 20 min, the reaction was diluted with water (250 mL) and extracted with ether. The combined organic layers were washed with brine (100 mL), dried (magnesium sulfate), and concentrated. Chromatography (silica gel, 15% ethyl acetate/hexane) gave the title compound (4.29 g, 95%) as a light yellow oil which slowly, partially solidified. TLC R_{f} 0.41 (15% ethyl acetate/hexane); ¹H NMR (400 MHz, CDCl₃) δ 8.13 (d, J=1.5 Hz, 1 H), 7.90 (dd, J=8.0, 1.5 Hz, 1 H), 7.42 (d, J=8.0 Hz, 1 H), 5.07 (m, 1 H), 4.41 (d, J=6.3 Hz, 2 H), 1.59 (s, 9 H), 1.45 (s, 9 H); IR (carbon tetrachloride) 3450, 1719, 1496, 1394, 1371, 1300, 1255, 1172, 1120 cm⁻¹; MS(ES) m/e 410.0 [M+Na]⁺, 408.2 [M+ Na]⁺, 386.2 [M+H]⁺, 388 [M+H]⁺, 330.0 [M+H-C₄H₈]⁺, 332 [M+H-C₄H₈]⁺, 274 [M+H-2xC₄H₈]⁺, 276 [M+H-2xC₄H₈]⁺.
e) methyl 3-methoxycarbonyl-4-[5-(t-butoxycarbonyl)-2-[[(t-butoxycarbonyl)amino]methyl]phenyl]-3-butenoate
   Dimethyl itaconate (607 mg, 3.84 mmol) was added to a solution of the compound of Example 32(d) (1.14 g, 2.95 mmol), palladium(II) acetate (33 mg, 0.15 mmol), tri-ortho-tolylphosphine (90 mg, 0.30 mmol), and diisopropylethylamine (1.03 mL, 5.90 mmol) in propionitrile (15 mL). The solution was deoxygenated through three evacuation/argon purge cycles, and was heated to reflux under argon. After 55 min, the reaction was cooled in ice/water and poured into cold ether (150 mL). After standing for 10 min, the mixture was filtered, and the filter pad was washed with ether. The filtrate was concentrated to a yellow oil, which was concentrated from toluene to remove residual propionitrile. The residue was chromatographed (silica gel, step gradient, 15-30% ethyl acetate/hexane). All products with Rf 0.26 (major product) to R_{f} 0.65 (25% ethyl acetate/hexane) were isolated together as a cloudy, yellow oil. TLC Rf 0.26 (major product), 0.37, 0.49, 0.56, 0.65 (minor products) (25% ethyl acetate/hexane).
f) methyl (R,S)-3-methoxycarbonyl-4-[5-(t-butoxycarbonyl)-2-[[(t-butoxycarbonyl)amino]methyl]phenyl]butanoate
   10% Palladium on carbon (0.94 g) was added carefully to a well-stirred solution of the compound of Example 32(e) (1.36 g, 2.93 mmol) in ethyl acetate-methanol (1:1) (29 mL) at 0°C. The stirring bar was removed, and the mixture was shaken at RT under H₂ (3.45 bar). After 2 h, the mixture was filtered through Celite®, and the filtrate was concentrated to a pale yellow residue. The residue was dissolved in methanol (29 mL), and palladium(II) hydroxide on carbon (moist, Pearlman's catalyst, 0.59 g) was added. The mixture was shaken at RT under H₂ (3.45 bar) for 3 h, then was filtered through Celite®. The filtrate was concentrated to a cloudy, colorless oil, which was reconcentrated from toluene to remove residual methanol. Chromatography (silica gel, 30% ethyl acetate/hexane) gave the title compound (1.03 g, 76%) as a colorless oil. TLC Rf 0.35 (25% ethyl acetate/hexane); ¹H NMR (250 MHz, CDCl₃) δ 7.83 (dd, J=8.0, 1.6 Hz, 1 H), 7.73 (d, J=1.6 Hz, 1 H), 7.36 (d, J=8.0 Hz, 1 H), 4.90-5.05 (m, 1 H), 4.25-4.50 (m, 2 H), 3.66 (s, 3 H), 3.64 (s, 3 H), 3.03-3.20 (m, 2 H), 2.67-2.93 (m, 2 H), 2.48 (dd, J=16.8, 5.1 Hz, 1 H), 1.59 (s, 9 H), 1.45 (s, 9 H); IR (CCl₄) 3300-3480 (br), 1740, 1715, 1368, 1157 cm⁻¹; MS(ES) m/e 953.4 [2M + Na]⁺, 931.4 [2M+H]⁺, 488.0 [M + Na]⁺, 466.0 [M+H]⁺, 410.0 [M+H-C₄H₈]⁺, 354 [M+H-2xC₄H₈]⁺, 310 [M+H-2xC₄H₈-CO₂]⁺.
g) methyl (R,S)-3-methoxycarbonyl-4-[5-carboxy-2-(aminomethyl)phenyl]butanoate
   TFA (3.2 mL) was added all at once to a cloudy solution of the compound of Example 32(f) (296.5 mg, 0.64 mmol) in dry dichloromethane (3.2 mL) at 0°C under argon. The solution was warmed to RT, stirred for 2 h and concentrated. The residue was concentrated once from 2:1 1,2-dichloroethane:toluene (10 mL) to remove residual TFA. The resulting material was used without purification.
h) methyl (R,S)-7-carboxy-2,3,4,5-tetrahydro-3-oxo-1H-2-benzazepine-4-acetate
   The compound of Example 32(g) was dissolved in anhydrous methanol (3.2 mL) and the solution was cooled to 0°C under argon. Freshly prepared 1.0 M sodium methoxide/methanol (3.2 mL, 3.2 mmol) was added rapidly, and the ice bath was removed. The reaction was allowed to warm to RT over 10 min and heated to reflux under argon. After 2 h, the reaction was cooled in ice, and cold 1N hydrochloric acid (3.4 mL, 3.4 mmol) was added rapidly. The mixture was filtered after 1 h at 0°C, and the filter pad was washed with water and then with cold methanol, to afford the title compound (149.2 mg, 84%) as a colorless powder. TLC R_{f} 0.38 (10% methanol/chloroform); ¹H NMR (400 MHz, DMSO-d₆) δ 8.08 (app t, 1 H), 7.65-7.72 (m, 2 H), 7.27 (d, J=7.8 Hz, 1 H), 4.79 (dd, J=16.4, 4.9 Hz, 1 H), 4.01 (dd, J=16.4, 6.7 Hz, 1 H), 3.59 (s, 3 H), 3.50-3.61 (m, 1 H), 3.07 (dd, J=17.2, 3.6 Hz, 1 H), 2.87 (dd, J=17.2, 13.0 Hz, 1 H), 2.70 (dd, J=16.7, 8.7 Hz, 1 H), 2.45 (dd, J=16.7, 5.2 Hz, 1 H); MS(ES) m/e 278.0 [M+H]⁺.
i) methyl (R,S)-2,3,4,5-tetrahydro-3-oxo-7-[[[2-(pyrid-4-yl)ethyl]methylamino]carbonyl]-1 H-2-benzazepine-4-acetate
   1-(3-dimethylaminopropyl)-3-ethylcarbodiimide hydrochloride (90 mg, 0.47 mmol) was added to a solution of the compound of Example 32(h) (107.6 mg, 0.39 mmol), N-methyl-2-(pyrid-4-yl)ethanamine (64 mg, 0.47 mmol), 1-hydroxybenzotriazole (63 mg, 0.47 mmol), and diisopropylethylamine (0.14 mL, 0.78 mmol) in dry dimethylformamide (2 mL) at RT under argon. The reaction was stirred for 23 h and concentrated. The residue was partitioned between ethyl acetate and water. The layers were separated, and the organic phase was washed with water. The combined aqueous layers were back-extracted with ethyl acetate. The combined organic layers were dried (sodium sulfate) and concentrated to leave a yellow oil. The combined aqueous layers were concentrated and sodium chloride was added. The resulting mixture was exhaustively extracted with chloroform. The combined organic phase was dried (sodium sulfate) and concentrated to give a yellow oil, which was combined with the material from the ethyl acetate phase. Chromatography (silica gel, 10% methanol/chloroform) gave the title compound (139.7 mg, 91%) as a colorless oil. TLC R_{f} 0.45 (10% methanol/chloroform); ¹H NMR (400 MHz, CDCl₃) Rotameric mixture; δ 8.41-8.63 (m, 2 H), 6.72-7.28 (m, 5 H), 6.53 (t, J=6.0 Hz, 1 H), 4.83 (dd, J=16.3, 4.8 Hz, 1 H), 4.09 (dd, J=16.3, 6.5 Hz, 1 H), 3.45-3.88 (m, 3 H), 3.72 (s, 3 H), 2.70-3.25 (m, 8 H), 2.50 (dd, J=16.9, 6.1 Hz, 1 H); IR (chloroform) 3400, 1727, 1660, 1623 cm⁻¹; MS(ES) m/e 396.2 [M+H]⁺.
j) methyl (R,S)-2,3,4,5-tetrahydro-3-oxo-7-[[[2-(piperidin-4-yl)ethyl]methylamino]carbonyl]-1H-2-benzazepine-4-acetate
   Platinum oxide (4 mg) was added to a solution of the compound of Example 32(i) (139.7 mg, 0.35 mmol) and 1N hydrochloric acid (0.35 mL, 0.35 mmol) in methanol (3.5 mL), and the mixture was stirred briskly under H₂ (balloon pressure). After 7 h, the reaction was filtered through Celite® and concentrated, and the residue was dissolved in methanol (3.5 mL). Platinum oxide (4 mg) was added, and the mixture was stirred briskly under H2 (balloon pressure) for 16.5 h. Filtration through Celite® and concentration left a colorless oil. This was used without purification. HPLC k' 3.2 (PRP-1®, 15% AN/W-TFA).
k) (R,S)-2,3,4,5-tetrahydro-3-oxo-7-[[[2-(piperidin-4-yl)ethyl]methylamino]carbonyl]-1H-2-benzazepine-4-acetic acid
   The compound of Example 32(j) was dissolved in methanol (12 mL) and cooled to 0°C. 1N Sodium hydroxide (1.05 mL, 1.05 mmol) was added dropwise, and the solution was allowed to warm to RT. The reaction was stirred at RT for 24 h and concentrated. The residue was concentrated from 1:1 acetonitrile:water (4 mL) to remove methanol, the residue dissolved in 1:1 acetonitrile:water (4 mL), and cooled in ice. TFA (0.27 mL, 3.5 mmol) was added and the reaction was concentrated. The residue was purified by reversed-phase flash chromatography (ODS, 10-12.5% AN/W-TFA). Concentration and lyophilization gave the title compound (97.5 mg, 47%) as a colorless powder. HPLC k'5.19 (PRP-1®, 10% AN/W-TFA); ¹H NMR (400 MHz, DMSO-d₆) Rotameric mixture; δ 8.52-8.72 (m, 1 H), 8.15-8.43 (m, 1 H), 8.07 (t, J=5.8 Hz, 1 H), 7.20 (d, J=8.3 Hz, 1 H), 7.08-7.15 (m, 2 H), 4.77 (dd, J=16.3, 4.7 Hz, 1 H), 3.96 (dd, J=16.3, 6.7 Hz, 1 H), 2.70-3.55 (m, 12 H), 2.65 (dd, J=16.8, 8.4 Hz, 1 H), 2.34 (dd, J=16.8, 5.2 Hz, 1 H), 1.82-1.95 (m, 1 H), 1.02-1.62 (m, 6 H); MS(ES) m/e 388.2 [M+H]⁺; Anal. (C₂₁H₂₉N₃O₄ · 1.75 (CF₃CO₂H)) calcd: C, 50.13; H, 5.28; N, 7.16. found: C, 50.20; H, 5.21; N, 7.21

### Example 33

### Preparation of (R,S)-2,3,4,5-tetrahydro-3-oxo-2-(2-phenylethyl)-7-[[[2-(piperidin-4-yl)ethyl]methylamino]-carbonyl]-1H-2-benzazepine-4-acetic acid;

a) methyl (R,S)-2,3,4,5-tetrahydro-3-oxo-2-(2-phenylethyl)-7-[[[2-(pyrid-4-yl)ethyl]methylamino]carbonyl]-1H-2-benzazepine-4-acetate
   Using the procedure of Example 14(a), except substituting the compound A(g) for methyl (R,S)-8-carboxy-1,2,4,5-tetrahydro-3-oxo-4-(2-phenylethyl)-3H-1,4-benzodiazepine-2-acetate, gave the title compound. HPLC RT 16.2 min (YMC ODS-AQ, 6x250 mm, 1.5 mL/min, gradient, A:acetonitrile B:water-0.1% TFA, 10-80% A during 20 min, UV detection at 220 nm).
b) methyl (R,S)-2,3,4,5-tetrahydro-3-oxo-2-(2-phenylethyl)-7-[[[2-(piperidin-4-yl)ethyl]methylamino]carbonyl]-1H-2-benzazepine-4-acetate; and methyl (R,S)-2,3,4,5-tetrahydro-3-oxo-2-(cyclohexylethyl)-7-[[[2-(piperidin-4-yl)ethyl]methylamino]carbonyl]-1H-2-benzazepine-4-acetate
   Using the procedure of Example 17(a), except substituting the compound of Example 33(a) for the compound of Example 14(a), gave a mixture of the title compounds.
   2-(2-phenylethyl) compound: MS(ES) m/e 506 [M+H]⁺; HPLC RT 16.47 min (YMC ODS-AQ, 6x250 mm, 1.5 mL/min, gradient, A:acetonitrile B:water-0.1% TFA, 10-80% A during 20 min, UV detection at 220 nm).
   2-(2-cyclohexylethyl) compound: MS(ES) m/e 511 [M+H]⁺; HPLC RT 19.14 min (YMC ODS-AQ, 6x250 mm, 1.5 mL/min, gradient, A:acetonitrile B:water-0.1% TFA, 10-80% A during 20 min, UV detection at 220 nm).
c) (R,S)-2,3,4,5-tetrahydro-3-oxo-2-(2-phenylethyl)-7-[[[2-(piperidin-4-yl)ethyl]methylamino]carbonyl]-1H-2-benzazepine-4-acetic acid; and
   Using the procedure of Preparation A(j), except substituting the compounds of Example 33(b) for the compound A(i), yielded the title compound which was separated from the 2-ethylcyclhexyl compound by HPLC. HPLC RT 14.59 min (YMC ODS-AQ, 6x250 mm, 1.5 mL/min, gradient, A:acetonitrile B:water-0.1% TFA, 10-80% A during 20 min, UV detection at 220 nm); MS(ES) m/e 492 [M+H]⁺.

### Example 34

### (R,S)-2,3,4,5-tetrahydro-3-oxo-2-(2-cyclohexylethyl)-7-[[[2-(piperidin-4-yl)ethyl]methylamino]carbonyl]-1H-2-benzazepine-4-acetic acid

The title compound was isolated from the reaction mixture of Example 33(c) by HPLC. HPLC RT 17.61 min (YMC ODS-AQ, 6x250 mm, 1.5 mL/min, gradient, A:acetonitrile B:water-0.1% TFA, 10-80% A during 20 min, UV detection at 220 nm); MS(ES) m/e 498 [M+H]⁺.

### Example 35

### Preparation of (R,S)-2,3,4,5-tetrahydro-3-oxo-4-(2-phenylethyl)-8-[4-(piperidin-4-yl)butyl]-1H-1,4-benzodiazepine-2-acetic acid

a) methyl (R,S)-8-[4-[(N-t-butoxycarbonyl)-piperidin-4-yl]butyl]-2,3,4,5-tetrahydro-3-oxo-4-(2-phenylethyl)- 1H-1,4-benzodiazepine-2-acetate
   A mixture of the E/Z isomers of Example 29(d) and 10% palladium on carbon in acetic acid was shaken under hydrogen ( 3.45 bar) for 4 h to give the title compound.
b) (R,S)-2,3,4,5-tetrahydro-3-oxo-4-(2-phenylethyl)-8-(piperidin-4-yl)butyl]-1H-1,4-benzodiazepine-2-acetic acid
   Using the procedure of Preparation D(b) and D(c), except substituting the compound of Example 35(a) for the compound D(a), gave the title compound. MS(ES) m/e 464.2 [M+H]⁺; Anal. (C₂₈H₃₇N₃O₃·CF₃CO₂H·1.25 H₂O) calcd: C, 59.91; H, 6.51; N, 6.73. found: C, 60.04; H, 6.80; N,7.00.; HPLC k' 4.73 (PRP-1®, 35% AN/W-TFA); ¹H NMR (CD₃OD) δ 1.32 (6H, m), 1.57 (3H, m), 1.87 (2H, d, J=14.0Hz), 2.49 (2H, t), 2.61 (1H, dd), 2.75 (2H, m), 2.90 (3H, m), 3.30 (2H, m), 3.68 (2H, m), 3.80 (1H, d, J=16.7Hz), 5.02 (1H, dd), 5.37 (1H, d, J=16.5Hz), 6.41 (1H, d, J=8.0Hz), 6.42 (1H, s), 6.80 (1H, d, J=8.0Hz), 7.17 (5H, m).

### Example 36

### Preparation of (R,S)-8-[[[2-[(2-amino)pyrid-4-yl]ethyl]methylamino]carbonyl]-2,3,4,5-tetrahydro-3-oxo-4-(2-phenylethyl)-1H-1,4-benzodiazepine-2-acetic acid

Using the procedure of Example 25, except substituting the compound of Preparation 13(b) for the compound of Preparation 8(j), and purifying the product by flash chromatography (YMC ODS-AQ S-50, 20-25% AN/W-TFA) gave the title compound. MS(ES) m/e 502.2 [M+H]⁺; HPLC k' 4.92 (PRP-1®, 25% AN/W-TFA); ¹H NMR (400 MHz, CD₃OD) δ 2.65 (1H, m), 2.75 (2H, m), 2.84 (1H, t), 2.92 (1H, m), 2.99 (3H, s), 3.10 (1H, s), 3.63 (2H, m), 3.72 (1H, m), 3.79 (1H, t), 3.87 (1H, d, J=16.8Hz), 5.11 (1H, 2t), 5.43 (1H, d, J=15.6Hz), 6.35, 6.53 (1H, 2s), 6.40, 6.49 (1H, 2d, J=7.5Hz), 6.46, 6.88 (1H, 2d), 6.61, 6.89 (1H, 2s), 6.94, 6.97 (1H, 2d), 7.17 (5H, m), 7.57, 7.74 (1H, 2d, J=6.5Hz).

### Example 37

### Preparation of (R,S)-7-[[[2-[(2-amino)pyrid-4-yl]ethyl]methylamino]carbonyl]-2,3,4,5-tetrahydro-3-oxo-2-(2-phenylethyl)-1H-2-benzazepine-4-acetic acid

Using the procedure of Example 36, except substituting the compound A(g) for methyl (R,S)-8-carboxy-2,3,4,5-tetrahydro-3-oxo-4-(2-phenylethyl)-1H-1,4-benzodiazepine-2-acetate, gave the title compound: MS(ES) m/e 501.2 [M+H]⁺; Anal. (C₂₉H₃₂N₄O₄·1.25 CF₃CO₂H·0.5 H₂O) calcd: C, 57.97; H, 5.22; N, 8.60. found: C, 58.01; H, 5.29; N,8.59.; HPLC k' 3.42 (PRP-1®, 25% AN/W-TFA); ¹H NMR (400 MHz, CD₃OD) δ 2.46 (1H. m), 2.70 (3H, m), 2.83 (2H, m), 2.98 (3H, s), 3.02 (1H, m), 3.13 (1H, s), 3.57 (1H, m), 3.64 (1H, m), 3.81 (3H, m), 4.03 (1H, d, J=16.7Hz), 5.21 (1H, d, J=16.7Hz), 6.47-7.01 (3H, m), 7.01-7.22 (7H, m), 7.57, 7.76 (1H, 2d).

### Preparation F

a) ethyl 7-[[(trifluoromethyl)sulfonyl]oxy]-2-(2-phenyl-ethyl)-3-oxo-2,3,4,5-tetrahydro-1 H-2-benzazepine-4-acetate
   A solution of the compound of Example 21(j) (360 mg, 1 mmol) and N,N-diisopropylethylamine (1 mL) in dichloromethane (8 mL) was stirred in an ice bath and treated with triflic anhydride (0.25 mL, 1.2 mmol). The mixture was stirred for 30 min, and allowed to warm to RT and stirred for 4 h. The mixture was diluted with dichloromethane, washed sequentially with 3N hydrochloric acid, 5% sodium bicarbonate and brine, dried (magnesium sulfate) and concentrated. The residue was chromatographed (silica gel, 1% methanol/dichloromethane) to give the title compound (0.35 g, 70%) as an amber oil. MS (ES) m/e 500 [M+H]⁺, ¹H NMR (400 MHz, CDCl₃) δ 7.2-7.1 (m, 3H), 7.0-7.1 (m, 2H), 6.96 (s, 3H), 5.16-5.0 (d, J=17Hz, 1H), 4.15 (q, 2H), 3.9 (m, 1H), 3.78-3.70 (d, J=17Hz, 1H), 3.6 (m, 1H), 3.0-2.9 (m, 2H), 2.8-2.7 (m, 3H), 2.4-2.3 (q, 1H), 1.3 (t, 3H).

### Example 38

### Preparation of 7-[1-[4-(piperazin-1-yl)but-1-ynyl]]-2-(2-phenylethyl)-3-oxo-2,3,4,5-tetrahydro-1H-2-benzazepine-4-acetic acid

a) ethyl 7-[1-[4-[N-(t-butoxycarbonyl)piperazin-1-yl]-but-1-ynyl]]-2-(2-phenylethyl)-3-oxo-2,3,4,5-tetrahydro-1H-2-benzazepine-4-acetate
   A mixture of the compound F (0.3 g, 0.7 mmol), the compound of Preparation 14 (190 mg, 0.8 mmol), triethylamine (3 mL), bis(triphenylphosphine)-palladium chloride (10 mg, 0.14 mmol) and cuprous iodide (2.5 mg, 0.14 mmol) in acetonitrile (10 mL) was heated to reflux for 20 h. The mixture was diluted with water and extracted with ethyl acetate. The organic phase was dried (magnesium sulfate), concentrated and the residue was purified by flash chromatography (silica gel, 2% methanol/dichloromethane) to yield the title compound (90 mg, 25%). ¹H NMR (400 MHz, CDCl₃) δ 7.15-7.26 (m, 5H), 7.10 (s, 1H), 7.06 (d, J=8Hz, 1H), 6.8 (d, J=8Hz, 1H), 5.1 (d, J=17Hz, 1H), 4.15 (q, 2H), 3.8 (m, 2H), 3.7 (d, J=17Hz, 1H), 3.6 (m, 1H), 3.5 (s, 4H), 3.0 (m, 2H), 2.8 (m, 1H), 2.7 (t, 2H), 2.6 (m, 2H), 2.5 (s, 4H), 2.4 (q, 1H), 1.4 (s, 9H), 1.3 (t, 3H).
b) ethyl 7-[1-[4-(piperazin-1-yl)but-1-ynyl]]-2-(2-phenylethyl)-3-oxo-2,3,4,5-tetrahydro-1 H-2-benzazepine-4-acetate
   The compound of Example 38(a) is stirred with TFA in dichloromethane. The mixture is washed with water, dried and concentrated to give the title compound.
c) 7-[1-[4-(piperazin-1-yl)but- 1-ynyl]]-2-(2-phenylethyl)-3-oxo-2,3,4,5-tetrahydro-1H-2-benzazepine-4-acetic acid
   The compound of Example 38(b) is stirred with dilute aqueous sodium hydroxide in methanol and then treated with dilute hydrochloric acid to give the title compound.

### Example 39

### Preparation of (R,S)-7-[[[2-(piperidin-4-yl)ethyl] methylamino]carbonyl]-2,3,4,5-tetrahydro-2-methyl-3-oxo-1H-2-benzazepine-4-acetic acid

a) methyl (R,S)-7-benzyloxycarbonyl-2,3,4,5-tetrahydro-3-oxo- 1H-2-benzazepine-4-acetate
   1-(3-Dimethylaminopropyl)-3-ethylcarbodiimide hydrochloride (1.19 g, 6.23 mmol) was added all at once to a solution of the compound of Example 32(h) (1.44 g, 5.19 mmol), benzyl alcohol (2.7 mL, 25.95 mmol), diisopropylethylamine (1.8 mL, 10.38 mmol), and 4-(dimethylamino)pyridine (761 mg, 6.23 mmol) in anhydrous dimethylformamide (26 mL) at RT. The reaction was stirred for 24 h and concentrated to leave a pale yellow oil. The oil was diluted with ethyl acetate (200 mL), and the cloudy mixture was washed sequentially with 1N hydrochloric acid (2 x 20 mL) and water (20 mL). The combined aqueous layers were extracted with ethyl acetate (50 mL), and the combined organic layers were dried (magnesium sulfate) and concentrated. Chromatography (silica gel, 5% methanol in 1:1 ethyl acetate:chloroform) gave the title compound (1.59 g, 83%) as a colorless, very viscous oil which could be induced to solidify on scratching and treatment with a little ethyl acetate. TLC R_{f} 0.52 (5% methanol in 1:1 ethyl acetate:chloroform); ¹H NMR (250, CDCl₃) δ 7.72-7.92 (m, 2H), 7.28-7.55 (m, 5H), 7.14 (d, J=8.3 Hz, 1H), 6.76 (br t, 1H), 5.35 (s, 2H), 4.85 (dd, J=16.5, 5.2 Hz, 1H), 4.12 (dd, J=16.5, 6.7 Hz, 1H), 3.71 (s, 3H), 3.50-3.70 (m, 1H), 3.00-3.17 (m, 2H), 2.96 (dd, J=16.9, 7.4 Hz, 1H), 2.50 (dd, J=16.9, 6.1 Hz, 1H); IR (CHCl₃) 3410, 1718, 1677, 1274 cm⁻¹; MS(ES) m/e 390.2 [M+Na]⁺, 268.2 [M+H]⁺.
b) methyl (R,S)-7-benzyloxycarbonyl-2,3,4,5-tetrahydro-2-methyl-3-oxo-1H-2-benzazepine-4-acetate
   The compound of Example 39(a) (220.4 mg, 0.60 mmol) was suspended in toluene (5-10 mL), and the mixture was carefully concentrated to remove water and residual solvents. The resulting solid was dissolved in dry 1:1 THF:DMF (12 mL), and iodomethane (0.19 mL, 3.0 mmol) was added. Sodium hydride (60% in mineral oil, 29 mg, 0.72 mmol) was added, causing gas evolution and slight warming. After 15 min, the reaction was cooled to 0°C and quenched with saturated aqueous ammonium chloride (2 mL). The mixture was diluted with ether (50 mL) and washed with water. The combined aqueous layers were extracted with ether, and the combined organic layers were dried (magnesium sulfate) and concentrated. Chromatography (silica gel, 1:1 ethyl acetate:toluene) gave the title compound (229.2 mg, 100%) as a colorless oil. TLC Rf 0.66 (silica gel, ethyl acetate); ¹H NMR (400, CDCl₃) δ 7.77-7.87 (m, 2H), 7.31-7.47 (m, 5H), 7.16 (d, J=8.4 Hz, 1H), 5.27-5.38 (m, 3H), 3.90 (d, J=16.7 Hz, 1H), 3.77-3.90 (m, 1H), 3.71 (s, 3H), 3.11 (dd, J=17.4, 4.2 Hz, 1H), 3.04 (s, 3H), 2.91-3.07 (m, 2H), 2.43 (dd, J=16.7, 5.4 Hz, 1H); IR (CCl₄) 1724, 1666, 1273 cm⁻¹; MS(ES) m/e 382.0 [M + H]⁺.
c) methyl (R,S)-7-carboxy-2,3,4,5-tetrahydro-2-methyl-3-oxo-1H-2-benzazepine-4-acetate
   10% Palladium-on-carbon (64 mg, 0.06 mmol) was added carefully to a solution of the compound of Example 39(b) (229.2 mg, 0.60 mmol) in methanol (12 mL). The mixture was purged with hydrogen and stirred briskly at RT under hydrogen (balloon pressure). After 15 h, the mixture was filtered through Celite®, and the filtrate was concentrated to afford the title compound (162.7 mg, 93%) as a colorless solid which was used without further purification: ¹H NMR (250, CD₃OD) δ 7.65-7.85 (m, 2H), 7.28 (d, J=7.7 Hz, 1H), 5.38 (d, J=16.6 Hz, 2H), 4.11 (d, J=16.6 Hz, 1H), 3.85-4.01 (m, 1H), 3.68 (s, 3H), 3.17 (dd, J=17.1, 4.3 Hz, 1H), 3.01 (s, 3H), 2.75-2.95 (m, 2H), 2.51 (dd, J=16.9, 4.7 Hz, 1H); MS(ES) m/e 605.2 [2M + Na]⁺, 314.0 [M + Na]⁺, 292.0 [M + H]⁺.
d) Methyl (R,S)-7-[[[2-(pyrid-4-yl)ethyl]methylamino] carbonyl]-2,3,4,5-tetrahydro-2-methyl-3-oxo-1H-2-benzazepine-4-acetate
   Using the procedure of Example 32(i), except substituting the compound of Example 39(c) for the compound of Example 32(h), gave the title compound (221.3 mg, 97%) as a faintly yellow, very viscous oil: TLC R_{f} 0.37 (10% methanol in 1:1 ethyl acetate:chloroform); ¹H NMR (400 MHz, CDCl₃) rotameric mixture; δ 8.42-8.62 (m, 2H), 6.70-7.40 (m, 5H), 5.30 (d, J=16.7 Hz, 1H), 3.46-3.92 (m, 4H), 3.72 (s, 3H), 2.73-3.22 (m, 8H), 3.04 (s, 3H), 2.43 (dd, J=16.7, 5.5 Hz, 1H); IR (CHCl₃) 1730, 1643 cm⁻¹; MS(ES) m/e 410.0 [M + H]⁺.
e) Methyl (R,S)-7-[[[2-(piperidin-4-yl)ethyl]methylamino] carbonyl]-2,3,4,5-tetrahydro-2-methyl-3-oxo-1H-2-benzazepine-4-acetate
   Using the procedure of Example 32(j), except substituting the compound of Example 39(d) for the compound of Example 32(i) gave the title compound: NMR (250 MHz, CD₃OD) δ 7.05-7.45 (m, 3H), 5.36 (d, J=16.6 Hz, 1H), 4.10 (d, J=16.6 Hz, 1H), 3.84-4.03 (m, 1H), 3.67 (s, 3H), 2.65-3.77 (m, 12H), 3.00 (s, 3H), 2.42-2.60 (m, 1H), 1.89-2.18 (m, 1H), 1.15-1.80 (m, 6H).
f) (R,S)-7-[[[2-(Piperidin-4-yl)ethyl]methylamino]carbonyl]-2,3,4,5-tetrahydro-2-methyl-3-oxo-lH-2-benzazepine-4-acetic acid
   Using the procedure of Example 32(k), except substituting the compound of Example 39(e) for the compound of Example 32(j) gave the title compound. HPLC k'=2.11 (PRP-1®; 15% AN/W-TFA);
   ¹H NMR (400 MHz, DMSO-d₆) rotameric mixture δ 8.44-8.63 (m, 1H), 8.11-8.37 (m, 1H), 7.27 (d, J=7.6 Hz, 1H), 7.07-7.17 (m, 2H), 5.26 (d, J=16.5 Hz, 1H), 4.04 (d, J=16.5 Hz, 1H), 3.71-3.82 (m, 1H), 3.00-3.52 (m, 5H), 2.60-2.99 (m, 10H), 2.35 (dd, J=16.8, 4.6 Hz, 1H), 1.80-1.93 (m, 1H), 1.01-1.63 (m, 6H); MS(ES) m/e 402.2 [M + H]⁺; Anal. [C₂₂H₃₁N₃O₄·1.75 (CF₃CO₂H)] calcd: C, 50.96; H, 5.49; N, 6.99; found: C, 51.14; H, 5.54; N, 7.00.

### Example 40

### Preparation of (R,S)-7-[(4,4'-bipiperidin-1-yl)carbonyl]-2,3,4,5-tetrahydro-3-oxo-4-(2-phenylethyl)-1H-1,4-benzodiazepine-2-acetic acid

a) methyl (R,S)-7-[1'-(t-butoxycarbonyl)-4,4'-bipiperidin-1-yl]carbonyl]-2,3,4,5-tetrahydro-3-oxo-4-(2-phenylethyl)-1H-1,4-benzodiazepine-2-acetate
   Using the procedure of Example 14(a), except substituting (R,S)-7-carboxy-2,3,4,5-tetrahydro-3-oxo-4-(2-phenylethyl)-1H-1,4-benzodiazepine-2-acetate for (R,S)-8-carboxy-2,3,4,5-tetrahydro-3-oxo-4-(phenylethyl)- 1H-1,4-benzodiazepine-2-acetate and substituting the compound of Preparation 15 for N-methyl-2-(4-pyridyl)-ethanamine and deleting triethylamine gave the title compound (0.43g, 68%): MS(ES) m/e 633.2 [M+H]⁺.
b) methyl (R,S)-7-[(4,4'-bipiperidin-1-yl]carbonyl]-2,3,4,5-tetrahydro-3-oxo-4-(2-phenylethyl)-1H-1,4-benzodiazepine-2-acetate
   Using the procedure of Preparation D(b), except substituting the compound of Example 40(a) for the compound D(a), gave the title compound (0.27g, 75%): MS(ES) m/e 533.2 [M+H]⁺; HPLC k'=9.4 (Vydac C18, 4.6 x 250 mm, 1.5 mL/min, gradient A:acetonitrile B:water-0.1% TFA, 5-50% acetonitrile during 20 min, UV detection at 254 nm).
c) (R,S)-7-[(4,4'-bipiperidin-1-yl)carbonyl]-2,3,4,5-tetrahydro-3-oxo-4-(2-phenylethyl)-1H-1,4-benzodiazepine-2-acetic acid
   The compound of Example 40(b) (0.2 g, 0.3 mmol) was suspended in acetone (2 mL) and treated lithium hydroxide hydrate (25 mg) in water (2 mL). The mixture was stirred overnight, treated with methanol and additional lithium hydroxide hydrate (5.5 mg) was added in two portions over 9 h. The mixture was concentrated and the aqueous residue was neutralized with N hydrochloric acid and concentrated. The residue was placed in the refrigerator overnight and filtered. The filter cake was washed with cold water, acetone and ether to yield the title compound (0.07 g). MS(ES) m/e 519.2 [M+H]⁺; HPLC k'=7.9 (Vydac ODS, 4.6 x 250 mm, 1.5 mL/min, gradient A:acetonitrile B:water-0.1% TFA, 5-50% A during 20 min, UV detection at 254 nm); ¹H NMR (400 MHz, CD₃OD) δ 7.2 (t, 2H), 7.14 (d, 3H), 7.10 (d, 1H), 7.08 (d, 1H), 7.04 (s, 1H), 6.59 (d, 1H), 5.46 (d, 1H), 5.15 (q, 1H), 3.87 (d, 1H), 3.76 ( m, 1H), 3.63 (m, 1H), 3.38 (d, 2H), 2.93 (dd, 4 H), 2.77 (t, 2H), 2.63 (dd, 1H), 1.93 (m, 2H) 1.45( bs, 2H), 1.23 (m, 4H), 1.23 (m, 2H).

### Example 41

### Preparation of 7-[3-(piperazin-1-yl)propyloxy]-2-(2-phenylethyl)-3-oxo-2,3-dihydro-1H-2-benzazepine-4-acetic acid

a) ethyl 7-methoxy-3-oxo-2,3-dihydro-2-(2-phenylethyl)-1H-2-benzazepine-4-acetate
   A solution of the compound of Example 21(h) (2.6 g, 6.8 mmol) in methanol (120 mL) was treated with 25% sodium methoxide/methanol solution (14.1 mL, 68 mmol) and heated to reflux for 2.5 h. The mixture was quenched with ice-water (300 mL), acidified with 3N hydrochloric acid, extracted with ethyl acetate, dried and concentrated to give the acid. MS(ES) m/e 352 [M+H]⁺. The residue was dissolved in ethanol (75 mL) and heated to reflux in the presence of 4M hydrogen chloride/dioxane (2 mL) for 2 h and concentrated to give the title compound (2.5 g, 97%). ¹H NMR (400 MHz, CDCl₃) δ 7.18-7.26 (m, 5H), 7.08 (d J=7.95 Hz, 1H), 6.89 (s 1H), 6.82 (m, 2H), 4.18 (m, 4H), 3.81 (s, 3H), 3.80 (t, J=5.57, 2H), 3.71 (t, J=5.57 Hz, 3H), 2.85 (t, J=7.62 Hz, 2H), 1.28 (t, J=6.5 Hz, 3H).
b) ethyl 7-hydroxy-2-3-oxo-(2-phenylethyl)-2,3-dihydro-1H-2-benzazepine-4-acetate
   To a solution of the compound of Example 41(a) (2.67 g, 7.0 mmol) and ethanethiol (2.6 mL, 35 mmol) in dichloromethane (50 mL) at 0°C was added aluminum chloride (4.6 g, 35 mmol). The mixture was warmed to RT over 1 h, diluted with dichloromethane (100 mL), washed with cold 3N hydrochloric acid, dried (sodium sulfate), and concentrated to give the title compound (2.4 g, 93%). MS(ES) m/e 366.0 [M+H]⁺; ¹H NMR (400 MHz, CDCl₃) δ 7.17-7.26 (m, 5H), 7.02 (d J=8.16 Hz, 1H), 6.78 (s 1H), 6.75 (m, 1H), 6.67 (d, J=2.4, 1H), 4.18 (q, J=7.16, 2H), 3.71 (t, J=5.57 Hz, 4H), 2.85 (t, J=7.58 Hz, 4H), 1.26 (t, J=7.16 Hz, 3H).
c) ethyl 7-[3-[(4-t-butyloxycarbonyl)piperazin-1-yl]propyloxy]-3-oxo-2-(2-phenylethyl)-2,3-dihydro-1H-2-benzazepine-4-acetate
   A mixture of the compound of Example 41(b) (1.28 g, 3.5 mmol) and 3-[(4-t-butyloxycarbonyl)piperazin-1-yl]propyl chloride (1.3 g, 5 mmol), potassium carbonate (0.58 g, 4.2 mmol),and tetraethylammonium iodide (20 mg) in dimethylformamide (15 mL) was heated at 90°C for 2 h. The reaction mixture was quenched with ice-water, extracted with ethyl acetate, dried (sodium sulfate), filtered and concentrated to give the title compound (1.5 g, 75%). MS(ES) m/e 592.0 [M+H]⁺; ¹H NMR (400 MHz, CDCl₃) δ 7.2-7.26 (m, 5H), 7.02 (d J=8.16 Hz, 1H), 6.83 (s, 1H), 6.81 (d, J=2.4, 1H), 4.19 (m, 4H), 4.0 (m, 2H), 3.70 (m, 4H), 3.46 (bs, 4H), 2.84 (t, J=7.56 Hz, 2H), 2.52 (t, J=7.1 Hz, 2H), 1.96 (m, 2H), 1.45 (s, 9H), 1.28 (t, J=7.23 Hz, 3H).
d) 7-[3-(1-piperazinyl)propyloxy]-2-(2-phenylethyl)-3-oxo-2,3-dihydro-1H-2-benzazepine-4-acetic acid
   A mixture of the compound of Example 41(c) (0.7 g, 1.2 mmol) was dissolved in 1N sodium hydroxide solution (12 mL) and methanol (35 mL). After stirring at RT for 4 h, the reaction mixture was quenched with ice-water, acidified with 1N hydrochloric acid, extracted with ethyl acetate, dried (sodium sulfate), filtered and concentrated to give the N-Boc acid (0.42 g, 63%; MS(ES) m/e 564.2 [M+H]⁺), which was stirred in 4N hydrogen chloride/dioxane solution (4 mL) and methylene chloride (30 mL) for 18 h. The mixture was evaporated to give the title compound (0.39 g, 97%). MS (ES) m/e 464.2 [M+H]⁺; ¹H NMR (400 MHz, CDCl₃) 8 7.16-7.83 (m, 6H), 7.06 (s, 1H) 6.94 (m, 2H), 4.15 (m, 2H), 3.65 (m, 14H), 3.47 (m, 2H), 2.82 (m, 2H), 2.32 (m, 2H).

### Example 42

### Preparation of 7-[3-(piperazin-1-yl)propyloxy]-2-(2-phenylethyl)-3-oxo-2,3,4,5-tetrahydro-1H-2-benzazepine-4-acetic acid

A solution of the compound of Example 41(d) (0.01 g, 0.02 mmol) in methanol (15 mL) is treated with palladium hydroxide (0.05 mg) and hydrogenated (3.45 bar) for 4 h to give the title compound.

### Example 43

Using routine variations of the procedures disclosed above for preparing 2,3,4,5-tetrahydro-3-oxo-1H-1,4-benzazepines, the following compound was prepared.

8-[[[2-(2-amino-pyridin-4-yl)-ethyl]methylamino] carbonyl]-2,3,4,5-tetrahydro-3-oxo-4-(2-phenylethyl)-1H-1,4-benzodiazepine-2-acetic acid;

### Example 44

### Parenteral Dosage Unit Composition

A preparation which contains 20 mg of the compound of Example 2 as a sterile dry powder is prepared as follows: 20 mg of the compound is dissolved in 15 mL of distilled water. The solution is filtered under sterile conditions into a 25 mL multi-dose ampoule and lyophilized. The powder is reconstituted by addition of 20 mL of 5% dextrose in water (D5W) for intravenous or intramuscular injection. The dosage is thereby determined by the injection volume. Subsequent dilution may be made by addition of a metered volume of this dosage unit to another volume of D5W for injection, or a metered dose may be added to another mechanism for dispensing the drug, as in a bottle or bag for IV drip infusion or other injection-infusion system.

### Example 45

### Oral Dosage Unit Composition

A capsule for oral administration is prepared by mixing and milling 50 mg of the compound of Example 15 with 75 mg of lactose and 5 mg of magnesium stearate. The resulting powder is screened and filled into a hard gelatin capsule.

### Example 46

### Oral Dosage Unit Composition

A tablet for oral administration is prepared by mixing and granulating 20 mg of sucrose, 150 mg of calcium sulfate dihydrate and 50 mg of the compound of Example 15 with a 10% gelatin solution. The wet granules are screened, dried, mixed with 10 mg starch, 5 mg talc and 3 mg stearic acid; and compressed into a tablet.

The foregoing is illustrative of the making and using of this invention. This invention, however, is not limited to the precise embodiments described herein, but encompasses all modifications within the scope of the claims which follow.

## Claims

1. A compound of the formula (I): 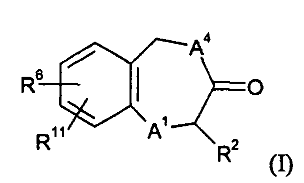 wherein
A¹ is O, S, N-R¹ or CHR¹;
A⁴ is N-R⁴ or CHR⁴;
R² is CH₂R⁷;
R¹ and R⁴ are H, Q-C₁₋₆alkyl, Q-C₁₋₆oxoalkyl, Q-C₂₋₆alkenyl, Q-C₃₋₄oxoalkenyl, Q-C₃₋₄oxoalkynyl, Q-C₂₋₄alkynyl, C₃₋₆cycloalkyl, Ar or Het, optionally substituted by one or more of R¹¹;
Q is H, C₃₋₆cycloalkyl, Het or Ar;
R⁶ is W-(CR'₂)_{q}-Z-(CR'R¹⁰)ᵣ-U-(CR'₂)ₛ-V-;
R⁷ is -COR⁸, -COCR'₂R⁹, -C(S)R⁸, -S(O)ₘOR', -S(O)ₘNR'R", -PO(OR'), -PO(OR')₂, -B(OR')₂, -NO₂ and Tet;
R⁸ is -OR', -NR'R", -NR'SO₂R', -NR'OR', -OCR'₂C(O)OR', -OCR'₂OC(O)-R', -OCR'₂C(O)NR'₂, CF₃ or AA1;
R⁹ is -OR', -CN, -S(O)ᵣR', S(O)ₘNR'₂, -C(O)R' C(O)NR'₂ or -CO₂R';
R¹⁰ is H, C₁₋₄alkyl or -NR'R";
R¹¹ is H, halo, -OR¹², -CN, -NR'R¹², -NO₂, -CF₃, CF₃S(O)ᵣ-, -CO₂R', -CONR'₂, Q-C₀₋₆alkyl-, Q-C₁₋₆oxoalkyl-, Q-C₂₋₆alkenyl-, Q-C₂₋₆alkynyl-, Q-C₀₋₆alkyloxy-, Q-C₀₋₆alkylamino- or Q-C₀₋₆alkyl-S(O)ᵣ₋;
R¹² is R', -C(O)R', -C(O)NR'₂, -C(O)OR¹⁵, -S(O)ₘR' or S(O)ₘNR'₂;
R¹⁵ is H, C₁₋₆alkyl or Ar-C₀₋₄alkyl;
R' is H, C₁₋₆alkyl, C₃₋₇cycloalkyl-C₀₋₄alkyl or Ar-C₀₋₄alkyl;
R" is R', -C(O)R' or -C(O)OR¹⁵;
R"' is R" or AA2;
AA1 is an amino acid attached through its amino group and having its carboxyl group optionally protected by replacing the OH of the carboxy group with R⁸, and AA2 is an amino acid attached through its carboxyl group, and having its amino group optionally protected by substituting the amino group with R¹²;
U and V are absent or CO, CR'₂, C(=CR'2), S(O)ₙ, O, NR', CR'OR', CR'(OR")CR'₂, CR'₂CR'(OR"), C(O)CR'₂, CR'₂C(O), CONR', NR'CO, OC(O), C(O)O, C(S)O, OC(S), C(S)NR', NR'C(S), S(O)ₙNR', NR'S(O)ₙ, N=N, NR'NR', NR'CR'₂,
CR'₂NR', CR'₂O, OCR'₂, C≡ C or CR'=CR', provided that U and V are not simultaneously absent;
W is R'R'''N- or 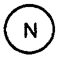 ;
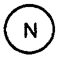 is a nitrogen heterocycle, which may be a saturated or unsaturated stable five-, six- or seven-membered monocyclic ring, or a seven- to ten-membered bicyclic ring containing up to three nitrogen atoms or containing one nitrogen atom and a heteroatom chosen from oxygen and sulfur, substituted in any stable position by R²⁰ or C₁₋₄alkyl substituted by R²⁰, wherein R²⁰ is H, C₁₋₄alkoxy, F, Cl, Br, I, NO₂, NR'₂, OH, CO₂R', CONHR', CF₃, Q-C₀₋₄alkyl, Q-C₁₋₄alkyl-S(O)ᵤ;
Het is an optionally substituted five- or six-membered monocyclic ring, or a nine-or ten-membered bicyclic ring containing one to three heteroatoms chosen from the group of nitrogen, oxygen and sulfur, substituted in any stable position by one to three moieties chosen from R¹¹;
Ar is phenyl or naphthyl, or phenyl or naphthyl substituted by one to three moieties chosen from R¹¹;
Tet is 5-tetrazolyl;
m is 1 or 2;
n is 0 to 3;
q is 0 to 3;
r is 0 to 2;
s is 0 to 2;
t is 0 to 2; and
u is 0, 1 or 2; or
a pharmaceutically acceptable salt thereof;
wherein either
(a) Z is non-aromatic Het;
or
(b) W is 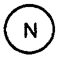 ;
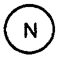 is a 4-substituted, 6-membered nitrogen heterocycle, which may be saturated or unsaturated containing up to three nitrogen atoms or containing one nitrogen atom and a heteroatom chosen from oxygen and sulfur, substituted in any stable position by R²⁰ or C₁₋₄alkyl substituted by R²⁰;
Z is (CH₂)ₜ;
V is absent;
U is chosen from NR'CO, CONR', CH₂O, OCH₂, CH₂CH₂, CR'=CR' or C≡ C;
s is 0; and
q+t+r is 1-3.

2. A compound according to claim 1 wherein:
R¹ and R⁴ are H, C₁₋₄alkyl, Ar-C₁₋₄alkyl or C₃₋₆cycloalkyl-C₁₋₄alkyl;
R² is CH₂CO₂R';
(CR'R¹⁰)ᵣ-U-(CR'₂)ₛ-V is (CH₂)₀₋₂NR'CO, (CH₂)₀₋₂CONR', (CH₂)₀₋₂CO,
(CH₂)₀₋₂CH=CH, (CH₂)₀₋₂ C≡C , (CH₂)₁₋₃O or (CH₂)₁₋₅;
W is 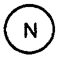 ; and
and Z is piperidinyl, piperazinyl or (CH₂)ₜ.

3. A compound according to claim 1 or 2 wherein A¹ is NR¹ or CHR¹ and A⁴ is NR⁴.

4. A compound according to any one of claims 1 to 3 wherein:
R¹ is H or methyl; and
R⁴ is H, methyl, cyclohexylethyl or phenylethyl.

5. A compound according to any one of claims 1-4 wherein R² is CH₂CO₂H.

6. A compound according to any one of claims 1-5 wherein R⁶ is: 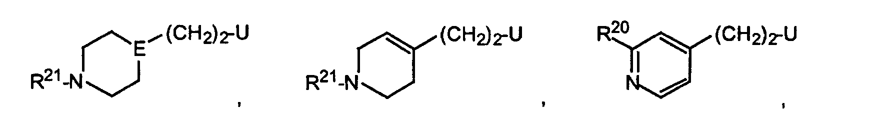 or 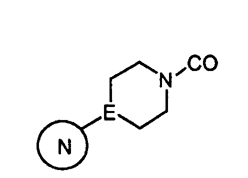 wherein E is N or CH, R²⁰ is hydrogen, amino, mono or di-C₁₋₄alkylamino, hydroxy or C₁₋₄alkyl and R²¹ is H or C₁₋₄alkyl.

7. A compound according to any one of claims 1 to 6 wherein 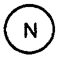 is 4-pyridyl, 4-(2-amino)pyridyl, 4-tetrahydropyridyl, 4-piperidyl or 4-piperazinyl.

8. A compound according to claim 6 wherein R⁶ is: 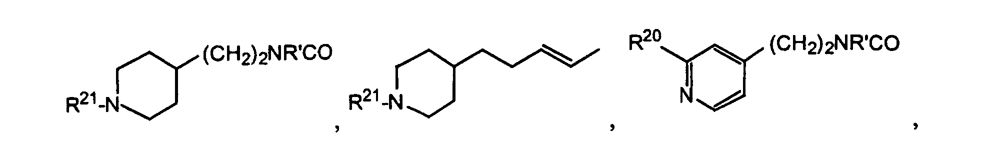 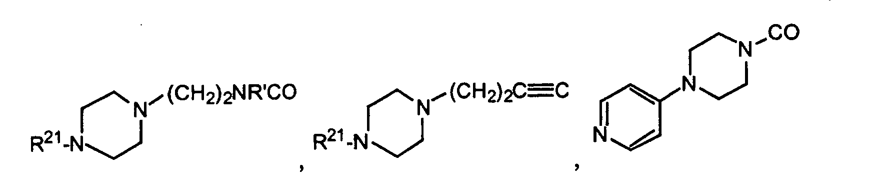 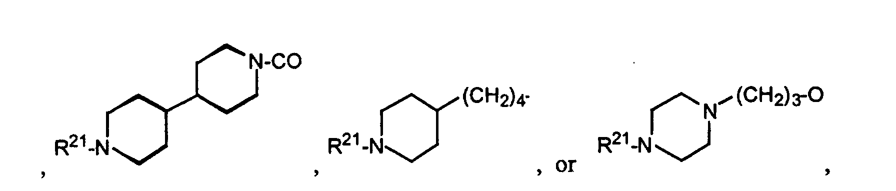 wherein R' and R²¹ are H or C₁₋₄alkyl, and R²⁰ is hydrogen, amino, mono- or di-C₁₋₄alkylamino, hydroxy or C₁₋₄alkyl.

9. A compound according to claim 8 wherein R⁶ is: 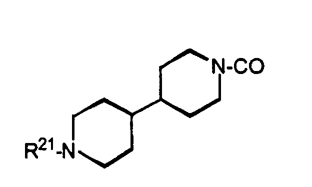 wherein R²¹ is H or C₁₋₄alkyl.

10. A compound according to claim 9 wherein R²¹ is H.

11. A compound which is:
(R,S)-2,3,4,5-tetrahydro-3-oxo-4-(2-phenylethyl)-8-[[[2-(4-pyridyl)ethyl]amino]carbonyl]-1H-1,4-benzodiazepine-2-acetic acid;
(R,S)-2,3,4,5-tetrahydro-3-oxo-4-(2-phenylethyl)-8-[[[2-(2-pyridyl)ethyl]amino]carbonyl]-1H-1,4-benzodiazepine-2-acetic acid;
(R,S)-8-[[(5-aminopentyl)amino]carbonyl]-2,3,4,5-tetrahydro-3-oxo-4-(2-phenylethyl)-1H-1,4-benzodiazepine-2-acetic acid;
(R,S)-8-[[[3-[4-piperidinyl]propyl]amino]carbonyl]-2,3,4,5-tetrahydro-3-oxo-4-(2-phenylethyl)-1H-1,4-benzodiazepine-2-acetic acid;
(R,S)-2,3,4,5-tetrahydro-3-oxo-4-(2-phenylethyl)-8-[[[4-(pyridyl)methyl]amino]carbonyl]-1H-1,4-benzodiazepine-2-acetic acid;
(R,S)-2,3,4,5-tetrahydro-8-[[[2-(1H-imidazol-1-yl)ethyl]amino]carbonyl]-3-oxo-4-(2-phenylethyl)-1H-1,4-benzodiazepine-2-acetic acid;
(R,S)-2,3,4,5-tetrahydro-8-[[[3-(1H-imidazol-1-yl)propyl]amino]carbonyl]-3-oxo-4-(2-phenylethyl)-1H-1,4-benzodiazepine-2-acetic acid;
(R,S)-8-[[[6-aminohexyl]amino]carbonyl]-2,3,4,5-tetrahydro-3-oxo-4-(2-phenylethyl)-1H-1,4-benzodiazepine-2-acetic acid;
(R,S)-8-[[[4-aminobutyl]amino]carbonyl]-2,3,4,5-tetrahydro-3-oxo-4-(2-phenylethyl)-1H-1,4-benzodiazepine-2-acetic acid;
(R,S)-2,3,4,5-tetrahydro-8-[[[2-(lH-imidazol-4-yl)ethyl]amino]carbonyl]-3-oxo-4-(2-phenylethyl)-1H-1,4-benzodiazepine-2-acetic acid;
(R,S)-2,3,4,5-tetrahydro-8-[[[2-(1-methyl- lH-imidazol-5-yl)ethyl]amino]carbonyl]-3-oxo-4-(2-phenylethyl)-1H-1,4-benzodiazepine-2-acetic acid;
(R,S)-8-[[[2-(4-pyridyl)ethyl]methyl-amino]carbonyl]-2,3,4,5-tetrahydro-3-oxo-4-(2-phenylethyl)-1H-1,4-benzodiazepine-2-acetic acid;
Methyl(R,S)-8-[[2-[4-[1-(methyl)pyridinium]-ethyl]methylamino]carbonyl]-2,3,4,5-tetrahydro-3-oxo-4-(2-phenylethyl)-1H-1,4-benzodiazepine-2-acetate Iodide;
(R,S)-2,3,4,5-tetrahydro-3-oxo-4-[2-(cyclohexyl)ethyl]-8-[[[2-(1-methyl-4-piperidinyl)ethyl]methylamino]carbonyl]-1H-1,4-benzodiazepine-2-acetic acid;
(R,S)-2,3,4,5-tetrahydro-3-oxo-4-(2-phenylethyl)-8-[[[2-(1-methyl-4-piperidinyl)ethyl]methylamino]carbonyl]-1H-1,4-benzodiazepine-2-acetic acid;
7-[3-(4-pyridyl)propyloxy]-2-(2-phenylethyl)-3-oxo-2,3,4,5-tetrahydro-1H-2-benzazepine-4-acetic acid;
7-[3-(4-piperidinyl)propyloxy]-2-(2-phenylethyl)-3-oxo-2,3,4,5-tetrahydro-1H-2-benzazepine-4-acetic acid;
8-[1-[4-(4-pyridyl)piperazinyl]carbonyl]-2,3,4,5-tetrahydro-3-oxo-4-(2-phenylethyl)-1H-1,4-benzodiazepine-2-acetic acid;
2-[3-(4-piperidinyl)propyloxy]-7-oxo-6,7,8,9-tetrahydro-5H-benzocycloheptene-6-acetic acid;
3-[3-(4-piperidinyl)propyloxy]-7-oxo-6,7,8,9-tetrahydro-5H-benzocycloheptene-6-acetic acid;
(R,S)-8-[[[2-[4-(2-aminopyridyl)]ethyl]-amino]carbonyl]-2,3,4,5-tetrahydro-3-oxo-4-(2-phenylethyl)-1H-1,4-benzodiazepine-2-acetic acid;
(R,S)-2,3,4,5-tetrahydro-3-oxo-4-(2-phenylethyl)-8-[[[2-(1-piperazinyl)ethyl]amino]carbonyl]-1H-1,4-benzodiazepine-2-acetic acid ;
(R,S)-2,3,4,5-tetrahydro-3-oxo-4-(2-phenylethyl)-8-[[[2-(4-piperidinyl)ethyl]amino]carbonyl]-1H-1,4-benzodiazepine-2-acetic acid;
(R,S)-2,3,4,5-tetrahydro-3-oxo-4-(2-phenylethyl)-8-[[[2-(1-piperazinyl)ethyl]methylamino]carbonyl]-1H-1,4-benzodiazepine-2-acetic acid;
(R,S)-2,3,4,5-tetrahydro-3-oxo-4-(2-phenylethyl)-8-[[[2-(4-piperidinyl)ethyl]methylamino]carbonyl]-1H-1,4-benzodiazepine-2-acetic acid ;
(R,S)-2,3,4,5-tetrahydro-3-oxo-4-[2-(cyclohexyl)-ethyl]-8-[[[2-(4-piperidinyl)ethyl]methylamino]carbonyl]-1H-1,4-benzodiazepine-2-acetic acid;
(R,S)-2,3,4,5-tetrahydro-8-[[[2-[1-methyl-1,2,5,6-tetrahydropyrid-4-yl]ethyl]methylamino]carbonyl]-3-oxo-4-(2-phenylethyl)- 1H-1,4-benzodiazepine-2-acetic acid;
(R,S)-2,3,4,5-tetrahydro-3-oxo-4-(2-phenylethyl)-8-[4-(piperidin-4-yl)-1(E)-butenyl]-1H-1,4-benzodiazepine-2-acetic acid;
(R,S)-2,3,4,5-tetrahydro-3-oxo-2-(2-cyclohexylethyl)-7-[[[2-(piperidin-4-yl)ethyl]methylamino]carbonyl]-1H-2-benzazepine-4-acetic acid;
(R,S)-2,3,4,5-tetrahydro-3-oxo-4-(2-phenylethyl)-7-[[4-(pyrid-4-yl)piperazin-1-yl]carbonyl]-1H-1,4-benzodiazepine-2-acetic acid;
(R,S)-2,3,4,5-tetrahydro-3-oxo-7-[[[2-(piperidin-4-yl)ethyl]methylamino]carbonyl]-1H-2-benzazepine-4-acetic acid;
(R,S)-7-[[[2-[(2-amino)pyrid-4-yl]ethyl]methylamino]carbonyl]-2,3,4,5-tetrahydro-3-oxo-2-(2-phenylethyl)-1H-2-benzazepine-4-acetic acid;
7-[1-[4-(piperazin-1-yl)but-1-ynyl]]-2-(2-phenylethyl)-3-oxo-2,3,4,5-tetrahydro-1H-2-benzazepine-4-acetic acid;
(R,S)-7-[[[2-(piperidin-4-yl)ethyl] methylamino]carbonyl]-2,3,4,5-tetrahydro-2-methyl-3-oxo-1H-2-benzazepine-4-acetic acid; or
8-[[[2-(2-amino-pyridin-4-yl)-ethyl]methylamino] carbonyl]-2,3,4,5-tetrahydro-3-oxo-4-(2-phenylethyl)-1H-1,4-benzodiazepine-2-acetic acid.

12. A compound according to claim 1 which is:
(R,S)-2,3,4,5-tetrahydro-3-oxo-2-(2-phenylethyl)-7-[[[2-(piperidin-4-yl)ethyl]methylamino]-carbonyl]-1H-2-benzazepine-4-acetic acid; or
(R,S)-7-[(4,4'-bipiperidin-1-yl)carbonyl]-2,3,4,5-tetrahydro-3-oxo-4-(2-phenylethyl)-1H-1,4-benzodiazepine-2-acetic acid.

13. An intermediate compound of the formula: 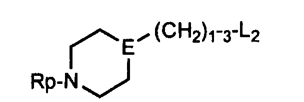 wherein
E is N or CH;
R^{P} is tosyl, butyloxycarbonyl, benzyloxycarbonyl or acetyl;
L² is CHO, CO₂R', C≡ C-H, OH, Cl, Br, I, CH₂-T or NR'R";
T is CF₃SO₃, OH, NHR", Cl, Br or I; and
R' and R" are as defined in claim 1.

14. A pharmaceutical composition comprising a compound according to any one of claims 1 to 12 and a pharmaceutically acceptable carrier.

15. A compound according to any one of claims 1 to 12 for use as a medicament.

16. The use of a compound according to any one of claims 1 to 12 in the manufacture of a medicament to inhibit platelet aggregation, or to treat myocardial infarction, transient ischemia attacks, stroke or reocclusion of an artery or vein following fibrinolytic therapy.

17. A process for preparing a compound of formula (I) according to claim 1, which comprises reacting a compound of formula (XI) with a compound of formula (XII): 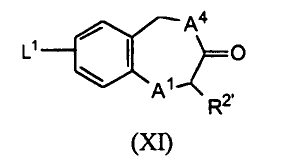
R^{6''}-L² (XII)
wherein A¹, A⁴, U, V, Z, R', R¹⁰, q, s and r are as defined in formula (I), with any reactive functional groups protected;
L¹ and L² are functional groups which are capable of reacting to form the linkage -(CR'R¹⁰)ᵣ-U-(CR'₂)ₛ-V-;
R^{6''} is W'-(CR'₂)_{q}-Z- and any portion of the group -(CR'R¹⁰)ᵣ-U-(CR'₂)ₛ-V- which is connected to L², with any reactive functional groups protected;
R^{2'} is R² as defined for formula (I) with any reactive group protected; and
W' is W as defined for formula (I) with any basic nitrogen group protected; to form a compound of the formula: 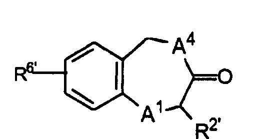
wherein R^{6'} is W'-(CR'₂)_{q}-Z-(CR'R¹⁰)ᵣ-U-(CR'₂)ₛ-V-;
and thereafter removing any protecting groups, and optionally forming a pharmaceutically acceptable salt.

## Patentansprüche

1. Verbindung der Formel (1): 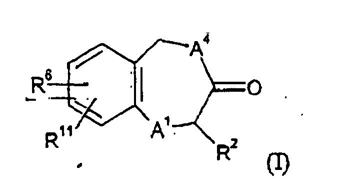 wobei
A¹ O, S, N-R¹ oder CHR¹ bedeutet;
A⁴ N-R⁴ oder CHR⁴ bedeutet;
R² CH₂R⁷ bedeutet;
R¹ und R⁴ H, einen Q-C₁₋₆-Alkyl-, Q-C₁₋₆-Oxoalkyl-, Q-C₂₋₆-Alkenyl-, Q-C₃₋₄-Oxoalkenyl-, Q-C₃₋₄-Oxoalkinyl-, Q-C₂₋₄-Alkinyl-, C₃₋₆-Cycloalkylrest, Ar oder Het bedeuten, welche gegebenenfalls mit einem oder mehreren der Reste R¹¹ substituiert sind;
Q H, einen C₃₋₆-Cycloalkylrest, Het oder Ar bedeutet;
R⁶ W-(CR'₂)_{q}-Z-(CR'R¹⁰)ᵣ-U-(CR'₂)ₛ-V- bedeutet;
R⁷ -COR⁸, -COCR'₂R⁹, -C(S)R⁸, -S(O)ₘOR', -S(O)ₘNR'R", -PO(OR'), -PO(OR')₂, -B(OR')₂, -NO₂ oder Tet bedeutet;
R⁸ -OR', -NR'R'', -NR'SO₂R', -NR'OR', -OCR'₂C(O)OR', -OCR'₂OC(O)-R', -OCR'₂C(O)NR'₂, CF₃ oder AA1 bedeutet;
R⁹-OR', -CN, -S(O)ᵣR', -S(O)ₘNR'₂, -C(O)R'C(O)NR'₂ oder -CO₂R' bedeutel;
R¹⁰ H, einen C₁₋₄-Alkylrest oder -NR'R'' bedeutet;
R¹¹ H, ein Halogenatom, -OR¹², -CN, -NR'R¹², -NO₂, -CF₃, CF₃S(O)ᵣ-, -CO₂R', -CONR'₂, einen Q-C₀₋₆-Alkyl-, Q-C₁₋₆-Oxoalkyl-, Q-C₂₋₆-Alkenyl-, Q-C₂₋₆-Alkinyl-, Q-C₀₋₆-Alkyloxy-, Q-C₀₋₆-Alkylamino- oder Q-C₀₋₆-Alkyl-S(O)ᵣ-Rest bedeutet;
R¹² R', -C(O)R', -C(O)NR'₂, -C(O)OR¹⁵, -S(O)ₘR' oder -S(O)ₘNR'₂ bedeutet;
R¹⁵ H, einen C₁₋₆-Alkyl- oder Ar-C₀₋₄-Alkylrest bedeutet;
R' H, einen C₁₋₆-Alkyl-, C₃₋₇-Cycloalkyl-C₀₋₄-Alkyl- oder Ar-C₀₋₄-Alkylrest bedeutet;
R" R', -C(O)R' oder -C(O)OR¹⁵ bedeutet;
R''' R" oder AA2 bedeutet;
AA1 eine Aminosäure bedeutet, welche über ihre Aminogruppe gebunden ist und deren Carboxylgruppe gegebenenfalls dadurch geschützt ist, daß die OH-Gruppe der Carboxygruppe durch R⁸ ersetzt ist, und AA2 eine Aminosäure bedeutet, welche über ihre Carboxylgruppe gebunden ist, und deren Aminogruppe gegebenenfalls dadurch geschützt ist, daß die Aminogruppe mit R¹² substituiert ist;
U und V abwesend sind oder CO, CR'₂, C(=CR'₂), S(O)ₙ, O, NR', CR'OR', CR'(OR")CR'₂, CR'₂CR'(OR"), C(O)CR'₂, CR'₂C(O), CONR', NR'CO, OC(O), C(O)O, C(S)O, OC(S), C(S)NR', NR'C(S), S(O)ₙNR', NR'S(O)ₙ, N=N, NR'NR', NR'CR'₂, CR'₂NR', CR'₂O, OCR'₂, C≡C oder CR'=CR' bedeuten, mit der Maßgabe, daß U und V nicht gleichzeitig abwesend sind;
W R'R'''N- oder 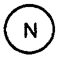 bedeutet;
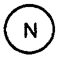 einen stickstoffhaltigen Heterocyclus bedeutet, welcher ein gesättigter oder ungesättigter stabiler 5-, 6- oder 7-gliedriger monocyclischer Ring, oder ein 7- bis 10-gliedriger bicyclischer Ring sein kann, welcher bis zu 3 Stickstoffatome, oder ein Stickstoffatom und ein Heteroatom enthält, welches ausgewählt ist aus einem Sauerstoff- und Schwefelatom, und welcher in beliebigen stabilen Positionen mit R²⁰ oder einem mit R²⁰ substituierten C₁₋₄-Alkylrest substituiert ist, wobei R²⁰ H, einen C₁₋₄-Alkoxyrest, F, Cl, Br, I, NO₂, NR'₂, OH, CO₂R', CONHR', CF₃, einen Q-C₀₋₄-Alkyl- oder Q-C₁₋₄-Alkyl-S(O)ᵤ-Rest bedeutet;
Het einen gegebenenfalls substituierten 5- oder 6-gliedrigen monocyclischen Ring oder einen 9- oder 10-gliedrigen bicyclischen Ring bedeutet, welcher ein bis drei Heteroatome enthält, welche ausgewählt sind aus einem Stickstoff-, Sauerstoff- und Schwefelatom, und welcher in beliebigen stabilen Positionen mit einem bis drei der Reste R" substituiert ist;
Ar eine Phenyl- oder Naphthylgruppe oder einen Phenyl- oder Naphthylrest bedeutet, welcher mit einem bis drei der Reste R¹¹ substituiert ist;
Tet gleich 5-Tetrazolyl ist;
m gleich 1 oder 2 ist;
n gleich 0 bis 3 ist;
q gleich 0 bis 3 ist;
r gleich 0 bis 2 ist;
s gleich 0 bis 2 ist;
t gleich 0 bis 2 ist; und
u gleich 0, 1 oder 2 ist; oder
ein pharmazeutisch verträgliches Salz davon;
wobei entweder
(a) Z ein nicht-aromatischer Rest Het ist;
oder
(b) W 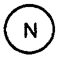 bedeutet;
wobei 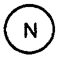 einen 4-substituierten, 6-gliedrigen, stickstoffhaltigen Heterocyclus bedeutet, welcher gesättigt oder ungesättigt sein kann und bis zu 3 Stickstoffatome oder ein Stickstoffatom und ein Heteroatom, ausgewählt aus einem Sauerstoff- und Schwefelatom, enthält, und welcher in beliebigen stabilen Positionen mit R²⁰ oder einem mit R²⁰ substituierten C₁₋₄-Alkylrest substituiert ist;
Z (CH₂)ₜ bedeutet;
V abwesend ist;
U ausgewählt ist aus NR'CO, CONR', CH₂O, OCH₂, CH₂CH₂, CR'=CR' oder C≡C-;
s 0 ist; und
q+t+r 1 bis 3 ist.

2. Verbindung gemäß Anspruch 1, wobei:
R¹ und R⁴ H, einen C₁₋₄-Alkyl-, Ar-C₁₋₄-Alkyl- oder C₃₋₆-Cycloalkyl-C₁₋₄-Alkylrest bedeuten;
R² CH₂CO₂R' ist;
(CR'R¹⁰)ᵣ-U-(CR'₂)ₛ-V (CH₂)₀₋₂NR'CO₂ (CH₂)₀₋₂CONR', (CH₂)₀₋₂CO, (CH₂)₀₋₂CH=CH, (CH₂)₀₋₂C≡C, (CH₂)₁₋₃O oder (CH₂)₁₋₅ ist;
W 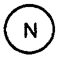 ist; und
Z eine Piperidinyl-, Piperazinylgruppe oder (CH₂)ₜ bedeutet.

3. Verbindung gemäß einem der Ansprüche 1 oder 2, wobei A¹ NR¹ oder CHR¹ und A⁴ gleich NR⁴ bedeutet.

4. Verbindung gemäß einem der Ansprüche 1 bis 3, wobei:
R¹ H oder eine Methylgruppe ist; und
R⁴ H, eine Methyl-, Cyclohexylethyl- oder Phenylethylgruppe ist.

5. Verbindung gemäß einem der Ansprüche 1 bis 4, wobei R² CH₂CO₂H ist.

6. Verbindung gemäß einem der Ansprüche 1 bis 5, wobei R⁶ 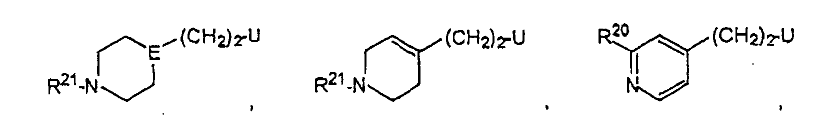 oder 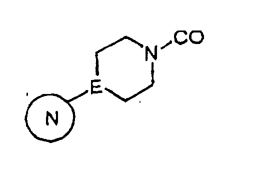 bedeutet, wobei E N oder CH ist, R²⁰ ein Wasserstoffatom, eine Aminogruppe, einen Mono- oder Di-C₁₋₄-Alkylaminorest, eine Hydroxygruppe oder einen C₁₋₄-Alkylrest bedeutet und R²¹ H oder ein C₁₋₄-Alkylrest ist.

7. Verbindung gemäß einem der Ansprüche 1 bis 6, wobei 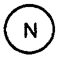 eine 4-Pyridyl-, 4-(2-Amino)pyridyl-, 4-Tetrahydropyridyl-, 4-Piperidyl- oder 4-Piperazinylgruppe bedeutet.

8. Verbindung gemäß Anspruch 6, wobei R⁶ 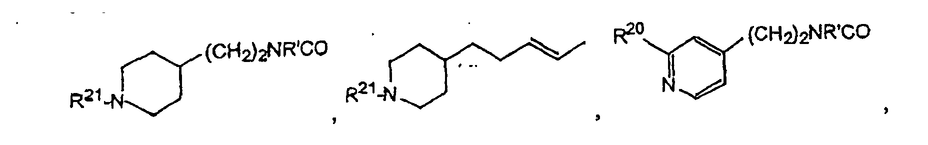 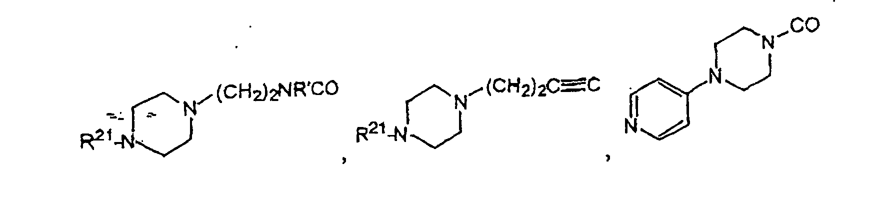 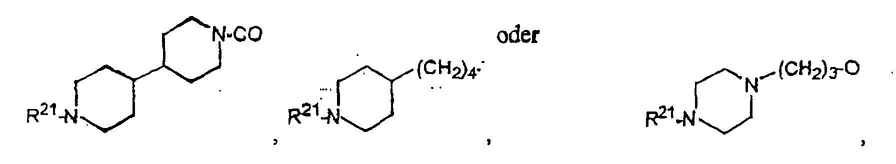 bedeutet, wobei R' und R²¹ H oder ein C₁₋₄-Alkylrest sind, und R²⁰ ein Wasscrstoffatom, eine Aminogruppe, einen Mono- oder Di-C₁₋₄-Alkylaminorest, eine Hydroxygruppe oder einen C₁₋₄-Alkylrest bedeutet.

9. Verbindung gemäß Anspruch 8, wobei R⁶ 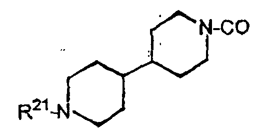 bedeutet, wobei R²¹ H oder ein C₁₋₄-Alkylrest ist.

10. Verbindung gemäß Anspruch 9, wobei R²¹ H ist.

11. Verbindung , nämlich:
(R,S)-2,3,4,5-Tetrahydro-3-oxo-4-(2-phenylethyl)-8-[[[2-(4-pyridyl)ethyl]amino]-carbonyl]-1H-1,4-benzodiazepin-2-essigsäure;
(R,S)-2,3,4,5-Tetrahydro-3-oxo-4-(2-phenylethyl)-8-[[[2-(2-pyridyl)ethyl]amino]-carbonyl]-1H-1,4-benzodiazepin-2-essigsäure;
(R,S)-8-[[(5-Aminopentyl)amino]carbonyl]-2,3,4,5-tetrahydro-3-oxo-4-(2-phenylethyl)-1H-1,4-benzodiazepin-2-essigsäure;
(R,S)-2,3,4,5-Tetrahydro-8-[[[2-(1H-imidazol-1-yl)ethyl]amino]carbonyl]-3-oxo-4-(2-phenylethyl)-1H-1,4-benzodiazepin-2-essigsäure;
(R,S)-2,3,4,5-Tetrahydro-8-[[[3-(1H-imidazol-1-yl)propyl]amino]carbonyl]-3-oxo-4-(2-phenylethyl)-1H-1,4-benzodiazepin-2-essigsäure;
(R,S)-8-[[[6-Aminohexyl]amino]carbonyl]-2,3,4,5-tetrahydro-3-oxo-4-(2-phenylethyl)-1H-1,4-benzodiazepin-2-essigsäure;
(R,S)-8-[[[4-Aminobutyl]amino]carbonyl]-2,3,4,5-tetrahydro-3-oxo-4-(2-phenylethyl)-1H-1,4-benzodiazepin-2-essigsäure;
(R,S)-2,3,4,5-Tetrahydro-8-[[[2-(1H-imidazol-4-yl)ethyl]amino]carbonyl]-3-oxo-4-(2-phenylethyl)-1H-1,4-benzodiazepin-2-essigsäure;
(R,S)-2,3,4,5-Tetrahydro-8-[[[2-(1-methyl-1H-imidazol-5-yl)ethyl]amino]carbonyl]-3-oxo-4-(2-phenylethyl)-1H-1,4-benzodiazepin-2-essigsäure;
(R,S)-8-[[[2-(4-Pyridyl)ethyl]methylamino]carbonyl]-2,3,4,5-tetrahydro-3-oxo-4-(2-phenylethyl)-1H-1,4-benzodiazepin-2-essigsäure;
Methyl(R,S)-8-[[2-[4-[1-(methyl)pyridinium]ethyl]methylamino]carbonyl]-2,3,4,5-tetrahydro-3-oxo-4-(2-phenylethyl)-1H-1,4-benzodiazepin-2-acetatiodid;
(R,S)-2,3,4,5-Tetrahydro-3-oxo-4-[2-(cyclohexyl)ethyl]-8-[[[2-(1-methyl-4-piperidinyl)-ethyl]methylamino]carbonyl]-1H-1,4-benzodiazepin-2-essigsäure;
(R,S)-2,3,4,5-Tetrahydro-3-oxo-4-(2-phenylethyl)-8-[[[2-(1-methyl-4-piperidinyl)-ethyl]methylamino]carbonyl]-1H-1,4-benzodiazepin-2-essigsäure;
7-[3-(4-Pyridyl)propyloxy]-2-(2-phenylethyl)-3-oxo-2,3,4,5-tetrahydro-1H-2-benzazepin-4-essigsäure;
7-[3-(4-Piperidinyl)propyloxy]-2-(2-phenylethyl)-3-oxo-2,3,4,5-tetrahydro-1H-2-benzazepin-4-essigsäure;
8-[1-[4-(4-Pyridyl)piperazinyl]carbonyl]-2,3,4,5-tetrahydro-3-oxo-4-(2-phenylethyl)-1 H-1,4-benzodiazepin-2-essigsäure;
2-[3-(4-Piperidinyl)propyloxy]-7-oxo-6,7,8,9-tetrahydro-5H-henzocyclohepten-6-essigsäure;
3-[3-(4-Piperidinyl)propyloxy]-7-oxo-6,7,8,9-tetrahydro-5H-benzocyclohepten-6-essigsäure;
(R,S)-8-[[[2-[4-(2-Aminopyridyl)]ethyl]amino]carbonyl]-2,3,4,5-tetrahydro-3-oxo-4-(2-phenylethyl)-1H-1,4-benzodiazepin-2-essigsäure; ;
(R,S)-2,3,4,5-Tetrahydro-3-oxo-4-(2-phenylethyl)-8-[[[2-(1-piperazinyl)ethyl]amino]-carbonyl]-1H-1,4-benzodiazepin-2-essigsäure;
(R,S)-2,3,4,5-Tetrahydro-3-oxo-4-(2-phenylethyl)-8-[[[2-(4-piperidinyl)ethyl]amino]-carbonyl]-1H-1,4-benzodiazepin-2-essigsäure;
(R,S)-2,3,4,5-Tetrahydro-3-oxo-4-(2-phenylethyl)-8-[[[2-(1-piperazinyl)ethyl]methylamino]carbonyl]-1H-1,4-benzodiazepin-2-essigsäure;
(R,S)-2,3,4,5-Tetrahydro-3-oxo-4-(2-phenylethyl)-8-[[[2-(4-piperidinyl)ethyl]methylamino]carbonyl]-1H-1,4-benzodiazepin-2-essigsäure;
(R,S)-2,3,4,5-Tetrahydro-3-oxo-4-[2-(cyclohexyl)ethyl]-8-[[[2-(4-piperidinyl)ethyl]-methylamino]carbonyl]-1H-1,4-benzodiazepin-2-essigsäure;
(R,S)-2,3,4,5-Tetrahydro-8-[[[2-[1-methyl-1,2,5,6-tetrahydropyrid-4-yl]ethyl]methylamino]carbonyl]-3-oxo-4-(2-phenylethyl)-1H-1,4-benzodiazepin-2-essigsäure;
(R,S)-2,3,4,5-Tetrahydro-3-oxo-4-(2-phenylethyl)-8-[4-(piperidin-4-yl)-1(E)-butenyl]-1H-1,4-benzodiazepin-2-essigsäure;
(R,S)-2,3,4,5-Tetrahydro-3-oxo-2-(2-cyclohexylethyl)-7-[[[2-(piperidin-4-yl)ethyl]-methylamino]carbonyl]-1H-2-benzazepin-4-essigsäure;
(R,S)-2,3,4,5-Tetrahydro-3-oxo-4-(2-phenylethyl)-7-[[4-(pyrid-4-yl)piperazin-1-yl]carbonyl]-1H-1,4-benzodiazepin-2-essigsäure;
(R,S)-2,3,4,5-Tetrahydro-3-oxo-7-[[[2-(piperidin-4-yl)ethyl]methylamino]carbonyl]-1H-2-benzazepin-4-essigsäure;
(R,S)-7-[[[2-[(2-Amino)pyrid-4-yl]ethyl]methylamino]carbonyl]-2,3,4,5-tetrahydro-3-oxo-2-(2-phenylethyl)-1 H-2-benzazepin-4-essigsäure;
7-[1-[4-(Piperazin-1-yl)but-1-inyl]]-2-(2-phenylethyl)-3-oxo-2,3,4,5-tetrahydro-1H-2-benzazepin-4-essigsäure;
(R,S)-7-[[[2-(piperidin-4-yl)ethyl]methylamino]carbonyl]-2,3,4,5-tetrahydro-2-methyl-3-oxo-1H-2-benzazepin-4-essigsäure; oder
8-[[[2-(2-Aminopyridin-4-yl)ethyl]methylamino]carbonyl]-2,3,4,5-tetrahydro-3-oxo-4-(2-phenylethyl)-1H-1,4-benzodiazepin-2-essigsäure.

12. Verbindung, nämlich:
(R,S)-2,3,4,5-Tetrahydro-3-oxo-2-(2-phenylethyl)-7-[[[2-(piperidin-4-yl)ethyl]methylamino]carbonyl]-1H-2-benzazepin-4-essigsäure; oder
(R,S)-7-[(4,4'-Bipiperidin-1-yl)carbonyl]-2,3,4,5-tetrahydro-3-oxo-4-(2-phenylethyl)-1H-1,4-benzodiazepin-2-essigsäure.

13. Intermediäre Verbindung der Formel: 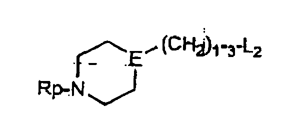 wobei
E gleich N oder CH ist;
R^{p} eine Tosyl-, Butyloxycarbonyl-, Benzyloxycarbonyl- oder Acetylgruppe is
L² gleich CHO, CO₂R', C≡C-H, OH, Cl, Br, I, CH₂-T oder NR'R" ist;
T gleich CF₃SO₃, OH, NHR", Cl, Br oder I ist; und
R' und R" wie in Anspruch 1 definiert sind.

14. Arzneimittel, umfassend eine Verbindung gemäß einem der Ansprüche 1 bis 12 und einen pharmazeutisch verträglichen Träger.

15. Verbindung gemäß einem der Ansprüche 1 bis 12 zur Verwendung als Medikament.

16. Verwendung einer Verbindung gemäß einem der Ansprüche 1 bis 12 zur Herstellung eines Medikaments zur Inhibierung von Blutplättchenaggregation oder um Herzinfarkt, transitorische ischämische Attacken, Schlaganfall oder Reocclusion einer Arterie oder Vene nach fibrinolytischer Therapie zu behandeln.

17. Verfahren zur Herstellung einer Verbindung der Formel (I) gemäß Anspruch 1, welches die Umsetzung einer Verbindung der Formel (XI) mit einer Verbindung der Formel (XII) umfasst: 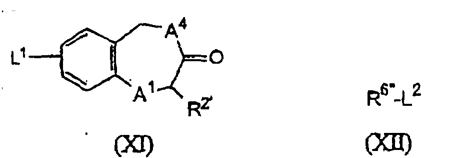
R^{6''} -L² (XII)
wobei A¹, A⁴, U, V, Z, R', R¹⁰, q, s und r wie für Formel (I) definiert sind, wobei sämtliche reaktiven funktionellen Gruppen geschützt sind;
L¹ und L² funktionelle Gruppen bedeuten, welche unter Bildung der verknüpfenden Einheit -(CR'R¹⁰)ᵣ-U-(CR'₂)ₛ-V- reagieren können;
R^{6"} W'-(CR'₂)_{q}-Z- und ein beliebiger Teil der Einheit -(CR'R¹⁰)ᵣ-U-(CR'₂)ₛ-V-, welcher an L² gebunden ist, bedeutet; wobei sämtliche reaktiven funktionellen Gruppen geschützt sind;
R^{2'} R², wie für Formel (I) definiert, bedeutet, wobei sämtliche reaktiven Gruppen geschützt sind; und
W' W, wie für Formel (I) definiert, bedeutet, wobei sämtliche basischen Stickstoffgruppen geschützt sind;
um eine Verbindung der Formel: 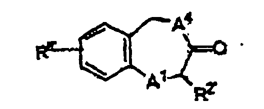 zu bilden, wobei
R⁶' W'-(CR'₂)_{q}-Z-(CR'R¹⁰)ᵣ-U-(CR'₂)ₛ-V- bedeutet; und
das anschließende Entfernen sämtlicher Schutzgruppen, und gegebenenfalls die Bildung eines pharmazeutisch verträglichen Salzes umfaßt.

## Revendications

1. Composé de formule (I) : 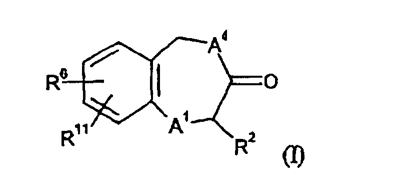 dans laquelle
A¹ représente un groupe O, S, N-R¹ ou CHR¹ ;
A⁴ représente un groupe N-R⁴ ou CHR⁴ ;
R² représente un groupe CH₂R⁷ ;
R¹ et R⁴ représentent H, un groupe Q-(alkyle en C₁ à C₆), Q(oxoalkyle en C₁ à C₆), Q(alcényle en C₂ à C₆), Q(oxoalcényle en C₃ à C₄), Q(oxoalcynyle en C₃ ou C₄), Q(alcynyle en C₂ à C₄), cycloalkyle en C₃ à C₆, Ar ou Het, facultativement substitué avec un ou plusieurs groupes R¹¹ ;
Q représente H, un groupe cycloalkyle en C₃ à C₆, Het ou Ar ;
R⁶ représente un groupe W-(CR'₂)_{q}-Z-(CR'R¹⁰)ᵣ-U-(CR'₂)ₛ-V- ;
R⁷ représente un groupe -COR⁸, -COCR'₂R⁹, -C(S)R⁸, -S(O)ₘOR', -S(O)ₘNR'R", -PO(OR'), -PO(OR')₂, -B(OR')₂, -NO₂ et Tet ;
R⁸ représente un groupe -OR', -NR'R", -NR'SO₂R', -NR'OR', -OCR'₂C(O)OR', -OCR'₂OC(O)-R', -OCR'₂C(O)NR'₂, CF₃ ou AA1 ;
R⁹ représente un groupe -OR', -CN, -S(O)ᵣR', S(O)ₘNR'₂, -C(O)R'C(O)NR'₂ ou -CO₂R' ;
R¹⁰ représente H, un groupe alkyle en C₁ à C₄ ou -NR'R" ;
R¹¹ représente H, un groupe halogéno, -OR¹², -CN, -NR'R¹², -NO₂, -CF₃, CF₃S(O)ᵣ-, -CO₂R', -CONR'₂, Q(alkyle en C₀ à C₆)-, Q(oxoalkyle en C₁ à C₆)-, Q(alcényle en C₂ à C₆)-, Q(alcényle en C₂ à C₆)-, Q(alcynyle en C₂ à C₆), Q(alkyle en C₀ à C₆)oxy-, Q(alkyle en C₀ à C₆)amino- ou Q(alkyle en C₀ à C₆)S(O)ᵣ-,
R¹² représente un groupe R', -C(O)R', -C(O)NR'₂, -C(O)OR¹⁵, -S(O)ₘR' ou S(O)ₘNR'2 ;
R¹⁵ représente H, un groupe alkyle en C₁ à C₆ ou Ar(alkyle en C₀ à C₄) ;
R' représente H, un groupe alkyle en C₁ à C₆, (cycloalkyle en C₃ à C₇) (alkyle en C₀ à C₄) ou Ar(alkyle en C₀ àC₄) ;
R" représente un groupe R', -C(O)R' ou -C(O)OR¹⁵ ;
R"' représente un groupe R" ou AA2 ;
AA1 représente un aminoacide fixé par son groupe amino et ayant son groupe carboxyle facultativement protégé en remplaçant le groupe OH du groupe carboxy par R⁸, et AA2 représente un aminoacide fixé par son groupe carboxyle, et ayant son groupe amino facultativement protégé par substitution du groupe amine avec R¹² ;
U et V sont absents ou représentent des groupes CO, CR'₂, C(=CR'₂), S(O)ₙ, O, NR', CR'OR', CR'(OR")CR'₂, CR'₂CR'(OR"), C(O)CR'₂, CR'₂C(O), CONR', NR'CO, OC(O), C(O)O, C(S)O, OC(S), C(S)NR', NR'C(S), S(O)ₙNR', NR'S(O)ₙ, N=N, NR'NR', NR'CR'₂, CR'₂NR', CR'₂O, OCR'₂, C≡C ou CR'=CR', sous réserve que U et V ne soient pas simultanément absents ;
W représente un groupe R'R'' 'N- ou 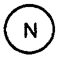 :
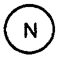 représente un hétérocycle azoté, qui peut être un noyau monocyclique penta-, hexa- ou heptagonal stable saturé ou insaturé, ou un noyau bicyclique hepta- à décagonal contenant jusqu'à trois atomes d'azote ou contenant un atome d'azote et un hétéroatome choisi entre l'oxygène et le soufre, substitué dans n'importe quelle position stable avec un groupe R²⁰ ou alkyle en C₁ à C₄ substitué avec un groupe R²⁰, dans lequel R²⁰ représente H, un groupe alkoxy en C₁ à C₄, F, Cl, Br, I, NO₂, NR'₂, OH, CO₂R', CONHR', CF₃, Q(alkyle en C₀ à C₄) ou Q(alkyle en C₁ à C₄)S(O)ᵤ ;
Het représente un noyau monocyclique penta- ou hexagonal facultativement substitué, ou un noyau bicyclique nona- ou décagonal contenant un à trois hétéroatomes choisis dans le groupe consistant en l'azote, l'oxygène et le soufre, substitué dans n'importe quelle position stable avec un à trois groupements choisis parmi les groupements R¹¹ ;
Ar représente un groupe phényle ou naphtyle, ou phényle ou naphtyle substitué avec un à trois groupements choisis parmi les groupements R¹¹ ;
Tet représente un groupe 5-tétrazolyle ;
m est égal à 1 ou 2 ;
n a une valeur de 0 à 3 ;
q a une valeur de 0 à 3 ;
r a une valeur de 0 à 2 ;
s a une valeur de 0 à 2 ;
t a une valeur de 0 à 2 ; et
u est égal à 0, 1 ou 2 ; ou
un de ses sels pharmaceutiquement acceptables ;
dans lequel soit
(a) Z représente un groupe Het non aromatique ;
soit
(b) W représente un groupe 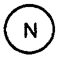
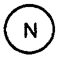 représente un hétérocycle azoté hexagonal substitué en position quatre, qui peut être saturé ou insaturé, contenant jusqu'à trois atomes d'azote ou contenant un atome d'azote et un hétéroatome choisi entre l'oxygène et le soufre, substitué dans n'importe quelle position stable avec un groupe R²⁰ ou alkyle en C₁ à C₄ substitué avec un groupe R²⁰ ;
Z représente un groupe (CH_{^{'}2})t ;
V est absent ;
U est choisi entre des groupes NR'CO, CONR', CH₃O, OCH₂, CH₂CH₂, CR'=CR' et C≡C ;
S est égal à 0 ; et
la somme q+t+r a une valeur de 1 à 3.

2. Composé suivant la revendication 1, dans lequel :
R¹ et R⁴ représentent H, des groupes alkyle en C₁ à C₄, Ar(alkyle en C₁ à C₄) ou (cycloalkyle en C₃ à C₆)(alkyle en C₁ à C₄) ;
R² représente un groupe CH₂CO₂R' ;
le groupe (CR'R¹⁰)ᵣ-U-(CR'₂)ₛ-V est un groupe (CH₂)₀₋₂NR'CO, (CH₂)₀₋₂CONR', (CH₂)₀₋₂CO, (CH₂)₀₋₂CH=CH, (CH₂)₀₋₂ C≡C, (CH₂)₁₋₃O ou (CH₂)₁₋₅ ;
W représente un groupe 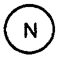 ; et
Z représente un groupe pipéridinyle, pipérazinyle ou (CH₂)t.

3. Composé suivant la revendication 1 ou 2, dans lequel A¹ représente un groupe NR¹ ou CHR¹ et A⁴ représente un groupe NR⁴.

4. Composé suivant l'une quelconque des revendications 1 à 3, dans lequel :
R¹ représente H ou un groupe méthyle ; et
R⁴ représente H, un groupe méthyle, cyclohexyléthyle ou phényléthyle.

5. Composé suivant l'une quelconque des revendications 1 à 4, dans lequel R² représente un groupe CH₂CO₂H.

6. Composé suivant l'une quelconque des revendications 1 à 5, dans lequel R⁶ représente un groupe 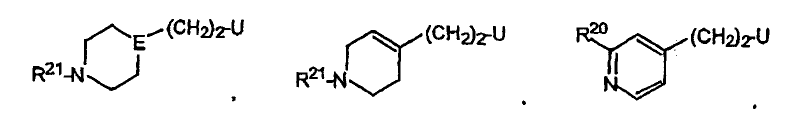 ou 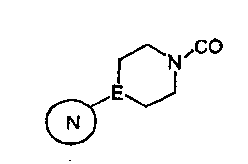 dans lequel E représente N ou un groupe CH, R²⁰ représente un atome d'hydrogène, un groupe amino, mono- ou di-(alkyle en C₁ à C₄)amino, hydroxy ou alkyle en C₁ à C₄ et R²¹ représente H ou un groupe alkyle en C₁ à C₄.

7. Composé suivant l'une quelconque des revendications 1 à 6, dans lequel le groupe 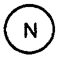 est un groupe 4-pyridyle, 4-(2-amino)pyridyle, 4-tétrahydropyridyle, 4-pipéridyle ou 4-pipérazinyle.

8. Composé suivant la revendication 6, dans lequel R⁶ représente un groupe : 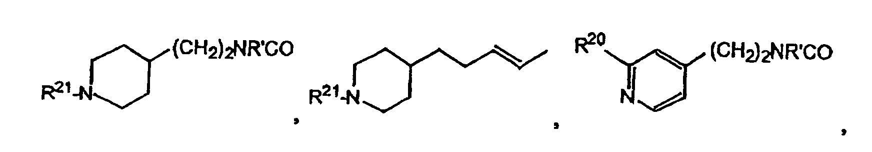 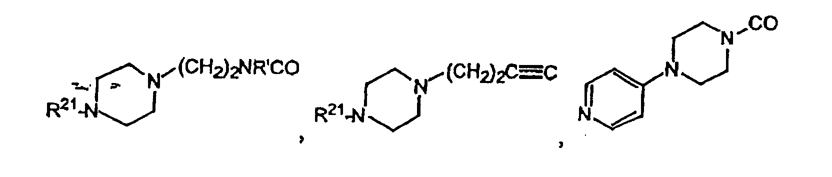 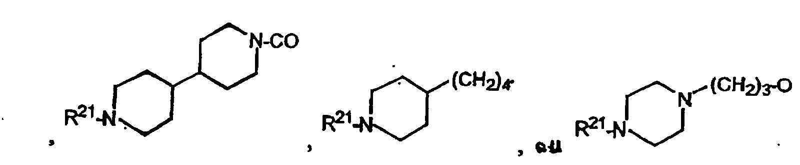 dans lequel R' et R²¹ représentent H ou des groupes alkyle en C₁ à C₄, et R²⁰ représente un atome d'hydrogène, un groupe amino, mono- ou di-(alkyle en C₁ à C₄)amino, hydroxy ou alkyle en C₁ à C₄.

9. Composé suivant la revendication 8, dans lequel R⁶ représente un groupe : 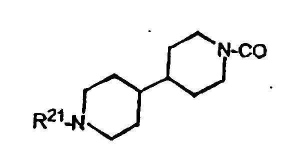 dans lequel R²¹ représente H ou un groupe alkyle en C₁ à C₄.

10. Composé suivant la revendication 9, dans lequel R²¹ représente H.

11. Composé, qui consiste en :
acide (R,S)-2,3,4,5-tétrahydro-3-oxo-4-(2-phényléthyl)-8-[[[2-(4-pyridyl)éthyl]amino]carbonyl]-1H-1,4-benzodiazépine-2-acétique ;
acide (R,S)-2,3,4,5-tétrahydro-3-oxo-4-(2-phényléthyl)-8-[[[2-(2-pyridyl)éthyl]amino]carbonyl]-1H-1,4-benzodiazépine-2-acétique ;
acide (R,S)-8-[[(5-aminopentyl]amino]carbonyl]-2,3,4,5-tétrahydro-3-oxo-4-(2-phényléthyl)-1H-1,4-benzodiazépine-2-acétique ;
acide (R,S)-2,3,4,5-tétrahydro-8-[[[2-(1H-imidazol-1-yl)éthyl]amino]carbonyl]-3-oxo-4-(2-phényl-éthyl)-1H-1,4-benzodiazépine-2-acétique ;
acide (R,S)-2,3,4,5-tétrahydro-8-[[[3-(1H-imidazol-1-yl)propyl]amino]carbonyl]-3-oxo-4- (2-phényl-éthyl)-1H-1,4-benzodiazépine-2-acétique ;
acide (R,S)-8-[[[(6-aminohexyl]amino]carbonyl]-2,3,4,5-tétrahydro-3-oxo-4-(2-phényléthyl)-lH-1,4-benzodiazépine-2-acétique;
acide (R,S)-8-[[[4-aminobutyl]amino]carbonyl]-2,3,4,5-tétrahydro-3-oxo-4-(2-phényléthyl)-1H-1,4-benzodiazépine-2-acétique;
acide (R,S)-2,3,4,5-tétrahydro-3-[[[2-(1H-imidazol-4-yl)éthyl]amino]carbonyl]-3-oxo-4-(2-phényl- éthyl)-1H-1,4-benzodiazépine-2-acétique ;
acide (R,S)-2,3,4,5-tétrahydro-8-[[[2-(1H-méthyl-1H-imidazol-5-yl)éthyl]amino]carbonyl]-3-oxo-4-(2-phényléthyl)-1H-1,4-benzodiazépine-2-acétique ;
acide (R,S)-8-[[[2-(4-pyridyl)éthyl]méthylamino]carbonyl]-2,3,4,5-tétrahydro-3-oxo-4-(2-phényléthyl)-1H-1,4-benzodiazépine-2-acétique ;
iodure de méthyl (R,S)-8-[[2-[4-[1-(méthyl)pyridinium]éthyl]méthylamino]carbonyl]-2,3,4,5-tétrahydro-3-oxo-4-(2-phényléthyl)-1H-1,4-benzodiazépine-2-acétique ;
acide (R,S)-2,3,4,5-tétrahydro-3-oxo-4-[2-(cyclohexyl)éthyl]-8-[[[2-(1H-méthyl-4-pipéridinyl)éthyl]méthylamino]carbonyl]-1H-1,4-benzodiazépine-2-acétique ;
acide (R,S) -2,3,4,5-tétrahydro-3-oxo-4-(2-(phényléthyl)-8-[[[2-(1-méthyl-4-pipéridinyl)éthyl]méthylamino]carbonyl]-1H-1,4-benzodiazépine-2-acétique ;
acide 7-[3-(4-pyridyl)propyloxy]-2-(2-phényléthyl)-3-oxo-2,3,4,5-tétrahydro-1H-2-benzazépine-4-acétique ;
acide 7-[3-(4-pipéridinyl)propyloxy]-2-(2-phényléthyl)-3-oxo-2,3,4,5-tétrahydro-1H-2-benzazépine-4-acétique ;
acide 8- [1-[4-(4-pyridyl)pipérazinyl)carbonyl]-2,3,4,5-tétrahydro-3-oxo-4-(2-phényléthyl)-1H-1,4-benzodiazépine-2-acétique ;
acide 2-[3-(4-pipéridinyl)propyloxy]-7-oxo-6,7,8,9-tétrahydro-5H-benzocycloheptène-6-acétique ;
acide 3-[3-(4-pipéridinyl)propyloxy]-7-oxo-6,7,8,9-tétrahydro-5H-benzocycloheptène-6-acétique ;
acide (R,S)-8-[[[2[4-(2-aminopyridyl)]éthyl]-amino]carbony]-2,3,4,5-tétrahydro-3-oxo-4-[2-(phényléthyl)-1H-1,4-benzodiazépine-2-acétique ;
acide (R,S)-2,3,4,5-tétrahydro-3-oxo-4-(2-phényléthyl)-8-[[[2-(1-pipérazinyl)éthyl]amino]carbonyl]-1H-1,4-benzodiazépine-2-acétique ;
acide (R,S)-2,3,4,5-tétrahydro-3-oxo-4-(2-phényléthyl)-8-[[[2-(4-pipéridinyl)éthyl]amino]carbonyl]-1H-1,4-benzodiazépine-2-acétique ;
acide (R,S)-2,3,4,5-tétrahydro-3-oxo-4-(2-phényléthyl)-8-[[[2-(1-pipérazinyl)éthyl]méthylamino]carbonyl]-1H-1,4-benzodiazépine-2-acétique ;
acide (R,S)-2,3,4,5-tétrahydro-3-oxo-4-(2-phényléthyl) -8- [[[2-(4-pipéridinyl)éthyl]méthylamino]carbonyl]-1H-1,4-benzodiazépine-2-acétique ;
acide (R,S)-2,3,4,5-tétrahydro-3-oxo-4-[2-(cyclohexyl)éthyl]-8-[[[2-(4-pipéridinyl)éthyl]méthylamino]-carbonyl]-1H-1,4-benzodiazépine-2-acétique ;
acide (R,S)-2,3,4,5-tétrahydro-8-[[[2-(1-méthyl-1,2,5,6-tétrahydropyrid-4-yl)éthyl]méthylamino]carbonyl]-3-oxo-4-[2-(phényléthyl)-1H-1,4-benzodiazépine-2-acétique ;
acide (R,S)-2,3,4,5-tétrahydro-3-oxo-4-[2-(phényléthyl)-8-[4-pipéridin-4-yl)-1(E)-butényl]-1H-1,4-benzodiazépine-2-acétique ;
acide (R,S)-2,3,4,5-tétrahydro-3-oxo-2-(2-cyclohexyléthyl)-7-[[[2-(pipéridin-4-yl) éthyl]méthylamino]-carbonyl]-1H-2-benzazépine-4-acétique ;
acide (R,S)-2,3,4,5-tétrahydro-3-oxo-4-(2-phényléthyl)-7-[[4-(pyrid-4-yl)pipérazin-1-yl)carbonyl]-1H-1,4-benzodiazépine-2-acétique ;
acide (R,S)-2,3,4,5-tétrahydro-3-oxo-7-[[[2-(pipéridin-4-yl)éthyl]méthylamino]carbonyl]-1H-2-benzazépine-4-acétique ;
acide (R,S)-7-[[[2-[(2-amino)pyrid-4-yl]éthyl]méthylamino]carbonyl]-2,3,4,5-tétrahydro-3-oxo-2-(2-phényléthyl)-1H-2-benzazépine-4-acétique ;
acide 7-[1-[4-pipérazin-1-yl)but-1-ynyl]]-2-(2-phényléthyl)-3-oxo-2,3,4,5-tétrahydro-1H-2-benzazépine-4-acétique ;
acide (R,S)-7-[[[2-(pipéridin-4-yl)éthyl)méthylamino]carbonyl]-2,3,4,5-tétrahydro-2-méthyl-3-oxo-1H-2-benzazépine-4-acétique; ou
acide 8-[[[2-(2-aminopyridin-4-yl)éthyl]méthylamino]carbonyl]-2,3,4,5-tétrahydro-3-oxo-4- (2-phényléthyl)-1H-1,4-benzodiazépine-2-acétique.

12. Composé, qui consiste en :
acide (R,S)-2,3,4,5-tétrahydro-3-oxo-2-(2-(phényléthyl)-7-[[[2-(pipéridin-4-yl)éthyl]méthylamino]-carbonyl]-1H-2-benzazépine-4-acétique ; ou
acide (R,S)-7-[(4,4'-bipipéridin-1-yl)carbony]-2,3,4,5-tétrahydro-3-oxo-4-(2-phényléthyl)-1H-1,4-benzodiazépine-2-acétique.

13. Composé intermédiaire, de formule : 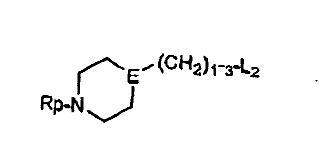 dans laquelle
E représente N ou un groupe CH ;
R^{p} représente un groupe tosyle, butyloxycarbonyle, benzyloxycarbonyle ou acétyle ;
L² représente CHO, CO₂R', C≡C-H, OH, Cl, Br, I, CH₂-T ou NR'R" ;
T représente un groupe CF₃SO₃, OH, NHR", Cl, Br ou I ; et
R' et R" répondent aux définitions suivant la revendication 1.

14. Composition pharmaceutique comprenant un composé suivant l'une quelconque des revendications 1 à 12 et un support pharmaceutiquement acceptable.

15. Composé suivant l'une quelconque des revendications 1 à 12, destiné à être utilisé comme médicament.

16. Utilisation d'un composé suivant l'une quelconque des revendications 1 à 12 dans la production d'un médicament destiné à inhiber l'agrégation plaquettaire, ou à traiter l'infarctus du myocarde, des attaques ischémiques transitoires, un ictus ou une réocclusion d'une artère ou veine après une thérapeutique fibrinolytique.

17. Procédé pour la préparation d'un composé de formule (I) suivant la revendication 1, qui comprend la réaction d'un composé de formule (XI) avec un composé de formule (XII) : 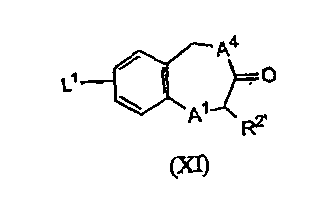
R^{6''} -L² (XII)
formules dans lesquelles A¹, A⁴, U, V, Z, R, R¹⁰, q, s et r répondent aux définitions mentionnées pour la formule (I), n'importe quels groupes fonctionnels réactifs étant protégés ;
L¹ et L² représentent des groupes fonctionnels qui sont capables de réagir pour former la liaison -(CR'R¹⁰)ᵣ-U-(CR'₂)ₛ-V- ;
R^{6"} représente un groupe W'-(CR'₂)_{q}-Z- et n'importe quelle partie du groupe -(CR'R¹⁰)ᵣ-U-(CR'₂)ₛ-V- qui est connectée à L², n'importe quels groupes fonctionnels réactifs étant protégés ;
R^{2'} représente un groupe R² répondant à la définition mentionnée par la formule (I), n'importe quel groupe réactif étant protégé ;
w' représente un groupe W répondant à la définition mentionnée par la formule (I), n'importe quel groupe azoté basique étant protégé ;
pour la formation d'un composé de formule : 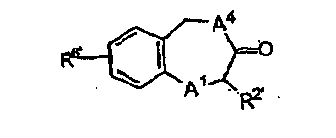 dans laquelle R^{6'} représente un groupe W'-(CR'₂)_{q}-Z(CR'R¹⁰)ᵣ-U-(CR'₂)ₛ-V- ;
et ensuite l'élimination de tous les groupes protecteurs, et facultativement la formation d'un sel pharmaceutiquement acceptable.
